(19) Europäisches Patentamt
European Patent Office
Office européen des brevets

(11) EP 1 987 010 B1

(12) **FASCICULE DE BREVET EUROPEEN**

(45) Date de publication et mention de la délivrance du brevet:
**07.04.2010 Bulletin 2010/14**

(21) Numéro de dépôt: **07730900.3**

(22) Date de dépôt: **01.02.2007**

(51) Int Cl.:
***C07D 235/02*** (2006.01) ***C07D 487/04*** (2006.01)
***C07D 471/04*** (2006.01) ***A61K 31/437*** (2006.01)
***A61K 31/4188*** (2006.01) ***A61P 29/00*** (2006.01)

(86) Numéro de dépôt international:
**PCT/FR2007/000183**

(87) Numéro de publication internationale:
**WO 2007/088277 (09.08.2007 Gazette 2007/32)**

(54) **DÉRIVÉS DE N-HETEROARYL-CARBOXAMIDES TRICYCLIQUES CONTENANT UN MOTIF BENZIMIDAZOLE, LEUR PRÉPARATION ET LEUR APPLICATION EN THÉRAPEUTIQUE**

TRICYCLISCHE N-HETEROARYL-CARBOXAMID-DERIVATE MIT EINER BENZIMIDAZOL-EINHEIT, VERFAHREN ZU IHRER HERSTELLUNG UND IHRE THERAPEUTISCHE VERWENDUNG

TRICYCLIC N-HETEROARYL-CARBOXAMIDE DERIVATIVES CONTAINING A BENZIMIDAZOLE UNIT, METHOD FOR PREPARING SAME AND THEIR THERAPEUTIC USE

(84) Etats contractants désignés:
**AT BE BG CH CY CZ DE DK EE ES FI FR GB GR HU IE IS IT LI LT LU LV MC NL PL PT RO SE SI SK TR**
Etats d'extension désignés:
**AL BA HR MK RS**

(30) Priorité: **03.02.2006 FR 0601007**

(43) Date de publication de la demande:
**05.11.2008 Bulletin 2008/45**

(73) Titulaire: **Sanofi-Aventis**
**75013 Paris (FR)**

(72) Inventeurs:
- **DUBOIS Laurent**
  **F-92160 ANTONY (FR)**
- **EVANNO, Yannick**
  **F-92160 ANTONY (FR)**
- **EVEN, Luc**
  **F-92160 ANTONY (FR)**
- **GILLE, Catherine**
  **F-92160 ANTONY (FR)**
- **MALANDA, André**
  **F-92160 ANTONY (FR)**
- **MACHNIK, David**
  **F-92160 Antony (FR)**
- **RAKOTOARISOA,Nathalie**
  **F-92160 Antony (FR)**

(74) Mandataire: **Gaslonde, Aude et al**
**Sanofi-Aventis**
**Département Brevets**
**174, Avenue de France**
**75013 Paris (FR)**

(56) Documents cités:
**EP-A- 1 535 922** **WO-A-2005/028445**
**WO-A-2005/105798**

- **DATABASE CHEMCATS CHEMICAL ABSTRACTS SERVICE, COLUMBUS, OHIO,US; 24 janvier 2006 (2006-01-24), XP002399484 extrait de STN Database accession no. 2006:2135904 & "Enamine Screening Library" 24 janvier 2006 (2006-01-24), ENAMINE , KIEV**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

Printed by Jouve, 75001 PARIS (FR)

EP 1 987 010 B1

## Description

L'invention se rapporte à des dérivés de N-hétéroaryl-carboxamides tricycliques contenant un motif benzimidazole, leur préparation et leur application en thérapeutique.

L'invention a pour objet des composés dérivés de N-hétéroaryl-carboxamides tricycliques contenant un motif benzimidazole, qui présentent une activité antagoniste ou agoniste *in vitro* et *in vivo* pour les récepteurs de types TRPV1 (ou VR1).

WO 2005/028445, WO 2005/05798 et EP 1535922 décrivent des composés capables de se lier aux récepteurs VR1.

L'invention a pour objet les composés répondant à la formule (I)

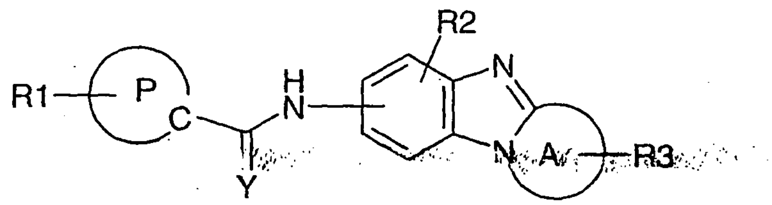

(I)

dans laquelle :

A représente, avec la liaison C-N du motif benzimidazole auquel il est fusionné, un hétérocycle monocyclique ou un hétéroaryle monocyclique de 4 à 7 chaînons, contenant de un à trois hétéroatomes choisis parmi O, S ou N , y compris l'atome d'azote du motif benzimidazole ;

P représente un hétérocycle bicyclique ou hétéroaryle bicyclique à 8-, 9-, 10- ou 11 chaînons, comprenant de 1 à 6 hétéroatomes sélectionnés parmi N, O et S ; P étant lié au groupe -C(Y)- par un atome de carbone ;
à la condition que, lorsque A représente un hétérocycle saturé à 7 chaînons, P est différent du groupe 2,3-dihydro-1,4-benzodioxane, du groupe 1-benzopyran-2-one, du groupe isoindole ;

$R_1$ représente de un à quatre atomes ou groupes, identiques ou différents, choisis parmi un atome d'hydrogène, un atome d'halogène, un groupe oxo, thio, $C_1$-$C_6$-alkyle, $C_3$-$C_7$-cycloalkyle, $C_3$-$C_7$-cydoalkyle-$C_1$-$C_3$-alkylène, $C_1$-$C_6$-fluoroalkyle, aryloxy-$C_1$-$C_6$-alkyle, hétéroaryloxy-$C_1$-$C_6$-alkyle, aryl-$C_1$-$C_3$-alkylènoxy-$C_1$-$C_6$-alkyle, hétéroaryl-$C_1$-$C_3$-alkylènoxy-$C_1$-$C_6$-alkyle, arylthio-$C_1$-$C_6$-alkyle, hétéroarylthio-$C_1$-$C_6$-alkyle, aryl-$C_1$-$C_3$-alkylène-thio-$C_1$-$C_6$-alkyle, hétéroaryl-$C_1$-$C_3$-alkylène-thio-$C_1$-$C_6$-alkyle, $C_1$-$C_6$-alcoxy, $C_3$-$C_7$-cycloalkyloxy, $C_3$-$C_7$-cycloalkyle-$C_1$-$C_3$-alkylènoxy, $C_1$-$C_6$-fluoroalcoxy, cyano, $C(O)NR_4R_5$, nitro, $NR_4R_5$, $C_1$-$C_6$-thioalkyle, $C_3$-$C_7$-cydoalkylthio, $C_3$-$C_7$-cycloalkyle-$C_1$-$C_3$-alkylène-thio, -S(O)-$C_1$-$C_6$-alkyle, -S(O)-$C_3$-$C_7$-cycloalkyle, -S(O)-$C_1$-$C_3$-alkylène-$C_3$-$C_7$-cycloalkyle, $C_1$-$C_6$-alkyle-$S(O)_2$-, $C_1$-$C_6$-fluoroalkyle-$S(O)_2$-, $C_3$-$C_7$-cycloalkyle-$S(O)_2$-, $C_3$-$C_7$-cycloalkyle-$C_1$-$C_3$-alkylène-$S(O)_2$-, $SO_2NR_4R_5$, $SF_5$, $NR_6C(O)R_7$, $NR_6SO_2R_8$, $R_4R_5NC(O)$-$C_1$-$C_3$-alkylène, aryle, hétéroaryle, aryl-$C_1$-$C_5$-alkylène, hétéroaryl-$C_1$-$C_5$-alkylène, aryloxy, arylthio, hétéroaryloxy ou hétéroarylthio ;
les dits groupes hétéroaryle ou aryle de $R_1$ étant éventuellement substitués par un ou plusieurs substituants $R_9$, identiques ou différents l'un de l'autre ;
à la condition que lorsque $R_1$ est attaché à un atome d'azote de P, alors $R_1$ est différent d'un atome d'halogène, d'un groupe oxo, thio, cyano, nitro, $SF_5$, $NR_4R_5$, $C_1$-$C_6$-thioalkyle, thioaryle, thiohétéroaryle, $C_1$-$C_6$-alcoxy, aryloxy, hétéroaryloxy, -$NR_6COR_7$ et $NR_6SO_2R_8$ ;

Y représente un atome d'oxygène ou de soufre ;

$R_2$ représente un atome d'hydrogène, un atome d'halogène, un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle ou $C_1$-$C_6$-alcoxy ;

$R_3$ représente de un à trois atomes ou groupes, identiques ou différents, choisis parmi un atome d'hydrogène, un atome d'halogène, un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, hydroxyle, $C_1$-$C_6$-alcoxy, $C_3$-$C_7$-cycloalkyloxy, $C_3$-$C_7$-cycloalkyle-$C_1$-$C_3$-alkylénoxy, lorsque $R_3$ est porté par un atome de carbone ;
ou

$R_3$ représente de un à deux atomes ou groupes, identiques ou différents, choisis parmi un atome d'hydrogène, un

atome d'halogène, un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, aryle-C(O)-, $C_1$-$C_6$-alkyle-C(O)-, $C_3$-$C_7$-cycloalkyle-C(O)-, $C_3$-$C_7$-cycloalkyle-$C_1$-$C_3$-alkylène-C(O)-, $C_1$-$C_6$-fluoroalkyle-C(O)-, aryle-$S(O)_2$-, $C_1$-$C_6$-alkyle-$S(O)_2$-, $C_1$-$C_6$-fluoroalkyle-$S(O)_2$-, $C_3$-$C_7$-cycloalkyle-$S(O)_2$-, $C_3$-$C_7$-cycloalkyle-$C_1$-$C_3$-alkylène-$S(O)_2$-, $C_1$-$C_6$-alkyle-O-C(O)-, aryle-$C_1$-$C_3$-alkyle-O-C(O)-, $C_3$-$C_7$-cycloalkyle-O-C(O)-, $C_3$-$C_7$-cycloalkyle-$C_1$-$C_3$-alkylène-O-C(O)-, $C_1$-$C_6$-fluoroalkyle-O-C(O)-, aryle-O-C(O)- , hétéroaryl-O-C(O)- , lorsque $R_3$ est porté par un atome d'azote ;

$R_4$ et $R_5$, représentent, indépendamment l'un de l'autre, un atome d'hydrogène ou un groupe $C_1$-$C_6$-alkyle, $C_3$-$C_7$-cycloalkyle, $C_3$-$C_7$-cycloalkyle-$C_1$-$C_3$-alkylène, aryle-$C_1$-$C_5$-alkylène ou aryle ;
ou $R_4$ et $R_5$ forment ensemble, avec l'atome d'azote qui les porte, un groupe azétidine, pyrrolidine, pipéridine, azépine, morpholine, thiomorpholine, pipérazine, homopipérazine, le groupe $NR_4R_5$ étant éventuellement substitué par un groupe $C_1$-$C_6$-alkyle, $C_3$-$C_7$-cycloalkyle, $C_3$-$C_7$-cycloalkyle-$C_1$-$C_3$-alkylène, aryle-$C_1$-$C_6$-alkylène, aryle, hétéroaryle, aryle-$S(O)_2$-, $C_1$-$C_6$-alkyle-$S(O)_2$-, $C_1$-$C_6$-fluoroalkyle-$S(O)_2$-, $C_3$-$C_7$-cycloalkyle-$S(O)_2$-, $C_3$-$C_7$-cycloalkyle-$C_1$-$C_3$-alkylène-$S(O)_2$-, aryle-C(O)-, $C_1$-$C_6$-alkyle-C(O)-, $C_3$-$C_7$-cycloalkyle-C(O)-, $C_3$-$C_7$-cycloalkyle-$C_1$-$C_3$-alkylène-C(O)-, $C_1$-$C_6$-fluoroalkyle-C(O)-, hydroxyle, $C_1$-$C_6$-alkyloxy, $C_1$-$C_6$-fluoroalkyle, aryloxy-$C_1$-$C_6$-alkylène, aryloxy, hétéroaryloxy-$C_1$-$C_6$-alkylène, hétéroaryloxy ;

$R_6$ et $R_7$ représentent, indépendamment l'un de l'autre, un atome d'hydrogène, un groupe $C_1$-$C_6$-alkyle, $C_3$-$C_7$-cycloalkyle, $C_3$-$C_7$-cycloalkyle-$C_1$-$C_3$-alkylène, aryle-$C_1$-$C_6$-alkylène ou aryle ;
ou $R_6$ et $R_7$ forment ensemble un lactame de 4 à 7 chaînons comprenant l'atome d'azote et le groupe C(O) qui les portent ;

$R_8$ représente un groupe $C_1$-$C_6$-alkyle, $C_3$-$C_7$-cycloalkyle, $C_3$-$C_7$-cycloalkyle-$C_1$-$C_3$-alkylène, aryle-$C_1$-$C_6$-alkylène ou aryle ;
ou $R_6$ et $R_8$ forment ensemble un sultame de 4 à 7 chaînons comprenant l'atome d'azote et le groupe $S(O)_2$ qui les portent ;

$R_9$ représente un atome d'halogène, un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, $C_1$-$C_6$-alcoxy, $C_1$-$C_6$-fluoroalcoxy, nitro, cyano, $NR_4R_5$, $R_4R_5N$-$C_1$-$C_3$-alkylène, aryle, hétéroaryle, aryloxy, arylthio, hétéroaryloxy ou hétéroarylthio, lesdits groupes hétéroaryle ou aryle étant éventuellement substitués par un ou plusieurs substituants choisis parmi un atome d'halogène, un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, $C_1$-$C_6$-alcoxy, $C_1$-$C_6$-fluoroalcoxy, nitro, cyano, $NR_4R_5$, $R_4R_5N$-$C_1$-$C_3$-alkylène.

le 2-(2-chlorophényl)-1,3-dioxo-N-(7,8,9,10-tétrahydro-6H-azepino[1,2-a]benzimidazol-3-yl)isoindoline-5-carboxamide étant exclu

Dans les composés de formule générale (I) :

- le ou les atomes de soufre de l'hétérocycle A peuvent être sous forme oxydée (S(O) ou $S(O)_2$) ;
- le ou les atomes d'azote peuvent être sous forme oxydée (N-oxyde).

Les composés de formule (I) peuvent comporter un ou plusieurs atomes de carbone asymétriques. Ils peuvent donc exister sous forme d'énantiomères ou de diastéréoisomères. Ces énantiomères, diastéréoisomères, ainsi que leurs mélanges, y compris les mélanges racémiques, font partie de l'invention.
Les composés de formule (I) peuvent exister à l'état de bases ou de sels d'addition à des acides. De tels sels d'addition font partie de l'invention.
Ces sels peuvent être préparés avec des acides pharmaceutiquement acceptables, mais les sels d'autres acides utiles, par exemple, pour la purification ou l'isolement des composés de formule (I) font également partie de l'invention.
Les composés de formule (I) peuvent également exister sous forme d'hydrates ou de solvats, à savoir sous forme d'associations ou de combinaisons avec une ou plusieurs molécules d'eau ou avec un solvant. De tels hydrates et solvats font également partie de l'invention.
Dans le cadre de la présente invention, on entend par :

- un atome d'halogène : un fluor, un chlore, un brome ou un iode ;
- $C_t$-$C_z$ : une chaîne carbonée pouvant avoir de t à z atomes de carbone où t et z peuvent prendre les valeurs de 1 à 7 ; par exemple $C_1$-$C_3$ est une chaîne carbonée qui peut avoir de 1 à 3 atomes de carbone ;

- un alkyle : un groupe aliphatique saturé linéaire ou ramifié. A titre d'exemples, on peut citer les groupes méthyle, éthyle, propyle, isopropyle, butyle, isobutyle, tertiobutyle, pentyle, etc ;
- un alkylène : un groupe alkyle divalent saturé, linéaire ou ramifié, par exemple un groupe $C_{1-3}$-alkylène représente une chaîne carbonée divalente de 1 à 3 atomes de carbone, linéaire ou ramifiée, plus particulièrement un méthylène, éthylène, 1-méthyléthylène, propylène ;
- un cycloalkyle : un groupe alkyle cyclique. A titre d'exemples, on peut citer les groupes cyclopropyle, cyclobutyle, cyclopentyle, cyclohexyle, etc ;
- un fluoroalkyle : un groupe alkyle dont un ou plusieurs atomes d'hydrogène ont été substitués par un atome de fluor ;
- un alcoxy : un radical -O-alkyle où le groupe alkyle est tel que précédemment défini ;
- un fluoroalcoxy : un groupe alcoxy dont un ou plusieurs atomes d'hydrogène ont été substitués par un atome de fluor ;
- un thioalkyle ou alkyle-thio : un radical -S-alkyle où le groupe alkyle est tel que précédemment défini ;
- un aryle : un groupe aromatique mono- ou bicyclique comprenant entre 6 et 10 atomes de carbones. A titre d'exemples de groupes aryles, on peut citer les groupes phényle ou naphtyle ;
- un hétérocycle : un groupe mono-, bi- ou tricyclique, saturé ou partiellement insaturé, de 5 à 17 chaînons, comprenant de 1 à 8 hétéroatomes choisis parmi O, S ou N.

A titre d'exemples d'hétérocycle monocyclique, on peut citer les groupes azétidinyle, pyrrolidinyle, pipéridinyle, azépinyle, morpholinyle, thiomorpholinyle, pipérazinyle, homopipérazinyle, dihydro-oxazolyle, dihydrothiazolyle, dihydroimidazolyle, dihydropyrrolyle ou tétrahydropyridinyle ;
à titre d'exemples d'hétérocycle bicyclique, on peut citer les groupes indolinyle, isoindolinyle, dihydrobenzofuranyle, dihydrobenzothiophényle, dihydrobenzoxazolinyle, dihydroisobenzofuranyle, dihydrobenzimidazolyle, dihydroisobenzothiazolyle, dihydroquinoléinyle, tétrahydroquinoléinyle, dihydroisoquinoléinyle, tétrahydroisoquinoléinyle, dihydrobenzoxazinyle, benzothiazinyle, dihydrobenzothiazinyle, dihydroquinazolinyle, tétrahydroquinazolinyle, dihydroquinoxalinyle, tétrahydroquinoxalinyle, dihydrophtalazinyle, tétrahydrophtalazinyle, tétrahydrobenzazépinyle, tétrahydrobenzo[1,4]diazépinyle, tétrahydrobenzo[1,4]oxazépinyle ou tétrahydrobenzo[1,4]thiazépinyle ;
à titre d'exemples d'hétérocycle tricyclique, on peut citer les groupes dihydroimidazo[1,2-a]benzimidazolyle, dihydropyrrolo[1,2-a]benzimidazolyle, tétrahydropyrido[1,2-a]benzimidazolyle ou dihydrothiazolo[1,2-a]benzimidazolyle, tétrahydropyrimido[1,2-a]benzimidazolyle, tétrahydrodiazépino[1,3][1,2-*a*]benzimidazolyle, dihydrooxazino[1,4][4,3-a]benzimidazolyle, tétrahydropyrazino[1,2-a]benzimidazlolyle ;

- un hétéroaryle : un groupe mono-, bi- ou tricyclique aromatique de 5 à 14 chaînons contenant de 1 à 8 hétéroatomes choisis parmi O, S ou N.

A titre d'exemples d'hétéroaryle monocyclique, on peut citer les groupes imidazolyle, pyrazolyle thiazolyle, oxazolyle, isothiazolyle, isoxazolyle, furanyle, thiophényle, oxadiazolyle, thiadiazolyle, triazolyle, tétrazolyle, pyridinyle, pyrazinyle, pyrimidinyle, pyridazinyle, triazinyle ;
à titre d'exemples d'hétéroaryle bicyclique, on peut citer les groupes indolyle, isoindolyle benzofuranyle, benzothiophényle, benzoxazolyle, benzimidazolyle, indazolyle, benzothiazolyle, isobenzofuranyle, isobenzothiazolyle, pyrrolo[2,3-c]pyridinyle, pyrrolo[2,3-b]pyridinyle, pyrrolo[3,2-b]pyridinyle, pyrrolo[3,2-c]pyridinyle, quinoléinyle, isoquinoléinyle, cinnolinyle, quinazolinyle ou quinoxalinyle ;
à titre d'exemples d'hétéroaryle tricyclique, on peut citer les groupes pyrido[1,2-a]benzimidazolyle, thiazolo[1,2-a]benzimidazolyle ou imidazo[1,2-a]benzimidazolyle , pyrimido[1,2-a]benzimidazolyle, pyrazino[1,2-a]benzimidazolyle ;

- « oxo » signifie « =O » ;
- « thio » signifie « =S ».

Parmi les composés de l'invention, un premier groupe de composés est constitué par les composés de formule générale (II)

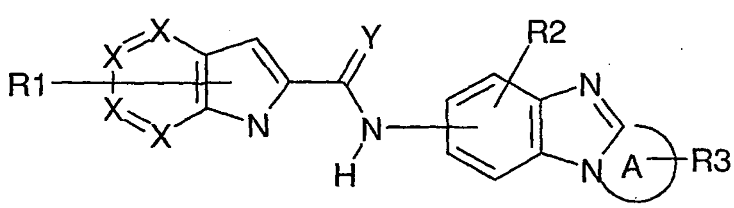

(II)

dans laquelle :

X représente un atome de carbone ou un atome d'azote ; lesdits X étant identiques ou différents entre eux et le nombre de X = N n'étant pas supérieur à 2 ;
$R_1$, $R_2$, $R_3$, Y et A étant tels que définis dans la formule générale (I), $R_1$ pouvant être-lié au motif à 6 éléments ou à 5 éléments du bicycle.

Parmi les composés de l'invention, un deuxième groupe de composés est constitué par les composés de formule (III)

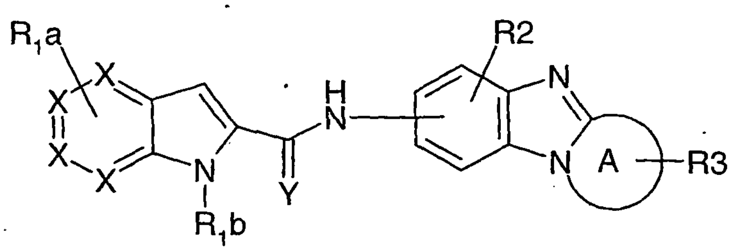

(III)

dans laquelle:

$R_{1a}$ représente un ou plusieurs atomes ou groupes, identiques ou différents, choisi parmi un atome d'hydrogène, un atome d'halogène ou un groupe $C_1$-$C_6$-alkyle, $C_1$-$C_6$-fluoroalkyle, $C_1$-$C_6$-alcoxy, $C_1$-$C_6$-thioalkyle, $C_1$-$C_6$-alkyle-$S(O)_2$-, $NR_4R_5$, nitro ;
$R_{1b}$ représente un atome d'hydrogène, un groupe $C_1$-$C_6$-alkyle, hétéroaryloxy-$C_1$-$C_6$-alkyle, aryl-$C_1$-$C_3$-alkylènoxy-$C_1$-$C_6$-alkyle, $R_4R_5NC(O)$-$C_1$-$C_3$-alkylène, aryle, hétéroaryle, aryl-$C_1$-$C_6$-alkylène ou hétéroaryl-$C_1$-$C_6$-alkylène ; lesdits groupes hétéroaryle ou aryle de $R_{1b}$ étant éventuellement substitués par un ou plusieurs substituants $R_9$, identiques ou différents l'un de l'autre ;
$R_9$ représente un atome d'halogène ou un groupe $C_1$-$C_6$-alkyle, $C_1$-$C_6$-alcoxy, $C_1$-$C_6$-fluoroalkyle, aryle, hétéroaryle, $NR_4R_5$, arylthio, lesdits groupes hétéroaryle ou aryle étant éventuellement substitués par un ou plusieurs substituants choisis parmi un atome d'halogène, un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, $C_1$-$C_6$-alcoxy, $C_1$-$C_6$-fluoroalcoxy, nitro, cyano, $R_4R_5N$-$C_1$-$C_3$-alkylène ;
$R_2$, $R_3$, $R_4$, $R_5$, A, X et Y étant tels que définis dans la formule générale (II).

Parmi les composés de l'invention, un troisième groupe de composés est constitué par les composés de formule générale (IV) :

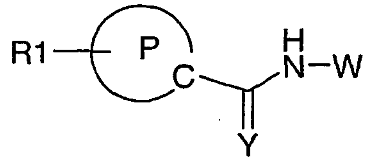

(IV)

dans laquelle W est un hétérocycle tricyclique ou un hétéroaryle tricyclique choisi parmi :

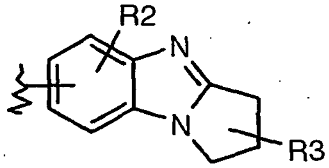

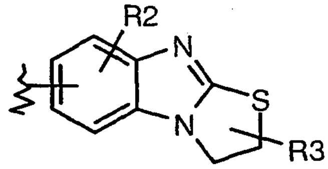

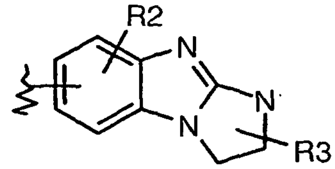

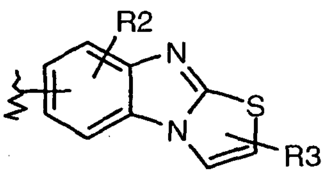
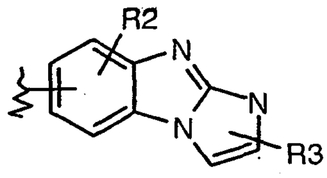
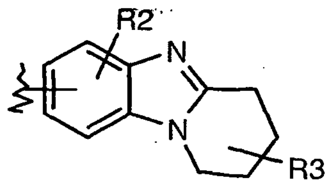

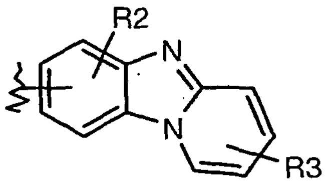
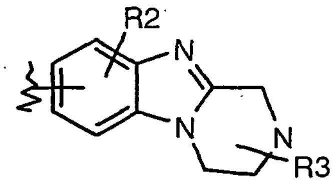
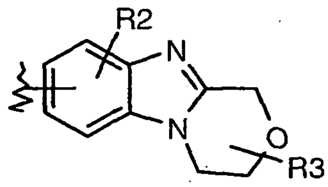

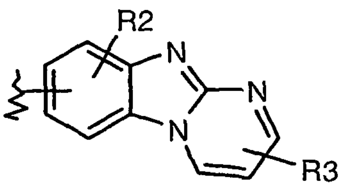
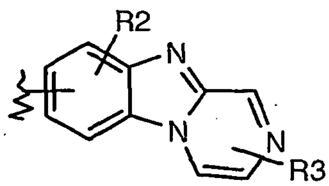
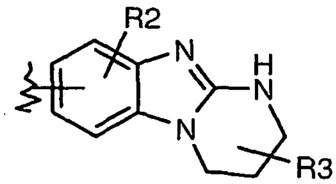

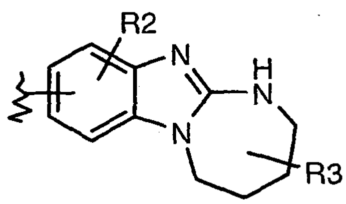
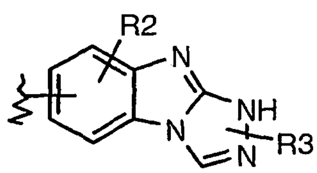

$R_1$, $R_2$, $R_3$, P et Y étant tels que définis dans la formule générale (I).

Parmi les composés de l'invention, un quatrième groupe de composés est constitué par les composés de formule générale (V)

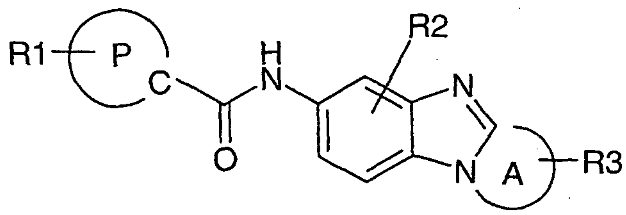

(V)

dans laquelle :

$R_1$, $R_2$, $R_3$, A et P sont tels que définis dans la formule générale (1).

Parmi les composés de l'invention, un cinquième groupe de composés est constitué par les composés de formule générale (V) dans laquelle

$R_2$ représente un atome d'hydrogène, un groupe $C_1$-$C_6$-alkyle ou $C_1$-$C_6$-alcoxy ;

$R_3$ représente de un à trois atomes ou groupes, identiques ou différents, choisis parmi un atome d'hydrogène, un groupe $C_1$-$C_6$-alkyle, $C_1$-$C_6$-alcoxy ou hydroxyle, lorsque $R_3$ est porté par un atome de carbone ;

ou
$R_3$ représente de un à deux atomes ou groupes, identiques ou différents, choisis parmi un atome d'hydrogène, un groupe $C_1$-$C_6$-alkyle, $C_1$-$C_6$-alkyle-O-C(O)-, aryl-$C_1$-$C_3$-alkyle-O-C(O)-, lorsque $R_3$ est porté par un atome d'azote ;
$R_1$, A et P étant tels que définis dans la formule générale (I).

Parmi les composés de l'invention, un sixième groupe de composés est constitué par les composés de formule générale (V) dans laquelle
A représente, avec la liaison C-N du motif benzimidazole auquel il est fusionné, un hétérocycle monocyclique ou un hétéroaryle monocyclique de 5 à 7 chaînons, contenant de un à trois hétéroatomes choisis parmi O, S ou N, y compris l'atome d'azote du motif benzimidazole ;
$R_1$, $R_2$, $R_3$ et P étant tels que définis dans la formule générale (I).

Parmi les composés de l'invention, un septième groupe de composés est constitué par les composés de formule générale (Va) dans laquelle

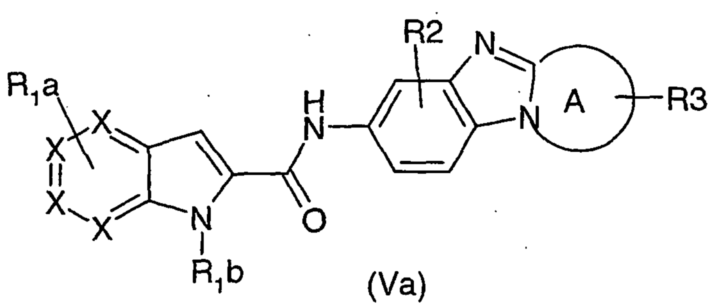

(Va)

A représente, avec la liaison C-N du motif benzimidazole auquel il est fusionné, un hétérocycle monocyclique ou un hétéroaryle monocyclique de 5 à 7 chaînons, contenant de un ou deux hétéroatomes choisis parmi O, S ou N, y compris l'atome d'azote du motif benzimidazole ;
X représente un atome de carbone ou un atome d'azote ; lesdits X étant identiques ou différents entre eux et le nombre de X = N n'étant pas supérieur à 1 ;
$R_{1a}$ représente un ou plusieurs atomes ou groupes, identiques ou différents, choisi parmi un atome d'hydrogène, un atome d'halogène, plus particulièrement fluor, brome, chlore, ou un groupe $C_1$-$C_6$-alkyle, plus particulièrement méthyle, éthyle, isopropyle, tertiobutyle, $C_1$-$C_6$-fluoroalkyle, plus particulièrement trifluorométhyle, $C_1$-$C_6$-alcoxy, plus particulièrement méthoxy, $C_1$-$C_6$-thioalkyle, plus particulièrement $SCH_3$, $C_1$-$C_6$-alkyle-$S(O)_2$-, plus particulièrement-$SO_2CH_3$, $NR_4R_5$, nitro ;
$R_{1b}$ représente un atome d'hydrogène, un groupe $C_1$-$C_6$-alkyle, plus particulièrement méthyle, hétéroaryloxy-$C_1$-$C_6$-alkyle, plus particulièrement pyridinyloxy-éthyle, aryl-$C_1$-$C_3$-alkylènoxy-$C_1$-$C_6$-alkyle, plus particulièrement benzyloxyéthyle, $R_4R_5NC(O)$-$C_1$-$C_3$-alkylène, plus particulièrement $R_4R_5NC(O)CH_2$-, aryle, plus particulièrement phényle, hétéroaryle, plus particulièrement pyridinyle, aryl-$C_1$-$C_6$-alkylène, plus particulièrement benzyle ou naphtylméthyle, phényléthyle, phénylpropyle, ou hétéroaryl-$C_1$-$C_6$-alkylène, plus particulièrement pyridinylméthyle, pyridinyléthyle, pyridinylpropyle, thiazolylméthyle, pyrimidinylméthyle, quinolinylméthyle, quinoxalinylméthyle, furanylméthyle, pyrazinylméthyle, benzothiazolylméthyle ;
lesdits groupes hétéroaryle ou aryle de $R_{1b}$ étant éventuellement substitués par un ou plusieurs substituants $R_9$, identiques ou différents l'un de l'autre
$R_2$ représente un atome d'hydrogène ;
$R_3$ représente de un atome ou groupe choisi parmi un atome d'hydrogène, un groupe $C_1$-$C_6$-alkyle, plus particulièrement méthyle, $C_1$-$C_6$-alcoxy, plus particulièrement méthoxy, ou hydroxyle, lorsque $R_3$ est porté par un atome de carbone ;
ou
$R_3$ représente de un atome ou groupe choisi parmi un atome d'hydrogène, un groupe $C_1$-$C_6$-alkyle, plus particulièrement méthyle, $C_1$-$C_6$-alkyle-O-C(O)-, plus particulièrement $(CH_3)_3C$-O-C(O)-, lorsque $R_3$ est porté par un atome d'azote ;
$R_4$ et $R_5$, représentent, indépendamment l'un de l'autre, un groupe $C_1$-$C_6$-alkyle, plus particulièrement méthyle ou éthyle ; ou $R_4$ et $R_5$ forment ensemble, avec l'atome d'azote qui les porte, un groupe pyrrolidine ou morpholine ;
$R_9$ représente un atome d'halogène, plus particulièrement de fluor ou de chlore, ou un groupe $C_1$-$C_6$-alkyle, plus particulièrement méthyle, $C_1$-$C_6$-alcoxy, plus particulièrement méthoxy, $C_1$-$C_6$-fluoroalkyle, plus particulièrement trifluorométhyle, aryle, plus particulièrement phényle, hétéroaryle, plus particulièrement imidazolyle, $NR_4R_5$, arylthio, plus particulièrement phénylthio, lesdits groupes aryle étant éventuellement substitués par un ou plusieurs groupes $C_1$-$C_6$-alkyle, plus particulièrement méthyle.

Parmi les composés de l'invention, un huitième groupe de composés est constitué par les composés de formule

générale (I) dans laquelle à la fois $R_1$ et/ou $R_2$ et/ou $R_3$, et/ou A, et/ou P et/ou Y sont tels que définis dans les groupes ci-dessus.

Parmi les composés de l'invention, un neuvième groupe de composés est constitué par les composés de formule générale (I')

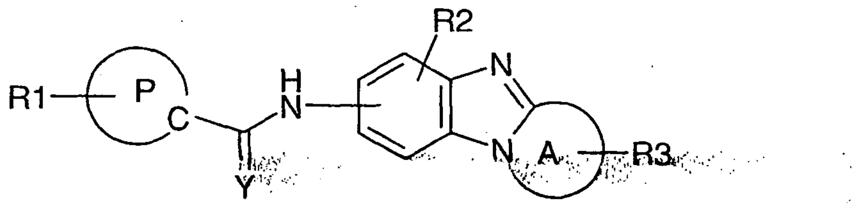

(I')

dans laquelle

A représente, avec la liaison C-N du motif benzimidazole auquel il est fusionné, un hétérocycle monocyclique ou un hétéroaryle monocyclique de 4 à 7 chaînons, contenant de 1 à 3 hétéroatomes choisis parmi O, S ou N, y compris l'atome d'azote du motif benzimidazole ;

P représente un hétérocycle bicyclique ou hétéroaryle bicyclique à 8-, 9-, 10- ou 11 chaînons, comprenant de 1 à 6 hétéroatomes sélectionnés parmi N, O et S ; à la condition que, lorsque A représente un hétérocycle saturé à 7 chaînons, P est différent du groupe 2,3-dihydro-1,4-benzodioxane et du groupe 1-benzopyran-2-one ;

$R_1$ représente de 1 à 4 atomes ou groupes, identiques ou différents, choisis parmi un atome d'hydrogène, un atome d'halogène, un groupe oxo, thio, $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, $C_1$-$C_6$-alcoxy, $C_3$-$C_7$-cycloalkyloxy, $C_3$-$C_7$-cycloalkyle-$C_1$-$C_3$-alkylénoxy, $C_1$-$C_6$-fluoroalcoxy, cyano, $C(O)NR_4R_5$, nitro, $NR_4R_5$, $C_1$-$C_6$-thioalkyle, $C_3$-$C_7$-cycloalkylthio, $C_3$-$C_7$-cycloalkyle-$C_1$-$C_3$-alkylène-thio, -S(O)-$C_1$-$C_6$-alkyle, -S(O)-$C_3$-$C_7$-cycloalkyle, -S(O)-$C_1$-$C_3$-alkylène-$C_3$-$C_7$-cycloalkyle, $C_1$-$C_6$-alkyle-$S(O)_2$-, $C_1$-$C_6$-fluoroalkyle-$S(O)_2$-, $C_3$-$C_7$-cycloalkyle-$S(O)_2$-, $C_3$-$C_7$-cycloalkyle-$C_1$-$C_3$-alkylène-$S(O)_2$-, $SO_2NR_4R_5$, $NR_6C(O)R_7$, $NR_6SO_2R_8$, $R_4R_5NC(O)$-$C_1$-$C_3$-alkylène, aryle, hétéroaryle, aryl-$C_1$-$C_5$-alkylène, hétéroaryl-$C_1$-$C_5$-alkylène, aryloxy, arylthio, hétéroaryloxy ou hétéroarylthio, lesdits groupes hétéroaryle ou aryle étant éventuellement substitués par un ou plusieurs substituants choisis parmi un atome d'halogène, un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, $C_1$-$C_6$-alcoxy, $C_1$-$C_6$-fluoroalcoxy, nitro, cyano, $NR_4R_5$, $R_4R_5N$-$C_1$-$C_3$-alkylène ;
à la condition que lorsque $R_1$ est attaché à un atome d'azote de P, alors $R_1$ est différent d'un atome d'halogène, d'un groupe oxo, thio, cyano, nitro, $NR_4R_5$, $C_1$-$C_6$-thioalkyle, thioaryle, thiohétéroaryle, $C_1$-$C_6$-alcoxy, aryloxy, hétéroaryloxy,-$NR_6COR_7$ et -$NR_6SO_2R_8$;

Y représente un atome d'oxygène ou de soufre ;

$R_2$ représente un atome d'hydrogène, un atome d'halogène, un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle ou $C_1$-$C_6$-alcoxy ;

$R_3$ représente de 1 à 3 atomes ou groupes, identiques ou différents, choisis parmi un atome d'hydrogène, un atome d'halogène, $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, $C_1$-$C_6$-alcoxy, $C_3$-$C_7$-cycloalkyloxy, $C_3$-$C_7$-cycloalkyle-$C_1$-$C_3$-alkylénoxy, lorsque $R_3$ est porté par un atome de carbone ;
ou
représente de 1 à 2 atomes ou groupes, identiques ou différents, choisis parmi un atome d'hydrogène, un atome d'halogène, un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, aryle-C(O)-, $C_1$-$C_6$-alkyle-C(O)-, $C_3$-$C_7$-cycloalkyle-C(O)-, $C_3$-$C_7$-cycloalkyle-$C_1$-$C_3$-alkylène-C(O)-, $C_1$-$C_6$-fluoroalkyle-C(O)-, aryle-$S(O)_2$-, $C_1$-$C_6$-alkyle-$S(O)_2$-, $C_1$-$C_6$-fluoroalkyle-$S(O)_2$-, $C_3$-$C_7$-cycloalkyle-$S(O)_2$-, $C_3$-$C_7$-cycloalkyle-$C_1$-$C_3$-alkylène-$S(O)_2$-, $C_1$-$C_6$-alkyle-O-C(O)-, $C_3$-$C_7$-cycloalkyle-O-C(O)-, $C_3$-$C_7$-cycloalkyle-$C_1$-$C_3$-alkylène-O-C(O)-, $C_1$-$C_6$-fluoroalkyle-O-C(O)-, aryle-O-C(O)- , hétéroaryl-O-C(O)- , lorsque $R_3$ est porté par un atome d'azote ;

$R_4$ et $R_5$, représentent, indépendamment l'un de l'autre, un atome d'hydrogène ou un groupe $C_1$-$C_6$-alkyle, $C_3$-$C_7$-cycloalkyle, $C_3$-$C_7$-cycloalkyle-$C_1$-$C_3$-alkylène, aryle-$C_1$-$C_5$-alkylène ou aryle ;
ou $R_4$ et $R_5$ forment ensemble, avec l'atome d'azote qui les porte, un groupe azétidine, pyrrolidine, pipéridine, azépine, morpholine, thiomorpholine, pipérazine, homopipérazine, ce groupe étant éventuellement substitué par un groupe $C_1$-$C_6$-alkyle, $C_3$-$C_7$-cycloalkyle, $C_3$-$C_7$-cycloalkyle-$C_1$-$C_3$-alkylène, aryle-$C_1$-$C_6$-alkylè-

ne, aryle, hétéroaryle, aryle-$S(O)_2$-, $C_1$-$C_6$-alkyle-$S(O)_2$-, $C_1$-$C_6$-fluoroalkyle-$S(O)_2$-, $C_3$-$C_7$-cycloalkyle-S$(O)_2$-, $C_3$-$C_7$-cycloalkyle-$C_1$-$C_3$-alkylène-$S(O)_2$-, aryle-C(O)-, $C_1$-$C_6$-alkyle-C(O)-, $C_3$-$C_7$-cycloalkyle-C(O)-, $C_3$-$C_7$-cycloalkyle-$C_1$-$C_3$-alkylène-C(O)-, $C_1$-$C_6$-fluoroalkyle-C(O)-;

$R_6$ et $R_7$ représentent, indépendamment l'un de l'autre, un atome d'hydrogène, un groupe $C_1$-$C_6$-alkyle, $C_3$-$C_7$-cycloalkyle, $C_3$-$C_7$-cycloalkyle-$C_1$-$C_3$-alkylène-, aryle-$C_1$-$C_6$-alkylène- ou aryle ;

$R_8$ représente un groupe $C_1$-$C_6$-alkyle, $C_3$-$C_7$-cycloalkyle, $C_3$-$C_7$-cycloalkyle-$C_1$-$C_3$-alkylène-, aryle-$C_1$-$C_6$-alkylène- ou aryle.

Parmi les composés de l'invention, un dixième groupe de composés est constitué par les composés de formule générale (II') dans laquelle

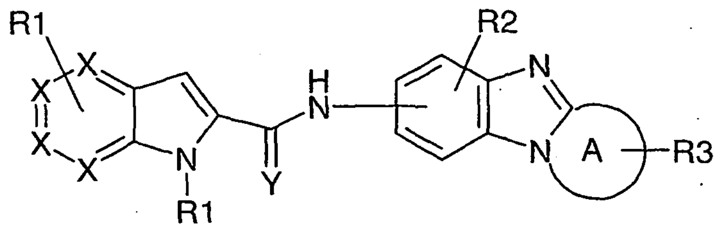

(II')

dans laquelle

X représente un atome de carbone ou un atome d'azote ; lesdits X étant identiques ou différents entre eux et le nombre de X = N n'étant pas supérieur à 2 ;

$R_1$, $R_2$, $R_3$, Y et A étant tels que définis dans la formule générale (I) ; $R_1$ pouvant être lié au motif à 5 ou 6 éléments du bicycle.

Parmi les composés de l'invention, un onzième groupe de composés est constitué par les composés de formule (III')

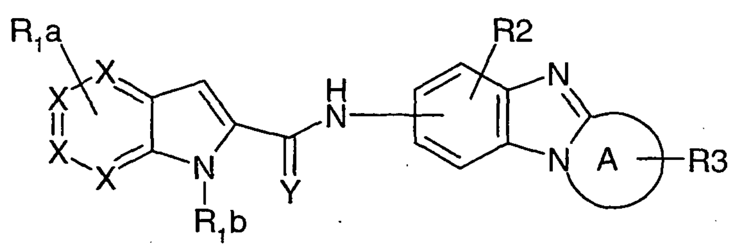

(III')

dans laquelle

$R_{1a}$ représente un ou plusieurs atomes ou groupes, identiques ou différents, choisis parmi un atome d'hydrogène, un atome d'halogène ou un groupe $C_1$-$C_6$-fluoroalkyle ;

$R_{1b}$ représente un atome d'hydrogène, un groupe $C_1$-$C_6$-alkyle, aryle, hétéroaryle, aryl-$C_1$-$C_6$-alkylène- ou hétéroaryl-$C_1$-$C_6$-alkylène-, lesdits groupes étant éventuellement substitués par un ou plusieurs groupes ou atomes, identiques ou différents, choisis parmi un atome d'halogène ou un groupe $C_1$-$C_6$-alkyle;

$R_2$, $R_3$, A, X et Y étant tels que définis dans la formule générale (II).

Parmi les composés de l'invention, un douzième groupe de composés est constitué par les composés de formule générale (IV') :

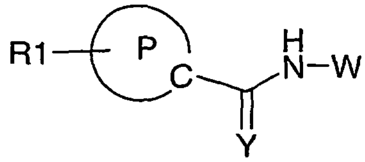

(IV')

dans laquelle W est un hétérocycle tricyclique ou un hétéroaryle tricyclique choisi parmi :

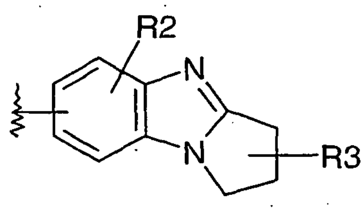

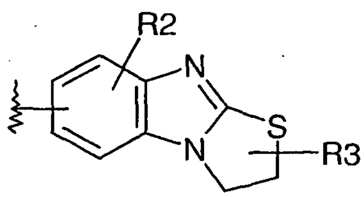

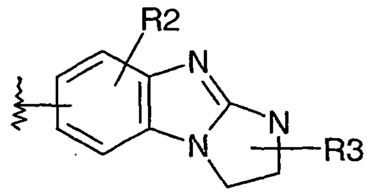

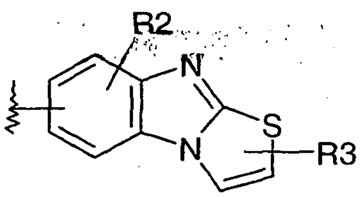

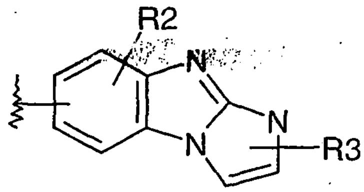

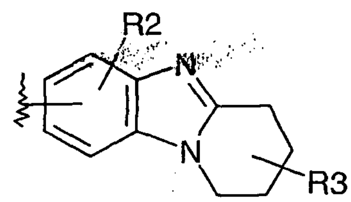

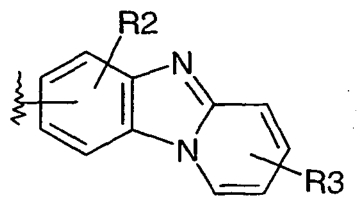

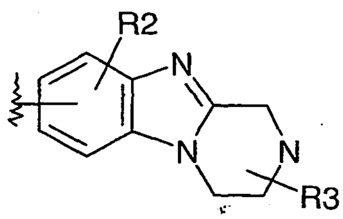

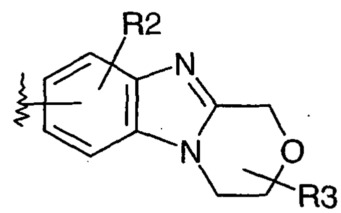

$R_1$, $R_2$, $R_3$, P et Y étant tels que définis dans la formule générale (I).

Parmi les composés de l'invention, un treizième groupe de composés est constitué par les composés de formule générale (V')

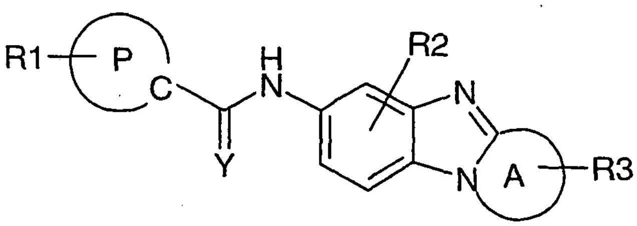

(V')

dans laquelle

$R_1$, $R_2$, $R_3$, A, P et Y sont tels que définis dans la formule générale (I).

Parmi les composés de l'invention, un quatorzième groupe de composés est constitué par les composés de formule générale (V') dans laquelle

$R_2$ et $R_3$ représentent, indépendamment l'un de l'autre, un atome d'hydrogène ou un groupe $C_1$-$C_6$-alkyle ;

$R_1$, A, P et Y étant tels que définis dans la formule générale (I).

Parmi les composés de l'invention, un quinzième groupe de composés est constitué par les composés de formule générale (I) dans laquelle à la fois $R_1$ et/ou $R_2$ et/ou $R_3$, et/ou A, et/ou P et/ou Y sont tels que définis dans les groupes 9 à 14 ci-dessus.

On entend par groupe partant, dans ce qui suit, un groupe pouvant être facilement clivé d'une molécule par rupture d'une liaison hétérolytique, avec départ d'une paire électronique. Ce groupe peut ainsi être remplacé facilement par un autre groupe lors d'une réaction de substitution par exemple. De tels groupes partants sont par exemple, les halogènes ou un groupe hydroxy activé tel qu'un méthanesulfonate, benzènesulfonate, p-toluènesulfonate, triflate, acétate, etc. Des exemples de groupes partants ainsi que des références pour leur préparation sont donnés dans « Advances in Organic Chemistry », J. March, 5th Edition, Wiley Interscience, 2001.

On entend par groupe protecteur, dans ce qui suit, un groupe pouvant être momentanément incorporé à une structure chimique dans le but d'inactiver temporairement une partie de la molécule lors d'une réaction et qui peut être facilement être enlevé à une étape ultérieure de la synthèse. Des exemples de groupes protecteurs ainsi que des références concernant leurs propriétés sont donnés dans T.W. Greene, P.G.M. Wutz, 3rd Edition, Wiley Interscience 1999.

Conformément à l'invention, on peut préparer les composés de formule générale (I) selon le procédé illustré par le schéma général 1 qui suit :

Schéma 1

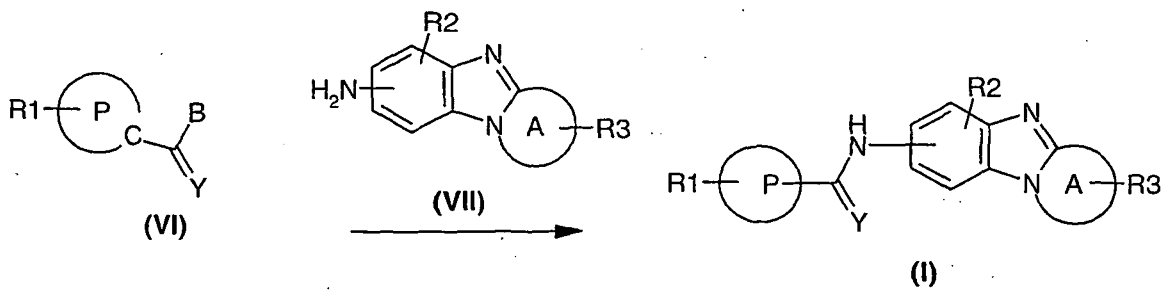

Les composés (I) peuvent être obtenus par réaction d'un composé de formule générale (VI) dans laquelle B représente un groupe $C_1$-$C_6$-alcoxy, $C_3$-$C_7$-cycloalkyloxy, $C_3$-$C_7$-cycloalkyle-$C_1$-$C_3$-alkylénoxy, aryle-$C_1$-$C_3$-alkylénoxy, et P, Y et $R_1$ sont tels que définis dans la formule générale (I), avec un amidure du composé de formule générale (VII), dans laquelle A, $R_2$ et $R_3$ sont tels que définis dans la formule générale (I) ci-dessus, au reflux d'un solvant tel que le toluène. L'amidure d'aluminium du composé de formule générale (VII) est préparé par action préalable du triméthylaluminium sur les amines de formule générale (VII).

En partant de composés de formule générale (VI), dans laquelle B représente un groupe hydroxyle, et P, Y et $R_1$ sont tels que définis dans la formule générale (I), la fonction acide carboxylique peut préalablement être transformée en halogénure d'acide tel qu'un chlorure d'acide par action, par exemple, du chlorure de thionyle, au reflux d'un solvant tel que le dichlorométhane ou le dichloroéthane. Le composé de formule générale (I) est alors obtenu par réaction des composés de formule générale (VI), dans laquelle B représente un atome de chlore, et P, Y et $R_1$ sont tels que définis dans la formule générale (I), avec le composé de formule générale (VII), dans laquelle A, $R_2$ et $R_3$ sont tels que définis dans la formule générale (I) ci-dessus, en présence d'une base telle que la triéthylamine ou le carbonate de sodium.

Alternativement, les composés de formule générale (VI), dans laquelle B représente un groupe hydroxyle, et P, Y et $R_1$ sont tels que définis dans la formule générale (I), peuvent être couplés avec les composés de formule générale (VII) en présence d'un agent de couplage tel qu'un dialkylcarbodiimide, l'hexafluorophosphate de [(benzotriazol-1-yl)oxy][tris (pyrrolidino)]phosphonium, le diéthylcyanophosphonate ou tout autre agent de couplage connu de l'homme du métier, en présence d'une base comme la triéthylamine, dans un solvant tel que par exemple le diméthylformamide.

Dans le schéma 1, les composés de formule générale (VI) et (VII) et les autres réactifs, quand leur mode de préparation n'est pas décrit, sont disponibles dans le commerce, décrits dans la littérature ou préparés par analogie à des procédés décrits dans la littérature (R.S. Begunov et al Russian J. Org. Chem. 2004, 40(11), 1740-1742 ; V.M. Reddy et al J. Indian Chem. Soc. 1984, (111), 89-91; K.V.B. Rao et al Eur.J. Med. Chem. 1981, 16(1), 35-38 ; R.J. North et al J.Het.Chem. 1969, 6, 655; A.R. Freedman et al J.Het.Chem., 1966, 3(3), 257 ; Mullock, E.B. J.Chem. Soc. Section C, 1970, (6), 829 - 833) par exemple.

Les composés de formule générale (VI) ou (I), C-substitués sur un groupe aryle ou hétéroaryle par un groupe alkyle, peuvent être obtenus par une réaction de couplage, catalysée par un métal tel que le palladium ou le fer, réalisée sur les composés de formules générales (VI) ou (I) correspondants, substitués par un atome d'halogène, tel qu'un chlore, en présence par exemple d'un halogénure d'alkylmagnésium ou d'un halogénure d'alkylzinc, selon les méthodes décrites

dans la littérature (A. Furstner et al J Am Chem Soc 2002, 124(46), 13856 ; G. Quéguiner et al J Org Chem 1998, 63 (9), 2892) par exemple, ou connues de l'homme du métier.

Les composés de formule générale (VI) ou (I), C-substitués sur un groupe aryle ou hétéroaryle par un groupe cyano, aryle ou hétéroaryle, peuvent être obtenus par une réaction de couplage, catalysée par un métal tel que le palladium, réalisée sur les composés de formule générale (VI) ou (I) correspondants, substitués, par exemple, par un atome de brome, en présence de cyanure de triméthylsilyle, d'acide arylboronique ou d'acide hétéroarylboronique, ou par toute autre méthode décrite dans la littérature ou connue de l'homme du métier.

Les composés de formule générale (VI) ou (I), dans laquelle P est N-substitué par un substituant $R_1$ correspondant à un groupe aryle ou hétéroaryle, peuvent être obtenus par une réaction de coupage, catalysée par un métal tel que le cuivre, réalisée sur les amines de formule générale (VI) ou (I) correspondante, en présence d'un halogénure d'aryle ou d'hétéroaryle, selon la méthode de Buchwald (S.L. Buchwald et al J Am Chem Soc 2002, 124, 11684), ou par toute autre méthode décrite dans la littérature ou connue de l'homme du métier.

Les composés de formule générale (I) ou (VI) C-substitués sur un groupe aryle ou hétéroaryle par un groupe $NR_4R_5$, $NR_6COR_7$ ou $NR_6SO_2R_8$, peuvent être obtenus à partir des composés de formule générale (I) ou (VI) correspondants, substitués, par exemple, par un atome de brome, par réaction de couplage respectivement avec une amine, un amide ou une sulfonamide en présence d'une base, d'une phosphine et d'un catalyseur à base de palladium, selon des méthodes décrites dans la littérature ou connues de l'homme du métier.

Les composés de formule générale (I) ou (VI) substitués par un groupe $C(O)NR_4R_5$, peuvent être obtenus à partir des composés de formule générale (I) ou (VI) correspondants substitués par un groupe cyano, selon des méthodes décrites dans la littérature ou connues de l'homme du métier.

Les composés de formule générale (I) ou (VI) substitués par un groupe -S(O)-alkyle ou-$S(O)_2$-alkyle, peuvent être obtenus par oxydation des composés de formule générale (VI) ou (I) correspondants, substitués par un groupe thioalkyle, selon des méthodes décrites dans la littérature ou connues de l'homme du métier.

Les composés de formule générale (VI) ou (I), substitués par un groupe $NR_4R_5$, $NR_6COR_7$ ou $NR_6SO_2R_8$, peuvent être obtenus à partir des composés de formule générale (VI) ou (I) correspondants, substitués par un groupe nitro, par exemple par réduction, puis acylation ou sulfonylation, selon des méthodes décrites dans la littérature ou connues de l'homme du métier.

Les composés de formule générale (VI) ou (I), substitués par un groupe $SO_2NR_4R_5$ peuvent être obtenus par une méthode analogue à celle décrite dans Pharmazie 1990, 45, 346, ou selon des méthodes décrites dans la littérature ou connues de l'homme du métier.

Les composés de formule générale (1) ou (VI) dans laquelle Y représente un atome de soufre peuvent, par exemple, être obtenus par réaction des composés de formule générale (I) ou (VI) correspondants dans lesquels Y représente un atome d'oxygène, avec un réactif tel que le réactif de Lawesson.

Les composés de formule générale (I) pour lesquelles $R_3$ correspond à un groupe protecteur porté par un azote, tel qu'un groupe éthoxycarbonyle, terbutyloxycarbonyle ou un groupe benzyloxycarbonyle, peuvent être déprotégés, selon des méthodes chimiques connues de l'homme de l'art, pour conduire à des composés de formule générale (I) où $R_3$ est un atome d'hydrogène.

L'invention, selon un autre de ses aspects, a également pour objet les composés de formule (VII-x) avec x allant de a à n. Ces composés sont utiles comme intermédiaires de synthèse des composés de formule (I).

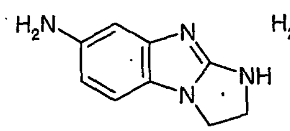
(VII-a)

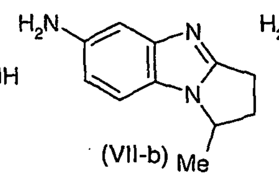
(VII-b)

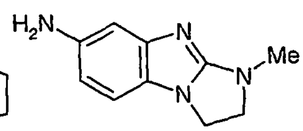
(VII-c)

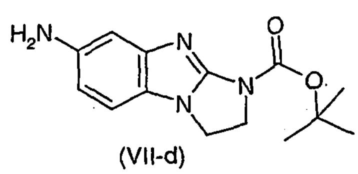
(VII-d)

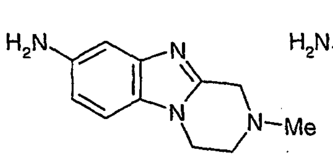
(VII-e)

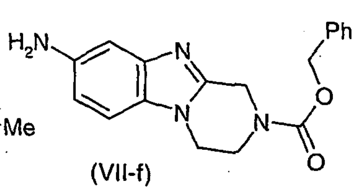
(VII-f)

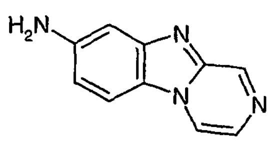
(VII-g)

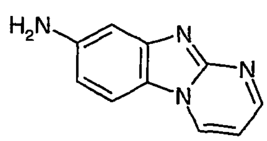
(VII-h)

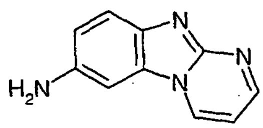
(VII-i)
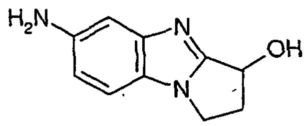
(VII-j)
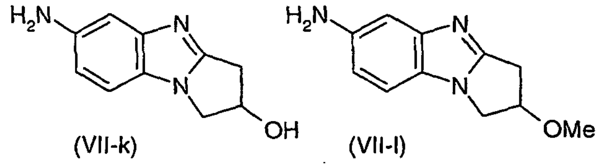
(VII-k) (VII-l)

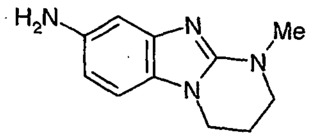
(VII-m)
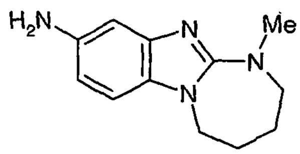
(VII-n)

Les amines (V)I-b), (VII-j), (VII-k) et (VII-l) peuvent être préparées selon le schéma 2 ci-après :

Schéma 2

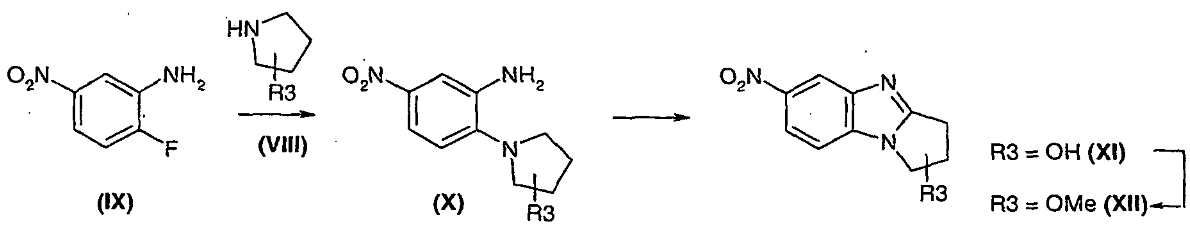

(IX) (X)

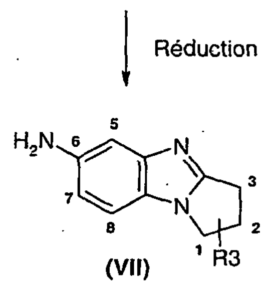


| N° | R3 |
|---|---|
| **VII-b** | 1-Me |
| **VII-j** | 3-OH |
| **VII-k** | 2-OH |
| **VII-l** | 2-OMe |

(VII)

Un procédé permettant de préparer les amines (VII-b), (VII-j), (VH-k) et (VII-l), sous leurs formes racémiques est résumé dans le schéma 2. Il consiste, dans une première étape, à substituer la 2-fluoro-5-nitroaniline (IX) par l'amine racémique (VIII). Cette réaction s'effectue, par exemple, en chauffant sans solvant les deux réactifs. Les produits d'addition (X), ainsi obtenus, sont ensuite cyclisés en hétérocycles (XI) en présence d'un réactif tel que l'acide trifluoroacétique et d'un réactif tel que le peroxyde d'hydrogène, la réaction étant réalisée en solution dans un solvant tel que le dichlorométhane (par optimisation d'un procédé décrit dans A.R. Freedman et al J.Het.Chem., 1966, 3(3), 257). Dans le cas ou $R_3$ est différent d'un atome d'hydrogène, la cyclisation peux conduire à un mélange de régioisomères.
Le composé (XI) ($R_3$ = hydroxyle) peut être transformé en composé (XII) avec $R_3$ = méthoxy, par exemple par alkylation en présence d'une base telle que l'hydrure de sodium et d'un agent alkylant tel que l'iodure de méthyle ou le diméthylsulfate. Le groupe nitro des composés (XI) ou (XII) est ensuite réduit pour donner les amines (VII-b), (VII-j), (VII-k) ou (VII-l), par hydrogénation catalytique en présence d'un catalyseur tel que le palladium sur charbon ou par toute autre méthode, de réduction d'un groupe nitro en amine, connue de l'homme du métier.

Les amines (VII-e) et (VII-f) peuvent être préparées selon le schéma 3 ci-après :

Schéma 3

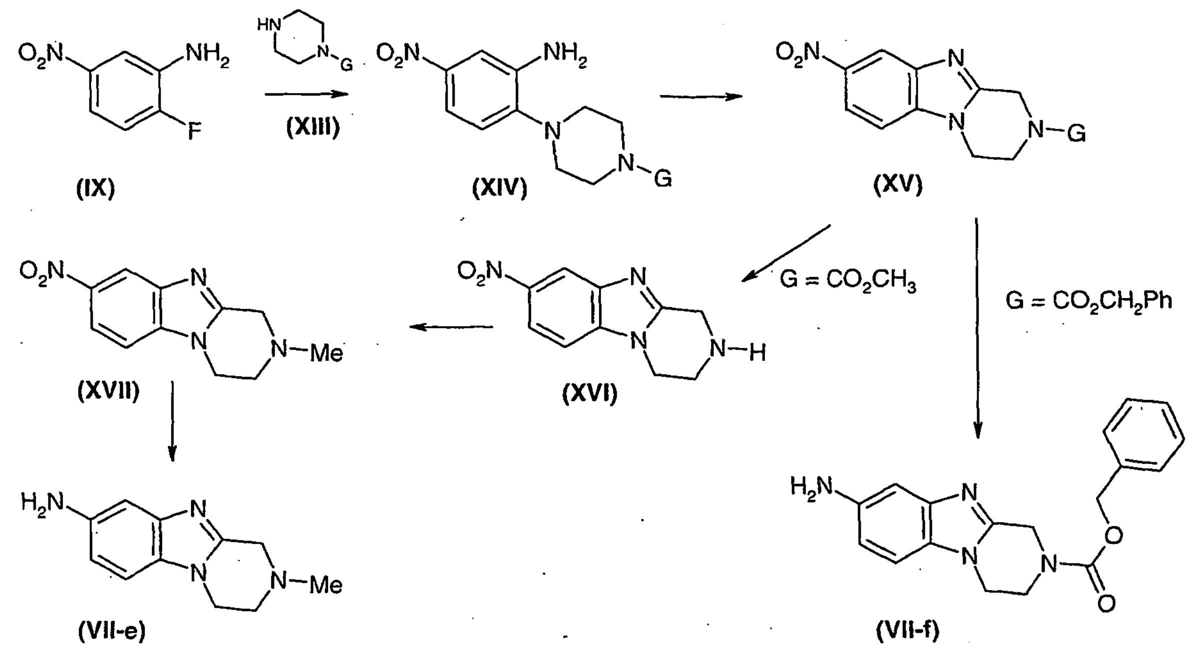


Un procédé permettant de préparer les amines (VII-e) et (VII-f) consiste, dans une première étape, à substituer la 2-fluoro-5-nitroaniline (IX) par la pipérazine (XIII), substituée sur l'un des azotes par un groupe protecteur G. Cette réaction s'effectue, par exemple, en chauffant sans solvant les deux réactifs. Le produit d'addition (XIV), ainsi obtenu, est ensuite cyclisé en composé hétérocyclique (XV) en présence d'un réactif tel que l'acide trifluoroacétique ou l'acide formique et d'un réactif tel que le peroxyde d'hydrogène, la réaction étant réalisée en solution dans un solvant tel que le dichlorométhane. Le composé hétérocyclique (XV) peut alors être déprotégé ; par exemple, si G = $CO_2CH_3$, l'hydrolyse de (XV) en présence d'un acide tel que l'acide chlorhydrique conduit à l'amine (XVI) qui peut être alkylée, par exemple en présence d'une base telle que l'hydrure de sodium et d'un agent d'alkylation tel que l'iodure de méthyle, dans un solvant tel que le tétrahydrofurane ou le diméthylformamide, pour conduire à l'hétérocycle (XVII). Le groupe nitro du composé (XVII) est ensuite réduit pour donner l'amine (VII-e) par hydrogénation catalytique en présence d'un catalyseur tel que le palladium sur charbon ou par toute autre méthode de réduction d'un groupe nitro en amine, connue de l'homme du métier. L'amine (VII-f) est préparée en réduisant le groupe nitro du composé (XV), par exemple si G = $CO_2CH_2Ph$ par réaction avec un réactif tel que le chlorure d'étain, dans un solvant tel que le diméthylformamide.

Les amines (VII-a), (VII-c) et (VII-d) peuvent être préparées selon le schéma 4 ci-après :

Schéma 4

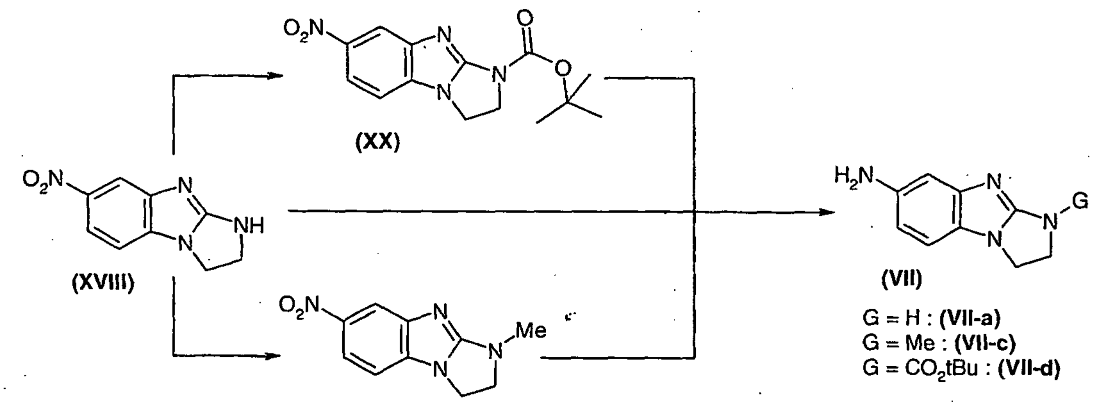


(XX) (XVIII) (XIX) (VII)

L'amine (VII-a) peut être préparée par réduction d'un précurseur nitro (XVIII) correspondant, décrit dans la littérature (R.J. North et al J.Het.Chem. 1969, 6, 655), par exemple, par hydrogénation catalytique en présence d'un catalyseur tel que le palladium sur charbon ou selon toute autre méthode, de réduction d'un groupe nitro en amine, connue de l'homme du métier.

L'amine (VII-c) peut être préparée en deux étapes à partir du précurseur nitro (XVIII) décrit ci-dessus. On prépare l'intermédiaire (XIX), à partir du précurseur (XVIII), en utilisant une base telle que l'hydrure de sodium et d'un agent d'alkylation tel que l'iodure de méthyle, dans un solvant tel que le tétrahydrofurane ou le diméthylformamide. Le composé (XIX), ainsi obtenu, peut ensuite être transformé en amine (VII-c) par hydrogénation catalytique en présence d'un catalyseur tel que le palladium sur charbon ou par toute autre méthode, de réduction d'un groupe nitro en amine, connue de l'homme du métier.

L'amine (VII-d) peut être préparée en deux étapes à partir du précurseur nitro (XVIII) décrit ci-dessus. On prépare l'intermédiaire (XX), à partir du précurseur (XVIII), en utilisant une base telle que l'hydrure de sodium et le dicarbonate de diterbutyle. Le composé (XX), ainsi obtenu, peut ensuite être transformé en amine (VII-d) par hydrogénation catalytique en présence d'un catalyseur tel que le palladium sur charbon ou par toute autre méthode, de réduction d'un groupe nitro en amine, connue de l'homme du métier.

L'amine (VII-g) peut être préparée selon le schéma 5 ci-après :

Schéma 5

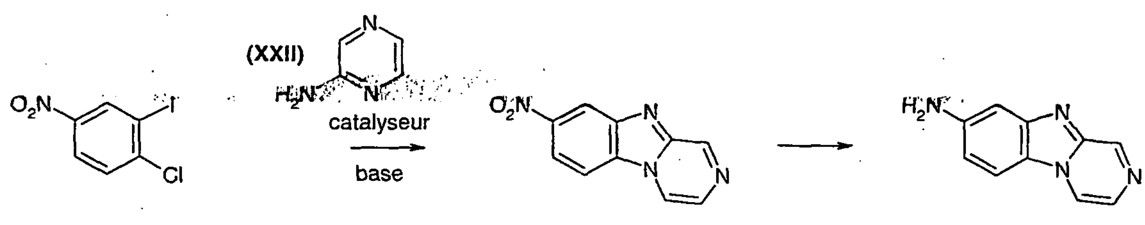


(XXII) (XXI) (XXIII) (VII-g)

L'amine (VII-g) peut être préparée en deux étapes à partir du 2-iodo-4-nitro-chlorobenzène (XXI) (G.A.Olah, J.Org.Chem. 1993 (58) 3194 - 3195). Dans un premier temps, le composé (XXI) réagit avec l'aminopyrazine (XXII) en présence d'un catalyseur comme le diacétate de palladium, d'une phosphine et d'une base telle que le carbonate de césium, dans un solvant tel que le toluène. Dans ces conditions, on isole le produit de cyclisation (XXIII), qui peut finalement conduire à l'amine (VII-g), par hydrogénation catalytique en présence d'un catalyseur tel que le palladium sur charbon ou selon toute autre méthode, de réduction d'un groupe nitro en amine, connue de l'homme du métier.

Les amines (VII-h) et (VII-i) peuvent être préparées selon le schéma 6 ci-après :

Schéma 6

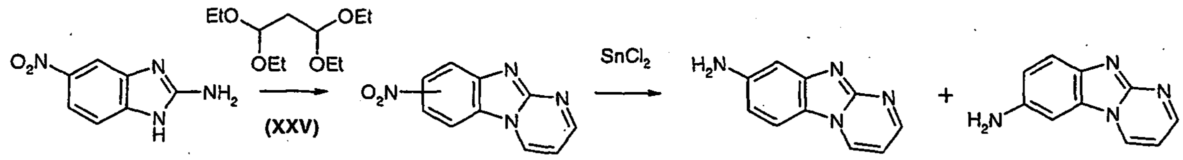


(XXIV) (XXV) (XXVI) (VII-h) (VII-i)

La condensation du 2-amino-5-nitrobenzimidazole (XXIV) avec un réactif tel que le diacétal (XXV) permet d'isoler un produit de cyclisation (XXVI), mélange de deux isomères. Le groupe nitro de l'hétérocycle (XXVI) peut être réduit en groupe amine par réaction avec le chlorure d'étain ou selon toute autre méthode, de réduction d'un groupe nitro en amine, connue de l'homme du métier. On accède ainsi aux amines (VII-h) et (VII-i).

Les amines (VII-m) et (VII-n) peuvent être préparées selon le schéma 7 ci-après :

Schéma 7

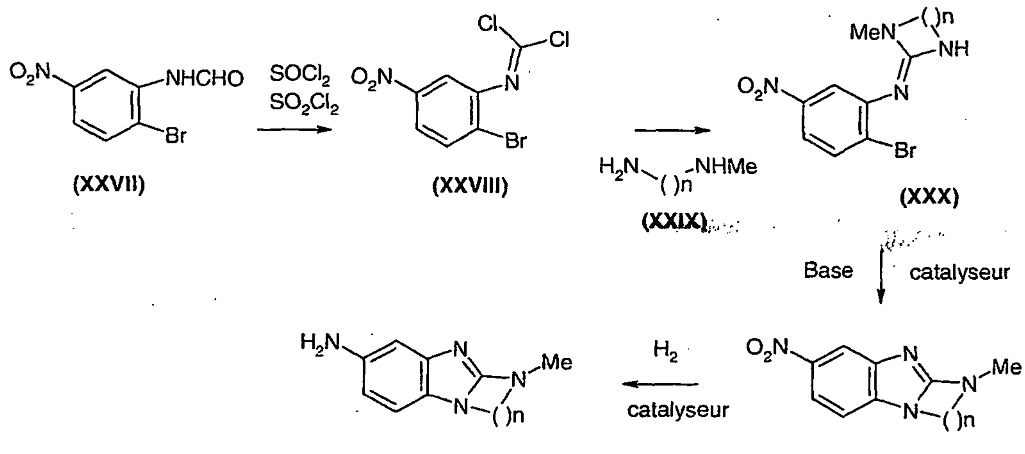


(XXVII) (XXVIII) (XXIX) (XXX) (XXXI)

n = 3 : (VII-m)
n = 4 : (VII-n)

Les amines (VII-m) et (VII-n) peuvent être préparées en quatre étapes à partir du formamide (XXVII) (S.L. Chupak, US2006135447). Le formamide (XXVII) peut être activé sous forme de dichloroimine (XXVIII) par réaction avec un mélange de $SOCl_2$ et de $SO_2Cl_2$. Le composé (XXVIII) peut ensuite être substitué par une diamine de structure (XXIX), dans laquelle n = 3 ou 4, pour conduire à un composé de structure (XXX). Ce dernier peut ensuite être transformé en benzimidazole tricyclique (XXXI) en présence d'un catalyseur tel que le diacétate de palladium, d'une phosphine et d'une base telle que le carbonate de césium. La réduction du groupe nitro de (XXXI) par hydrogénation catalytique en présence d'un catalyseur tel que le palladium sur charbon ou selon toute autre méthode, de réduction d'un groupe nitro en amine, connue de l'homme du métier, permet d'accéder aux amines (VII-m) ou (VII-n).

Le tableau qui suit illustre les structures chimiques et les propriétés physiques de quelques exemples de composés intermédiaires de formule (VII) selon l'invention.

Dans ce tableau :

- la colonne « PF (°C) » renseigne les points de fusion des produits en degrés Celsius (°C), à défaut « RMN » signale que le spectre RMN est décrit dans les exemples ;
- dans la colonne « Sel/base », « - » représente un composé sous forme de base libre, alors que « HCl » représente un composé sous forme de chlorhydrate et le rapport entre parenthèses est le rapport (acide:base)

**Tableau 1**

| N° | structure | Sel /Base | PF (°C) |
|---|---|---|---|
| VII-a | 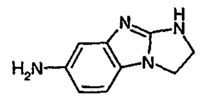 | - | 235 - 236 |
| VII-b | 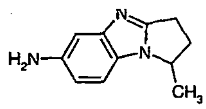 | - | 168-172 |
| VII-c | 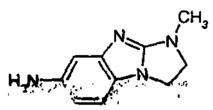 | - | 195-190 |
| VII-d | 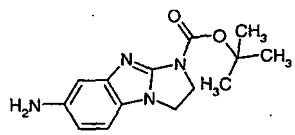 | - | 198-200 |
| VII-e | 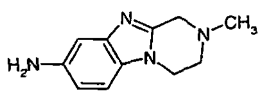 | - | RMN (exemple V) |
| VII-f | 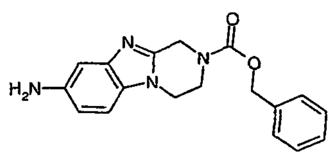 | - | 115-118 |
| VII-g | 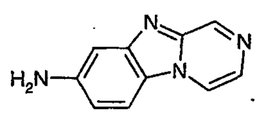 | - | 249 - 253 |
| VII-h | 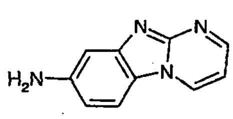 | - | RMN (Exemple VIII-2) |
| VII-i | 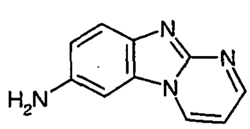 | - | RMN (Exemple VIII-3) |
| VII-j | 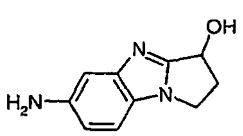 | - | RMN (Exemple X-3) |
| VII-k | 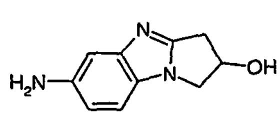 | HCl (1:1) | RMN (Exemple X-4) |

(suite)

| N° | structure | Sel /Base | PF (°C) |
|---|---|---|---|
| VII-l | 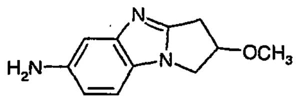 | - | 140 - 142 |
| VII-m | 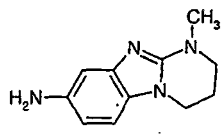 | HCl (1:1) | 335 - 340 335-340 |
| VII-n | 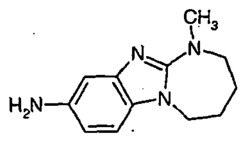 | HCl (2:1) | 330 - 336 |

Les exemples suivants décrivent la préparation de certains composés intermédiaires de formule (VII) conformes à l'invention. Les numéros des composés exemplifiés renvoient à ceux du tableau 1. Les microanalyses élémentaires, les analyses LC_MS (chromatographie liquide couplée à la spectrométrie de masse) les spectres I.R. ou R.M.N. confirment les structures des composés obtenus.

**<u>Exemple I</u> (Composé N°VII-a)**

6-amino-2,3-dihydro-1*H*-imidazo[1,2-*a*]benzimidazole

On ajoute sous argon, à une solution fortement agitée de 2,7 g (13,22 mmoles) de 2,3-dihydro-6-nitro-1*H*imidazo [1,2-*a*]benzimidazole (R.J. North et al J.HetChem. 1969, 6, 655) dans 100 mL de méthanol, 1 g de palladium sur charbon à 10 %. On ajoute, au mélange réactionnel, 15 g (0,23 mole) de formiate d'ammonium et la suspension est ensuite agitée pendant 12 h. On recueille par filtration un solide qui est purifié par chromatographie sur colonne de silice (éluant : dichlorométhane - méthanol). On obtient 0,82 g du produit attendu sous forme d'un solide gris après séchage sous pression réduite.
Point de fusion (base) : 235-236 °C
R.M.N. $^1$H (DMSO $D_6$), $\delta$ (ppm): 3,89 (m, 4H) ; 4,38 (m, 2H) ; 6,18 (dxd, 1H) ; 6,46 (d, 1H) ; 6,58 (s, 1H) ; 6,73 (d, 1H);

**<u>Exemple II</u> (Composé N°VII-b)**

6-amino-2,3-dihydro-1-méthyl-1*H*-pyrrolo[1,2-*a*]benzimidazole

II-1 2-(2-méthylpyrrolidin-1-yl)-5-nitroaniline

On ajoute, dans un réacteur à vis, 5 g (32,03 mmoles) de 2-fluoro-5-nitroaniline et 4,9 mL (48,04 mmoles) de 2-méthyl-pyrrolidine (racémique). Le réacteur est fermé et le mélange est chauffé à 100°C pendant 5 h. Après refroidissement, le mélange est repris avec 100 mL d'eau et 100 mL de dichlorométhane. La phase organique est séparée, lavée avec 50 mL d'une solution saturée en chlorure de sodium, séchée sur sulfate de magnésium puis concentrée sous pression réduite. Le produit résultant est utilisé tel quel dans la suite de la réaction.

II-2 2,3-dihydro-1-méthyl-6-nitro-1*H*-pyrrolo[1,2-*a*]benzimidazole

On ajoute, goutte à goutte, 2 mL (22,6 mmoles) d'une solution aqueuse de peroxyde d'hydrogène à 35% sur une solution de 1 g (4,52 mmoles) de 2-(2-méthyl-pyrrolidin-1-yl)-5-nitroaniline, obtenue à l'étape précédente, et de 4 mL (51,92 mmoles) d'acide trifluoroacétique dans 20 mL de dichlorométhane. Le mélange est chauffé 30 min à reflux puis refroidi et repris dans 100 mL de dichlorométhane et 100 mL d'une solution aqueuse saturée en hydrogénocarbonate de sodium. La phase organique est séparée, lavée avec 50 mL d'une solution saturée en chlorure de sodium, séchée sur sulfate de magnésium puis concentrée sous pression réduite. L'huile orange résultante (0,89 g) est utilisée telle quelle dans la suite de la réaction.

II-3 6-amino-2,3-dihydro-1-méthyl-1*H*-pyrrolo[1,2-*a*]benzimidazole (VIIb)

Un mélange de 0,25 g (1,15 mmole) de 2,3-dihydro-1-méthyl-6-nitro-1*H*-pyrrolo[1,2-*a*]benzimidazole, obtenu à l'étape précédente, et de 10 mg de palladium sur charbon à 10%, en suspension dans 15 mL d'éthanol et 1 mL d'une solution aqueuse d'acide chlorhydrique 1N, est agité quatre heures à température ambiante sous 4 bars d'hydrogène. Après ce temps, le mélange est filtré, concentré sous pression réduite, repris par 100 mL de dichlorométhane et 100 mL d'une solution aqueuse de soude à 30%. La phase organique est séparée, lavée avec 50 mL d'une solution saturée en chlorure de sodium, séchée sur sulfate de magnésium puis concentrée sous pression réduite. Le solide résultant est purifié par chromatographie sur colonne de silice (éluant : dichlorométhane - méthanol). On obtient 0,133 g du produit attendu sous forme d'un solide beige après séchage sous pression réduite.
Point de fusion (base) : 168 -172°C
R.M.N. $^1$H (DMSO $D_6$), δ (ppm): 1,49 (d, 3H) ; 2,16 (m, 1H) ; 2,75 (m, 1H) ; 2,9 (m, 2H) ; 3,2 (m, 2H) ; 4,41 (sext., 1H) ; 6,51 (dxd, 1H), 6,92 (d, 1H) ; 7,08 (d, 1H).

**<u>Exemple III</u> : 6-amino-2,3-dihydro-3-méthyl-1H-imidazo[1,2-*a*]benzimidazole (Composé N°VII-c)**

III-1 2,3-dihydro-3-méthyl-6-nitro-1*H*-imidazo[1,2-*a*]benzimidazole (Composé XIX)

On ajoute sous argon, à une suspension agitée à 20°C, de 0,023 g (0,59 mmole) d'hydrure de sodium à 60% dans 5 mL de tétrahydrofurane, 0,1 g (0,49 mmole) de 2,3-dihydro-6-nitro-1*H*-imidazo[1,2-*a*]benzimidazole (R.J. North et al J.Het.Chem. 1969, 6, 655) dans 10 mL de de tétrahydrofurane. Après 45 minutes d'agitation à 20°C, on ajoute 30 μL (0,54 mmole) d'iodométhane puis on agite 12 heures à 20°C. Après ce temps, le mélange réactionnel est versé sur 100 mL d'eau puis extrait avec trois fois 30 mL de dichlorométhane. Les phases organiques sont réunies, séchées sur sulfate de sodium, puis concentrées sous pression réduite. On obtient ainsi 0,1 g du produit (XIX) attendu sous la forme d'un solide jaune qui sera engagé tel quel dans la suite de la synthèse. Point de fusion (base) : 208 - 209°C

II-2 6-amino-2,3-dihydro-3-méthyl-1*H*-imidazo[1,2-*a*]benzimidazole (Composé N° VII-c)

On ajoute sous argon, à une solution fortement agitée de 0,81 g (3,71 mmoles) de composé (XIX), préparé à l'étape précédente, dans 70 mL de méthanol, 0,5 g de palladium sur charbon à 10%. On ajoute, au mélange réactionnel, 10 g (0,153 mole) de formiate d'ammonium et la suspension est ensuite agitée pendant 12 h. On recueille par filtration un solide qui est purifié par chromatographie sur colonne de silice (éluant : dichlorométhane - méthanol). On obtient 0,26 g du produit attendu sous forme d'un solide gris après séchage sous pression réduite.
Point de fusion (base) : 185 - 190 °C
R.M.N. $^1$H (DMSO $D_6$), δ (ppm): 2,84 (s, 3H) ; 3,76 (m, 2H) ; 3,91 (m, 2H) ; 4,41 (s, $NH_2$) ; 6,19 (dxd, 1H) ; 6,48 (d, 1H) ; 7,71 (d, 1H).

**<u>Exemple IV</u> : 6-amino-2,3-dihydro-3-terbutoxycarbonyl-1*H*-imidazo[1,2-*a*]benzimidazole**

**(Composé N°VII-d)**

IV-1 6-nitro-2,3-dihydro-3-terbutoxycarbonyl-1*H*-imidazo[1,2-*a*]benzimidazole (Composé N° XX):

On ajoute en plusieurs fois, à une suspension agitée à 20°C sous argon de 3,47 g (86,8 mmoles) d'hydrure de sodium à 60% dans 482 mL de tétrahydrofurane, 13,9 g (57,86 mmoles) de 2,3-dihydro-6-nitro-1*H*-imidazo[1,2-*a*] benzimidazole (R.J. North et al J.Het.Chem. 1969, 6, 655). Après 2 heures d'agitation à 20°C, on ajoute 15,1 mL (65,73 mmoles) de dicarbonate de diterbutyle puis on agite 1 h30 à 20°C. Après ce temps, le mélange réactionnel est versé sur 50 mL d'eau. On recueille par filtration un précipité qui est séché sous pression réduite. On obtient ainsi 14,31 g du produit (XX) attendu sous la forme d'un solide jaune qui sera engagé tel quel dans la suite de la synthèse.
Point de fusion (base) : 222 - 224°C

IV-2 6-amino-2,3-dihydro-3-terbutoxycarbonyl-1*H*-imidazo[1,2-*a*]benzimidazole (Composé N° VII-d):

Une suspension de 0,1 g (0,33 mmole) de composé (XX) et de 0,5 g de palladium sur charbon à 10% dans 150 mL d'éthanol est agitée à 20°C sous 5 bars d'hydrogène durant quatre heures. Après élimination des insolubles par filtration, la solution éthanolique résultante est concentrée sous pression réduite. On obtient ainsi 70 mg du produit attendu sous la forme d'un solide blanc.
Point de fusion (base) : 198 - 200 °C

R.M.N. $^1H$ ($CDCl_3$), δ (ppm): 1,51 (s, 9H) ; 2,25-3,55 (pic élargi, $NH_2$) ; 4,01 (m, 2H) ; 4,32 (m, 2H) ; 6,48 (dxd, 1H) ; 6,83 (d, 1H) ; 6,91 (d, 1H)

**Exemple V : 7-amino-3-méthyl-1,2,3,4-tétrahydropyrazino[1,2-*a*]benzimidazole (Composé N°VII-e)**

V-1 2-[4-(éthoxycarbonyl)pyrazin-1yl]-5-nitroaniline (Composé N° XIV, G = $CO_2Et$) :

On chauffe 12h à 140°C un mélange de 10 g (64,06 mmoles) de 2-fluoro-5-nitroaniline et de 20,08 mL (137,72 mmoles) de *N*-(éthoxycarbonyl)pipérazine. Après ce temps, le mélange est concentré sous pression réduite puis 5 mL d'acide acétique sont additionnés. On recueille par filtration un précipité qui est lavé à l'eau puis séché sous pression réduite. On isole ainsi 18,5 g du produit attendu sous la forme d'un solide jaune.
R.M.N. $^1H$ (DMSO $D_6$), δ (ppm): 1,15 (t, 3H) ; 2,82 (m, 4H) ; 3,51 (m, 4H) ; 4,02 (q, 2H) ; 5,35 (s, $NH_2$) ; 6,95 (d, 1H) ; 7,38 (dxd, 1H) ; 7,5 (d, 1H).

V-2 3-éthoxycarbonyl-7-nitro-1,2,3,4-tétrahydropyrazino[1,2-*a*]benzimidazole (Composé N° XV, G = $CO_2Et$) :

On ajoute, goutte à goutte, 15 mL (169,5 mmoles) d'une solution aqueuse de peroxyde d'hydrogène à 35% sur une solution de 17 g (57,76 mmoles) du composé (XIV) obtenu à l'étape précédente, et de 60 mL (51,92 mmoles) d'acide formique. Le mélange est chauffé une heure à 50°C puis refroidi, concentré sous pression réduite, et repris dans 200 mL de dichlorométhane et 300 mL d'une solution aqueuse saturée en bicarbonate de potassium. La phase organique est séparée, lavée avec 100 mL d'une solution saturée en chlorure de sodium, séchée sur sulfate de sodium puis concentrée sous pression réduite. La mousse résultante est purifiée par chromatographie sur colonne de silice en éluant avec un mélange de dichlorométhane et de méthanol. On isole ainsi 3,9 g du composé attendu sous la forme d'un solide qui sera utilisé tel quel dans la suite de la synthèse.

V-3 7-nitro -1,2,3,4-tétrahydropyrazino[1,2-*a*]benzimidazole (Composé N° XVI)

On chauffe à reflux durant 12 heures, une solution de 3,9 g (13,44 mmoles) de composé (XV), obtenu à l'étape précédente, dans 100 mL d'acide chlorhydrique 6N. Après ce temps, la solution refroidie est amenée à pH > 9 par ajouts successifs de soude concentrée puis extraite trois fois avec 100 mL de dichlorométhane. Les phases organiques sont rassemblées, lavées avec 150 mL d'eau, séchées sur sulfate de sodium puis concentrées sous pression réduite. Le produit résultant est purifié par chromatographie sur colonne de silice en éluant avec un mélange de dichlorométhane et de méthanol. On isole ainsi 0,3 g du composé attendu sous la forme d'un solide qui sera utilisé tel quel dans la suite de la synthèse.
R.M.N. $^1H$ ($CDCl_3$), δ (ppm): 1,91 (1 pic élargi, NH) ; 5,56 (m, 2H) ; 4,28 (m, 2H) ; 4,48 (s, 2H) ; 7,5 (d, 1H) ; 8,31 (dxd, 1H) ; 8,72 (d, 1H).

V-4. 3-méthyl-7-nitro-1,2,3,4-tétrahydropyrazino[1,2-*a*]benzimidazole (Composé N° XVII)

On ajoute sous argon, à une suspension agitée à 20°C, de 0,07 g (1,65 mmole) d'hydrure de sodium à 60% dans 15 mL de tétrahydrofurane, 0,3 g (1,37 mmole) de composé (XVI), obtenu à l'étape précédente, dans 20 μL (1,65 mmole) d'iodométhane puis on agite 12 heures à 20°C. Après ce temps, le mélange réactionnel est versé sur 100 mL d'eau puis extrait avec trois fois 30 mL de dichlorométhane. Les phases organiques sont réunies, séchées sur sulfate de sodium, puis concentrées sous pression réduite. On obtient ainsi 0,13 g du produit (XVII) attendu qui sera utilisé tel quel dans la suite de la synthèse.
R.M.N. $^1H$ ($CDCl_3$), δ (ppm) : 2,49 (s, 3H) ; 2,93 (m, 2H) ; 3,82 (s, 2H) ; 4,11 (m, 2H) ; 7,29 (d, 1H) ; 8,09 (dxd, 1H) ; 8,49 (d, 1H).

V-5 7-amino-3-méthyl-1,2,3,4-tétrahydropyrazino[1,2-*a*]benzimidazole (Composé N° VII-e).

On agite 12 heures à 20°C une suspension de 0,13 g (0,56 mmole) de composé (XVII) obtenu à l'étape précédente, de 0,2 g de palladium sur charbon à 10% et de 5 g (79,36 mmoles) de formiate d'ammonium. Après élimination des insolubles par filtration, le filtrat est concentré sous pression réduite. On obtient ainsi 80 mg du produit attendu sous la forme d'une huile.
R.M.N. $^1H$ ($CDCl_3$), δ (ppm) : 2,48 (s, 3H) ; 2,89 (m, 2H) ; 3,75 (s, 2H) ; 3,85 (pic élargi, $NH_2$) ; 3,97 (m, 2H) ; 6,57 (dxd, 1H) ; 6,91 (d, 1H) ; 7 (d, 1H).

**Exemple VI: 7-amino-3-benzyloxycarbonyl-1,2,3,4-tétrahydropyrazino[1,2-*a*]benzimidazole (Composé N° VII-f)**

VI-1 2-[4-(benzyloxycarbonyl)pyrazin-1-yl]-5-nitroaniline (Composé N° XIV, G = $CO_2CH_2Ph$) :

On chauffe 12h à 140°C un mélange de 9,44 g (60,5 mmoles) de 2-fluoro-5-nitroaniline et de 24,98 mL (130,08 mmoles) de *N*-(benzyloxycarbonyl)pipérazine. Après ce temps, le mélange est repris dans 200 mL de dichlorométhane. La phase organique est successivement lavée avec une solution aqueuse saturée en carbonate de potassium, trois fois avec 50 mL d'eau puis est séchée sur sulfate de magnésium et concentrée sous pression réduite. Le résidu obtenu est purifié par chromatographie sur colonne de silice en éluant avec un mélange de dichlorométhane et d'acétate d'éthyle. On isole ainsi 8,6 g du produit attendu.
R.M.N. $^1H$ ($CDCl_3$), δ (ppm): 2,91 (m, 4H) ; 3,68 (m, 4H) ; 4,16 (s, $NH_2$) ; 5,18 (s, 2H) ; 6,92 (d, 1H) ; 7,35 (m, 5H) ; 7,6 (m, 2H).

VI-2 3-benzyloxycarbonyl-7-nitro-1,2,3,4-tétrahydropyrazino[1,2-*a*]benzimidazole (Composé N° XV, G = $CO_2CH_2Ph$):

On ajoute, goutte à goutte en deux heures, 17,7 mL (0,2 mole) d'une solution aqueuse de peroxyde d'hydrogène à 35% sur une solution, agitée à 0°C, de 8,6 g (24,13 mmoles) de 2-[4-(benzyloxycarbonyl)pyrazin-1-yl]-5-nitroaniline obtenue à l'étape précédente et de 34,4 mL (29,4 mmoles) d'acide formique. Le mélange est agité durant 3 heures à 0°C, durant 12 heures à 20°C puis est versé sur 300 g de glace. Le pH du milieu est ajusté à 8, par ajouts d'une solution aqueuse d'ammoniaque, puis extrait avec 200 mL de dichlorométhane. La phase organique est lavée avec 100 mL d'une solution saturée en chlorure de sodium, séchée sur sulfate de magnésium puis concentrée sous pression réduite. On obtient un résidu qui est purifié par chromatographie sur colonne de silice en éluant avec un mélange de dichlorométhane et d'acétate d'éthyle. On isole ainsi 1,2 g du produit attendu.
R.M.N. $^1H$ ($CDCl_3$), δ (ppm): 4,11 (m, 2H) ; 4,21 (m, 2H) ; 5,05 (s, 2H) ; 5,18 (s, 2H) ; 7,38 (m, 6H) ; 8,21 (dxd, 1H) ; 8,61 (d, 1H).

VI-3 7-amino-3-benzyloxycarbonyl-1,2,3,4-tétrahydropyrazino[1,2-*a*]benzimidazole (Composé N° VII-f)

On ajoute, en plusieurs fois, 7,68 g (34,06 mmoles) de chlorure d'étain dihydrate à une solution, dans 56 mL de diméthylformamide, de 2 g (5,68 mmoles) de composé (XV), obtenu à l'étape précédente, et de 2,6 g (8,85 mmoles) de bromure de tétrabutylammonium. Le mélange est agité 24 heures à 20°C puis concentré et repris avec 100 mL d'eau. Le pH de la solution ainsi obtenue est ajusté à 9 par ajouts d'une solution d'ammoniaque. Le mélange est ensuite extrait deux fois, avec 100 mL d'acétate d'éthyle. Les phases organiques sont réunies, lavées deux fois avec 50 mL d'eau et une fois avec 50 mL d'une solution saturée en chlorure de sodium, séchées sur sulfate de magnésium puis concentrées sous pression réduite. On obtient un résidu qui est purifié par chromatographie sur colonne de silice en éluant avec un mélange de dichlorométhane et de méthanol. On isole ainsi 1,45 g du produit attendu.
PF = 115 -118°C
R.M.N. $^1H$ ($CDCl_3$), δ (ppm): 3,89 (m, 2H) ; 4 (m, 2H) ; 4,7 (m, 4H) ; 5,12 (s, 2H) ; 6,51 (dxd, 1H) ; 6,71 (d, 1H) ; 7,12 (d, 1H) ; 7,38 (m, 5H).

**Exemple VII : 7-aminopyrazino[1,2-*a*]benzimidazole (Composé N° VII-g)**

VII-1 7-nitropyrazino[1,2-*a*]benzimidazole (Composé N° XXIII):

On chauffe à 120°C durant 12 heures un mélange de 0,6 g (2,12 mmoles) de 2-iodo-4-nitro-chlorobenzène (XXI) (G.A.Olah, J.Org.Chem. 1993 (58) 3194 - 3195), de 0,2 g (2,1 mmoles) de 2-aminopyrazine (XXII), de 2,8 g (8,5 mmoles) de carbonate de césium, de 0,15 g (0,25 mmole) de Xantphos® (9,9-diméthyl-4,5-bis(diphénylphosphino) xanthène) et de 30 mg (0,13 mmole) de diacétate de palladium dans 20 mL de toluène sec. Après ce temps, le mélange est concentré sous pression réduite puis repris dans 350 mL de dichlorométhane et 200 mL d'eau. La phase organique est séparée, lavée avec 100 mL d'une solution saturée en chlorure de sodium, séchée sur sulfate de magnésium puis concentrée sous pression réduite. Le produit résultant est repris dans 10 mL de dichlorométhane, on isole 0,23 g du produit attendu sous la forme d'un précipité que l'on recueille par filtration. Le filtrat est purifié par chromatographie sur colonne de silice en éluant avec un mélange d'acétate d'éthyle et d'heptane. On isole ainsi 25 mg de produit supplémentaire.

VII-2 7-aminopyrazino[1,2-*a*]benzimidazole (Composé N° VII-g)

On agite 2 heures à 20°C, une suspension de 0,22 g (1,03 mmole) de 7-nitro-pyrazino[1,2-*a*]benzimidazole,

obtenu à l'étape précédente, de 0,5 g de Nickel de Raney et de 0,1 mL d'hydrazine monohydrate (2,05 mmoles) dans 100 mL d'éthanol. On élimine par filtration les insolubles et le filtrat est concentré sous pression réduite. On obtient ainsi 0,16 g de produit attendu sous la forme d'un solide beige.
PF = 249 - 253 °C
R.M.N. $^1$H (DMSO $D_6$), δ (ppm) : 5,27 (pic élargi, $NH_2$) ; 6,72 (m, 2H) ; 7,71 (d, 1H) ; 7,89 (d, 1H) ; 8,73 (d, 1H) ; 8,89 (s, 1H).

**Exemples VIII : 7-aminopyrimido[1,2-*a*]benzimidazole (Composé N° VII-h) et 8-aminopyrimido[1,2-*a*]benzimidazole (Composé N° VII-i)**

VIII-1 chlorhydrate de 7-nitropyrimido[1,2-*a*]benzimidazole et de 8-nitropyrimido[1,2-*a*]benzimidazole (Composés N° XXVI)

On chauffe à refiux durant 12 heures un mélange de 2 g (11,2 mmoles) de 2-amino-5-nitrobenzimidazole (J.Med.Chem. 1995, 38 (20), 4098 - 4105) et de 3,6 mL (15 mmoles) de 1,1,3,3-tétraéthoxypropane dans 50 mL d'éthanol et 2 mL d'acide chlorhydrique concentré. On recueille par filtration 1,97 g d'un précipité beige que l'on sèche sous pression réduite. On obtient un mélange d'isomères que l'on utilise tel quel dans l'étape suivante.

VIII-2 7-aminopyrimido[1,2-*a*]benzimidazole (Composé N° VII-h)

On ajoute 4,5 g (19,95 mmoles) de chlorure d'étain dihydrate dans 4 mL d'eau sur une suspension, chauffée à 100°C, de 1 g (3,99 mmoles) du mélange de composés (XXVI), préparé à l'étape précédente, dans 8 mL d'acide chlorhydrique concentré. Le mélange réactionnel est chauffé 3 heures puis refroidi et le pH du milieu est ajusté à 8 par ajouts successifs d'une solution d'ammoniaque à 30%. Le mélange est extrait trois fois avec 100 mL de dichlorométhane. Les phases organiques sont réunies, séchées sur sulfate de magnésium, concentrées sous pression réduite puis purifiées par chromatographie sur colonne de silice en éluant avec un mélange de dichlorométhane et de méthanol. On isole ainsi le 7-aminopyrimido[1,2-*a*]benzimidazole sous la forme d'un solide orange. R.M.N. $^1$H (DMSO $D_6$), δ (ppm) : 5,21 (m, $NH_2$) 6,7 (d, 1H) ; 6,81 (s, 1H) ; 6,95 (m, 1H) ; 7,9 (d, 1H) ; 7,58 (m, 1H) ; 9,25 (dxd, 1H).

VIII-3 8-aminopyrimido[1,2-*a*]benzimidazole (Composé N° VII-i)

Lors de l'extraction décrite dans l'exemple VIII-2, un insoluble précipite dans la phase aqueuse. On recueille cet insoluble par filtration sur un fritté, ce qui permet d'isoler 0,13 g d'un solide orange correspondant au composé n° VII-i. R.M.N. $^1$H (DMSO $D_6$), δ (ppm) : 5,21 (m, $NH_2$) ; 6,91 (m, 2H) ; 7,2 (d, 1H) ; 7,51 (d, 1H) ; 8,57 (m, 1H) ; 9,12 (dxd, 1H).

**Exemple IX : 6-amino-2,3-dihydro-3-hydroxy-1*H*-pyrrolo[1,2-*a*]benzimidazole (Composé N° VII-j) et chlorhydrate de 6-amino-2,3-dihydro-2-hydroxy-1*H* pyrrolo[1,2-*a*]benzimidazole (Composés N° VII-k)**

IX-1 2-(3-hydroxypyrrolidin-1-yl)-5-nitroaniline

Selon un procédé similaire à celui décrit à l'exemple II-1, en partant de 3 g (19,22 mmoles) de 2-fluoro-5-nitroaniline et de 1,76 mL (21,14 mmoles) de 3-hydroxypyrrolidine, on obtient 2 g de composé attendu sous la forme d'un solide jaune.

IX-2 2,3-dihydro-3-hydroxy-6-nitro-1*H*-pyrrolo[1,2-*a*]benzimidazole et 2,3-dihydro-2-hydroxy-6-nitro-1*H*-pyrrolo[1,2-*a*] benzimidazole

Selon un procédé similaire à celui décrit à l'exemple II-2, en partant de 2 g (7,7 mmoles) de 2-(3-hydroxypyrrolidin-1-yl)-5-nitroaniline, obtenue à l'étape précédente, on obtient, après purification par chromatographie sur colonne de silice, 48 mg d'un solide jaune correspondant au 2,3-dihydro-3-hydroxy-6-nitro-1*H*-pyrrolo[1,2-*a*]benzimidazole et 280 mg d'un solide jaune correspondant au 2,3-dihydro-2-hydroxy-6-nitro-1*H*-pyrrolo[1,2-*a*]benzimidazole.

IX-3 6-amino-2,3-dihydro-3-hydroxy-1*H*-pyrrolo[1,2-*a*]benzimidazole (Composé N° VII-j)

Selon un procédé similaire à celui décrit à l'exemple VII-2, en partant de 0,13 g (0,56 mmole) de 2,3-dihydro-3-hydroxy-6-nitro- 1*H*-pyrrolo[1,2-*a*]benzimidazole, préparé à l'étape IX-2, on isole 0,1 g de l'amine attendue sous la forme d'un solide marron (le mélange réactionnel est agité 2 jours à 20°C dans ce cas).
R.M.N. $^1$H ($CDCl_3$), δ (ppm) : 2,61 (m, 1H) ; 2,92 (m, 1H) ; 3,94 (m, 1H) ; 4,18 (m, 1H) ; 5,29 (m, 1H) ; 6,61 (d, 1H) ; 6,98 (s, 1H) ; 7,05 (d, 1H).

IX-4 Chlorhydrate de 6-amino-2,3-dihydro-2-hydroxy-1*H*-pyrrolo[1,2-*a*]benzimidazole (Composés N° VII-k)

Un mélange de 0,3 g (1,36 mmole) du 2,3-dihydro-2-hydroxy-6-nitro-1*H*-pyrrolo[1,2-*a*]benzimidazole obtenu à l'étape IX-2, et de 100 mg de palladium sur charbon à 10% dans 15 mL d'éthanol et 2 mL d'acide chlorhydrique 1 N est agité heures à 20°C sous 4 bars d'hydrogène. Après ce temps le mélange réactionnel est filtré sur un tampon de célite, le filtrat est concentré sous pression réduite et on obtient 0,35 g du produit attendu sous la forme d'un solide.
R.M.N. $^1$H (DMSO $D_6$), δ (ppm) : 3,05 (m, 1H) ; 3,55 (dxd, 1H) ; 4,15 (m, 1H) ; 4,45 (dxd, 1H) ; 5,1 (m, 1H) ; 7,0 (m, 1H) ; 7,3 (m, 1H) ; 7,66 (m, 1H).

**<u>Exemple X</u> : 6-amino-2,3-dihydro-2-méthoxy-1*H*-pyrrolo[1,2-*a*]benzimidazole (Composé N° VII-l)**

X-1 2,3-dihydro-2-méthoxy-6-nitro-1*H*-pyrrolo[1,2-*a*]benzimidazole

On ajoute, à une suspension, agitée sous argon à 20°C, de 0,2 g (5 mmoles) d'hydrure de sodium à 60% dans 10 mL de tétrahydrofurane, une solution de 0,28 g (1,28 mmole) de 2,3-dihydro-2-hydroxy-6-nitro-1*H*-pyrrolo[1,2-*a*] benzimidazole (étape IX-2 de l'exemple IX) dans 5 mL de tétrahydrofurane. Après quinze minutes d'agitation, on ajoute 0,24 mL (2,5 mmoles) de diméthylsulfate. Le mélange réactionnel est agité 12 heures à 20°C puis versé sur 100 mL d'eau et extrait trois fois avec 50 mL d'acétate d'éthyle. Les phases organiques sont réunies, lavées avec une solution aqueuse saturée en chlorure de sodium, séchées sur sulfate de magnésium et concentrées sous pression réduite. Le résidu obtenu est purifié par chromatographie sur colonne de silice en éluant avec un mélange de dichlorométhane et de méthanol. On isole ainsi 70 mg du produit attendu sous la forme d'une mousse.

X-2 6-amino-2,3-dihydro-2-méthoxy-1*H*-pyrrolo[1,2-*a*]benzimidazole (Composé N° VII-l)

Selon un procédé similaire à celui décrit à l'exemple VII-2, en partant de 0,185 g (0,79 mmole) de 2,3-dihydro-2-méthoxy-6-nitro-1*H*-pyrrolo[1,2-*a*]benzimidazole, on obtient, après purification par chromatographie sur colonne de silice, 60 mg d'un solide beige correspondant au produit attendu.
PF=140-142°C
R.M.N. $^1$H ($CDCl_3$), δ (ppm) : 3,01 (dxd, 1H) ; 3,25 (dxd, 1H) ; 3,38 (s, 3H) ; 3,94 (dxd, 1H) ; 4,19 (dxd, 1H) ; 4,62 (m, 1H) ; 6,57 (dxd, 1H) ; 6,95 (d, 1H) ; 7,02 (d, 1H).

**<u>Exemple XI</u> : Chlorhydrate (1:1) de 7-amino-4-méthyl-1,2,3,4-tétrahydropyrimido[1,2-*a*]benzimidazole (Composé N° VII-m)**

XI-1 dichlorure de *N*-(2-bromo-5-nitrophényl)formimidoyle (XXVIII)

On chauffe 24 heures à 60°C, une solution de 5 g (20,4 mmoles) de *N*-(2-bromo-5-nitrophényl)formamide (XXVII), dans 12 mL (164 mmoles) de chlorure de thionyle et 4,5 mL (56 mmoles) de chlorure de sulfuryle. Le mélange réactionnel est concentré sous pression réduite et on obtient 5,3 g d'un solide gris qui sera utilisé tel quel dans la suite de la synthèse.

XI-2 *N*-(2-bromo-5-nitrophényl)-*N*-(1-méthyl-1*H*-3,4,5,6-tétrahydropyrimidin-2-ylidène)amine (XXX)

On ajoute, en plusieurs fois à une solution agitée à 0°C de 10,5 mL (0,1 mole) de N-méthyl-1,3-propanediamine (XXIX, n = 3) dans 50 mL de tétrahydrofurane, une suspension de 5,3 g (17,8 mmoles) de l'intermédiaire (XXVIII) dans 30 mL de tétrahydrofurane. Le mélange réactionnel est ensuite agité 48 heures à 20°C puis versé dans 200 mL d'eau. On extrait le mélange avec trois fois 50 mL d'acétate d'éthyle. Les phases organiques sont réunies, lavées avec 100 mL d'une solution aqueuse saturée en chlorure de sodium puis séchées sur sulfate de sodium et concentrées sous pression réduite. On purifie le produit obtenu par chromatographie sur colonne de silice en éluant avec un mélange de dichlorométhane et de méthanol. On isole ainsi 0,72 g du produit (XXIX) attendu sous la forme d'un solide jaune.

XI-3 4-méthyl-7-nitro-1,2,3,4-tétrahydropyrimido[1,2-*a*]benzimidazole (XXXI)

On chauffe 24 heures à 120°C un mélange de 0,72 g (2,3 mmoles) d'intermédiaire (XXX), de 0,16 g (0,28 mmole) de Xanphos® (9,9-diméthyl-4,5-bis(diphénylphosphino)xanthène), de 1,5 g (4,6 mmoles) de carbonate de césium et de 30 mg (0,14 mmole) de diacétate de palladium dans 20 ml de toluène. Après ce temps, le mélange est concentré sous pression réduite puis purifié par chromatographie sur colonne de silice en éluant avec un mélange de dichlorométhane et de méthanol. On isole ainsi 0,44 g du produit (XXXI) attendu.

PF = 172 - 178°C
R.M.N. $^1$H ($CDCl_3$), δ (ppm) : 2,42 (quint., 2H) ; 3,39 (s, 3H) ; 3,58 (t, 2H) ; 4,15 (t, 2H) ; 7,09 (d, 1H) ; 8,09 (dxd, 1H) ; 8,38 (d, 1H).

XI-4 Chlorhydrate (1 :1) de 7-amino-4-méthyl-1,2,3,4-tétrahydropyrimido[1,2-*a*]benzimidazole (Composé N° VII-m)

On ajoute 0,2 mL (3,8 mmoles) d'hydrazine monohydrate à une suspension de 0,44 g (1,89 mmole) de composé (XXX) et 0,3 g de nickel de Raney. Le mélange est agité 3 heures à 20°C puis filtré sur un tampon de célite, concentré sous pression réduite et purifié par chromatographie sur colonne de silice en éluant avec un mélange de dichlorométhane et de méthanol. On isole ainsi 0,36 g du produit (VII-m) attendu. Le composé peut éventuellement être isolé sous la forme d'un chlorhydrate en reprenant le produit obtenu dans 50 ml d'une solution d'acide chlorhydrique 0, 1 N dans l'isopropanol et en concentrant la solution obtenue sous pression réduite. On isole ainsi le chlorhydrate de l'amine (VII-m).
PF (HCl 1 : 1) : 335 - 340°C
R.M.N. $^1$H ($CDCl_3$), δ (ppm) : 2,17 (quint., 2H) ; 3,1 (s, 3H) ; 3,25 (t, 2H) ; 3,82 (t, 2H) ; 6,31 (dxd, 1H) ; 6,7 (d, 1H) ; 6,76 (d, 1H).

**<u>Exemple XII</u> : 8-amino-5-méthyl-1,2,3,4-tétrahydro-5-*H*-[1,3]diazépino[1,2-*a*]benzimidazole (Composé N° VII-n)**

On prépare le composé (VII-n), selon un procédé similaire à celui décrit à l'exemple (XIII), à partir des intermédiaires (XXVIII) et (XXIX, n = 4).
PF (2HCl) : 330 - 336°C
R.M.N. $^1$H ($CDCl_3$), δ (ppm) : 1,84 (m, 4H) ; 3,06 (s, 3H) ; 3,08 (m, 2H) ; 3,8 (m, 2H) ; 6,42 (dxd, 1H) ; 6,81 (d, 1H) ; 6,85 (s, 1H).

Les exemples suivants décrivent la préparation de certains composés conformes à l'invention. Ces exemples ne sont pas limitatifs et ne font qu'illustrer la présente invention. Les numéros des composés exemplifiés renvoient à ceux des tableaux 2, 3 et 4. Les microanalyses élémentaires, les analyses LC-MS (chromatographie liquide couplée à la spectrométrie de masse) les spectres I.R. ou R.M.N. confirment les structures des composés obtenus.

**<u>Exemple 1</u> (Composé N°1 du tableau 2)**

*N*-(1,2,3,4-tétrahydropyrido[1,2-*a*]benzimidazol-7-yl)-5-fluoro-1-[(pyridin-4-yl)méthyl]-1*H*-indole-2-carboxamide

1.1. 5-fluoro-1-[(pyridin-4-yl)méthyl]-1*H*-indole-2-carboxylate d'éthyle

On ajoute sous argon à 20°C, au goutte à goutte, une solution de 5,5 g (21,72 mmoles) de 1,1'-azodicarbonyldipipéridine, en solution dans 15 mL de toluène sec, à une solution de 3 g (14,48 mmoles) de 5-fluoro-1*H*-indole-2-carboxylate d'éthyle, 2,37 g (21,72 mmoles) de 4-pyridylcarbinol et 5,45 mL (21,72 mmoles) de n-tributylphosphine dans 200 mL de toluène. Le mélange est agité 48h à 20°C. Le mélange réactionnel est ensuite concentré sous pression réduite. On purifie le résidu par chromatographie sur colonne de silice (éluant : heptane / acétate d'éthyle). On isole ainsi 3,2 g du produit attendu utilisé tel quel ,dans la suite de la synthèse.

1.2 *N*-(1,2,3,4-tétrahydropyrido[1,2-*a*]benzimidazol-7-yl)-5-fluoro-1-[(pyridin-4-yl)méthyl]-1*H*-indole-2-carboxamide (composé n°1)

On ajoute sous argon, à une solution de 0,376 g (2,01 mmoles) de 7-amino-1,2,3,4-tétrahydropyrido[1,2-*a*] benzimidazole (Saunders et al., J.Chem.Soc. 1955, 3275 - 3287) dans 70 mL de toluène sec, 1,26 mL de triméthylaluminium (2M dans le toluène). Après 15 min d'agitation à 0°C, on porte le mélange à 50°C et on maintient l'agitation durant 30 min. Le mélange est ensuite refroidi à 0°C. On ajoute 0,5 g (1,68 mmole) de 5-fluoro-1-[(pyridin-4-yl)méthyl]-1*H*-indole-2-carboxylate d'éthyle, obtenu à l'étape 1.1. On porte à reflux le mélange réactionnel pendant 12 h puis on le refroidit et on l'ajoute à 20 mL d'une solution glacée d'acide chlorhydrique 1 N. Le pH de la phase aqueuse est ajusté à 9. Un insoluble, recueillit par filtration, est purifié par chromatographie sur colonne de silice (éluant : dichlorométhane - méthanol). Le produit ainsi purifié est recristallisé dans un mélange de dichlorométhane et d'heptane. On obtient 0,41 g d'un solide blanc après séchage sous pression réduite.
Point de fusion (base) : 292 - 293 °C
R.M.N. $^1$H (DMSO $D_6$), δ (ppm): 1,91 (m, 4H), 2,89 (t, 2H), 4,02 (t, 2H) ; 5,9 (s, 2H) ; 6,93 (d, 2H) ; 7,09 (txd, 1H) ; 7,5 (m, 5H) ; 7,89 (d, 1H) ; 8,4 (d, 2H) ; 10,35 (s, 1H) ;

**<u>Exemple 2</u> (Composé N°2 du tableau 2)**

*N*-(1,2,3,4-tétrahydropyrido[1,2-*a*]benzimidazol-7-yl)-5-fluoro-1-(3-fluorobenzyl)-1*H*-indole-2carboxamide

2.1 5-fluoro-1-(3-fluorobenzyl)-1*H*-indole-2-carboxylate d'éthyle

On agite une suspension de 1,035 g (5 mmoles) de 5-fluoro-1*H*-indole-2-carboxylate d'éthyle, 0,865 g (6 mmoles) de chlorure de 3-fluorobenzyle et 1,38 g (10 mmoles) de carbonate de potassium dans 50 mL de diméthylformamide pendant 24 h à 60°C. On refroidit ensuite le mélange réactionnel, on le verse dans un mélange d'eau glacée et d'acétate d'éthyle. Après décantation, on sépare la phase organique puis on la lave avec deux fois 200 mL d'eau puis avec 200 mL d'une solution saturée en chlorure de sodium. On sèche la solution sur sulfate de magnésium, on la filtre puis on concentre le filtrat sous pression réduite. On obtient 0,97 g d'une huile utilisée telle quelle dans l'étape suivante.

2.2 *N*-(1,2,3,4-tétrahydropyrido[1,2-*a*]benzimidazol-7-yl)-5-fluoro-1-(3-fluorobenzyl)-1*H*-indole-2-carboxamide (composé n°2)

On ajoute sous argon, à une solution de 0,426 g (2,28 mmoles) de 7-amino-1,2,3,4-tétrahydropyrido[1,2-*a*] benzimidazole (Saunders et al., J.Chem.Soc. 1955, 3275 - 3287*)* dans 70 mL de toluène sec, 1,43 mL de triméthylaluminium (2M dans le toluène). Après 15 min d'agitation à 0°C, on porte le mélange à 50°C et on maintient l'agitation durant 30 min. Le mélange est ensuite refroidi à 0°C et on ajoute 0,6 g (1,9 mmole) de 5-fluoro-1-(3-fluorobenzyl)-1*H*-indole-2-carboxylate d'éthyle, obtenu à l'étape 2.1. On porte à reflux le mélange réactionnel pendant 12 h puis on le refroidit et l'ajoute à 20 mL d'une solution glacée d'acide chlorhydrique 1 N. Le pH de la phase aqueuse est ajusté à pH 9. On recueille par filtration un insoluble que l'on purifie par chromatographie sur colonne de silice (éluant : dichlorométhane - méthanol). Le produit ainsi purifié est recristallisé dans le méthanol. On obtient 0,46 g d'un solide blanc après séchage sous pression réduite.
Point de fusion (base) : 286 - 287 °C
R.M.N. $^1$H (DMSO $D_6$), δ (ppm): 2 (m, 4H), 2,9 (t, 2H), 4,05 (t, 2H) ; 5,88 (s, 2H) ; 7,11 (m, 10H) ; 7,91 (s, 1H) ; 10,38 (s, 1H).

**<u>Exemple 3</u> (Composé N°3 du tableau 2)**

*N*-(1,2,3,4-tétrahydropyrido[1,2-*a*]benzimidazol-7-yl)-1-benzyl-1*H*-pyrrolo[2,3-*b*]pyridine-2-carboxamide

3.1 1-benzyl-1*H*-pyrrolo[2,3-*b*]pyridine-2-carboxylate d'éthyle

A une suspension de 0,44 g (11 mmoles) d'hydrure de sodium dans 50 mL de DMF, agitée à 20°C, on ajoute par portions, 2 g (10 mmoles) de pyrrolo[2,3-*b*]pyridine-2-carboxylate d'éthyle (WO2004101563). Après 1h d'agitation a température ambiante, on ajoute, au goutte à goutte, 2,1 g (12 mmoles) de bromure de benzyle et on agite le mélange réactionnel 20 h à température ambiante. 150 mL d'eau et 150 mL d'éther éthylique sont ensuite ajoutés sous agitation. La phase aqueuse est séparée et extraite deux fois avec 50 mL d'éther éthylique. Les phases organiques sont rassemblées, lavées avec 100 mL d'eau, séchées sur sulfate de sodium puis concentrées sous pression réduite. Le produit résultant est purifié par chromatographie sur colonne de silice en éluant avec un mélange d'heptane et de dichlorométhane. On isole ainsi 2,3 g du produit attendu.
Point de fusion = 71 - 72°C

3.2 *N*-(1,2,3,4-tétrahydropyrido[1,2-*a*]benzimidazol-7-yl)-1-benzyl-1*H*-pyrrolo[2,3-*b*]pyridine-2-carboxamide (composé n°3)

On ajoute sous argon, à une solution de 0,32 g (1,71 mmole) de 7-amino-1,2,3,4-tétrahydropyrido[1,2-*a*]benzimidazole (Saunders et al., J.Chem.Soc. 1955, 3275 - 3287*)* dans 20 mL de toluène sec, 1,1 mL de triméthylaluminium (2M dans le toluène). Après 15 min d'agitation à 0°C, on porte le mélange à 50°C et on maintient l'agitation durant 30 min. Le mélange est ensuite refroidi à 0°C et on ajoute 0,4 g (1,43 mmole) de 1-benzyl-1*H*-pyrrolo[2,3-*b*]pyridine-2-carboxylate d'éthyle, obtenu à l'étape 3.1. On porte à reflux le mélange réactionnel pendant 6 h puis on le refroidit et on ajoute 150 mL d'eau, 2 gouttes d'une solution aqueuse de soude à 35% et 150 mL de dichlorométhane, sous agitation. La phase aqueuse est séparée et extraite deux fois avec 50 mL de dichlorométhane. Les phases organiques sont rassemblées, lavées avec 100 mL d'eau, séchées sur sulfate de sodium puis concentrées sous pression réduite. Le produit résultant est purifié par chromatographie sur colonne de silice en éluant avec un mélange de méthanol - dichlorométhane. On isole ainsi 0,55 g de produit que l'on recristallise dans l'éthanol pour obtenir, après séchage sous vide,

0,45 g du composé attendu sous la forme d'une poudre.
Point de fusion = 233 - 235 °C
R.M.N. $^1$H (DMSO $D_6$), δ (ppm): 1,91 (m, 4H), 2,91 (t, 2H), 4,02 (t, 2H) ; 5,9 (s, 2H) ; 7,12 (m, 6H) ; 7,38 (m, 2H) ; 7,48 (m, 1H), 7,9 (s, 1H), 8,18 (dxd, 1H), 8,4 (dxd, 1H).

**<u>Exemple 4</u> (Composé N°4 du tableau 2)**

*N*-(1,2,3,4-tétrahydropyrido[1,2-*a*]benzimidazol-7-yl)-1-benzyl-5-trifluorométhyl-1*H* pyrrolo[2,3-*b*]pyridine-2-carboxamide

4.1 1-benzyl-5-trifluorométhyl-1*H*-pyrrolo[2,3-*b*]pyridine-2-carboxylate d'éthyle

A une suspension de 0,9 g (3,5 mmoles) de 5-trifluorométhyl-1*H*-pyrrolo[2,3-*b*]pyridine-2-carboxylate d'éthyle (Nazare, M. et al. Angewandte Chemie, International Ed., 2004, 43(34), 4526-4528) dans 45 mL de tétrahydrofurane sec, on ajoute, sous agitation, 0,58 g (5,23 mmoles) d'alcool benzylique, 1,4 g (5,23 mmoles) de triphénylphosphine et 0,94 g (5,23 mmoles) d'azodicarboxylate de diéthyle (DEAD). Après 20 h d'agitation à température ambiante, le mélange réactionnel est concentré sous pression réduite, et le produit résultant est purifié par chromatographie sur colonne de silice en éluant avec un mélange de dichlorométhane et d'heptane. On obtient ainsi 1 g du produit attendu.
Point de fusion = 72 - 73°C

4.2 *N*-(1,2,3,4-tétrahydropyrido[1,2-*a*]benzimidazol-7-yl)-1-benzyl-5-trifluorométhyl-1*H*-pyrrolo[2,3-*b*]pyridine-2-carboxamide (composé n°4)

On ajoute sous argon, à une solution de 0,19 g (1,03 mmole) de 7-amino-1,2,3,4-tétrahydropyrido[1,2-*a*]benzimidazole (Saunders et al., J.Chem.Soc. 1955, 3275 - 3287*)* dans 20 mL de toluène sec, 0,65 mL de triméthylaluminium (2M dans le toluène). Après 15 min d'agitation à 0°C, on porte le mélange à 50°C et on maintient l'agitation durant 30 min. Le mélange est ensuite refroidi à 0°C et on ajoute 0,3 g (0,86 mmole) de 1-benzyl-5-trifluorométhyl-1*H*-pyrrolo [2,3-*b*]pyridine-2-carboxylate d'éthyle, obtenu à l'étape 4.1. On porte à reflux le mélange réactionnel pendant 6 h puis on le refroidit et on ajoute 150 mL d'eau, 2 gouttes d'une solution aqueuse de soude à 35% et 150 mL de dichlorométhane, sous agitation. La phase aqueuse est séparée et extraite deux fois avec 50 mL de dichlorométhane. Les phases organiques sont rassemblées, lavées avec 100 mL d'eau, séchées sur sulfate de sodium puis concentrées sous pression réduite. Le produit résultant est purifié par chromatographie sur colonne de silice en éluant avec un mélange de méthanol - dichlorométhane. On isole ainsi 0,4 g de produit que l'on recristallise dans l'acétonitrile pour obtenir, après séchage sous vide, 0,35 g du composé attendu sous la forme d'une poudre.
Point de fusion = 241 - 243 °C
R.M.N. $^1$H (DMSO $D_6$), δ (ppm): 1,91 (m, 4H), 2,93 (t, 2H), 4,05 (t, 2H) ; 5,98 (s, 2H) ; 7,15 (m, 5H) ; 7,42 (m, 3H) ; 7,9 (d, 1H), 8,7 (dxd, 2H), 10,5 (s, 1H).

**<u>Exemple 5</u> (Composé N°5 du tableau 2)**

*N*-(2,3-dihydro-1*H*-pyrrolo[1,2-a]benzimidazol-6-yl)-5-fluoro-1-[(pyridin-4-yl)méthyl]-1*H* indole-2-carboxamide

On ajoute sous argon, à une solution de 0,35 g (2,01 mmoles) de 6-amino-2,3-dihydro-1*H*-pyrrolo[1,2-*a*]benzimidazole (Freedman et al., J.Het.Chem. 1966, 3, (3), 257 - 259*)* dans 70 mL de toluène sec, 1,26 mL de triméthylaluminium (2M dans le toluène). Après 15 min d'agitation à 0°C, on porte le mélange à 50°C et on maintient l'agitation durant 30 min. Le mélange est refroidi à 0°C et on ajoute 0,5 g (1,68 mmole) de 5-fluoro-1-[(pyridin-4-yl)méthyl]-1*H*-indole-2-carboxylate d'éthyle, obtenu à l'étape 1.1. On porte à reflux le mélange réactionnel pendant 12 h puis on le refroidit et le verse sur 20 mL d'une solution glacée d'acide chlorhydrique 1 N. Le pH de la phase aqueuse est ajusté à 9. On recueille par filtration un insoluble que l'on purifie par chromatographie sur colonne de silice (éluant : dichlorométhane - méthanol). Le produit ainsi purifié est recristallisé dans un mélange de dichlorométhane - heptane. On obtient 0,23 g d'un solide blanc après séchage sous pression réduite.
Point de fusion (base) : 266 - 267°C
R.M.N. $^1$H (DMSO $D_6$), δ (ppm): 2,6 (m, 2H), 2,9 (t, 2H), 4,08 (t, 2H) ; 5,9 (s, 2H) ; 6,95 (d, 2H) ; 7,1 (txd, 1H) ; 7,45 (m, 5H) ; 7,92 (d, 1H) ; 8,42 (d, 2H), 10,31 (s, 1H).

**Exemple 6 (Composé N°6 du tableau 2)**

*N*-(2,3-dihydro-1*H*-pyrrolo[1,2-*a*]benzimidazol-6-yl)-5-fluoro-1-(3-fluorobenzyl)-1*H*-indole-2-carboxamide

On ajoute sous argon, à une solution de 0,39 g (2,28 mmoles) de 6-amino-2,3-dihydro-1*H*-pyrrolo[1,2-*a*]benzimidazole (Freedman et al., J.Het.Chem. 1966, 3, (3), 257-259*)* dans 70 mL de toluène sec, 1,43 mL de triméthylaluminium (2M dans le toluène). Après 15 min d'agitation à 0°C, on porte le mésange à 50°C et on maintient l'agitation durant 30 min. Le mélange est ensuite refroidi à 0°C et on ajoute 0,6 g (1,9 mmole) de 5-fluoro-1-(3-fluorobenzyl)-1*H*-indole-2-carboxylate d'éthyle, obtenu à l'étape 2.1. On porte à reflux le mélange réactionnel pendant 12 h puis on le refroidit et le verse sur 20 mL d'une solution glacée d'acide chlorhydrique 1 N. Le pH de la phase aqueuse est ajusté à 9. On recueille par filtration un insoluble que l'on purifie par chromatographie sur colonne de silice (éluant : dichlorométhane - méthanol). Le produit ainsi purifié est recristallisé dans un mélange de dichlorométhane et d'heptane. On obtient 0,45 g d'un solide blanc après séchage sous pression réduite.
Point de fusion (base) : 256-257 °C
R.M.N. $^1$H (DMSO $D_6$), δ (ppm): 2,56 (m, 2H), 2,91 (t, 2H), 4,04 (t, 2H) ; 5.9 (s, 2H) ; 7,2 (m, 10H) ; 7,92 (d, 1H) ; 10,32 (s, 1H).

**Exemple 7 (Composé N°7 du tableau 2)**

*N*-(2,3-dihydro-1*H*-pyrrolo[1,2-*a*]benzimidazol-6-yl)-1-benzyl-1*H*-pyrrolo[2,3-*b*]pyridine-2-carboxamide

On ajoute sous argon, à une solution de 0,185 g (1,07 mmole) de 6-amino-2,3-dihydro-1*H*-pyrrolo[1,2-*a*]benzimidazole (Freedman et al., J.Het.Chem. 1966, 3, (3), 257 - 259*)* dans 20 mL de toluène sec, 0,67 mL de triméthylaluminium (2M dans le toluène). Après 15 min d'agitation à 0°C, on porte le mélange à 50°C et on maintient l'agitation durant 30 min. Le mélange est ensuite refroidi à 0°C et on ajoute 0,25 g (0,89 mmole) de 1-benzyl-1*H*-pyrrolo[2,3-*b*]pyridine-2-carboxylate d'éthyle, obtenu à l'étape 3.1. On porte à reflux le mélange réactionnel pendant 6 h puis on le refroidit et on ajoute 150 mL d'eau, 2 gouttes d'une solution aqueuse de soude à 35% et 150 mL de dichlorométhane, sous agitation. La phase aqueuse est séparée et extraite deux fois avec 50 mL de dichlorométhane. Les phases organiques sont rassemblées, lavées avec 100 mL d'eau, séchées sur sulfate de sodium puis concentrées sous pression réduite. Le produit résultant est purifié par chromatographie sur colonne de silice en éluant avec un mélange de méthanol et de dichlorométhane. On isole ainsi 0,34 g de produit que l'on recristallise dans l'éthanol pour obtenir, après séchage sous vide, 0,31 g du composé attendu sous la forme d'une poudre.
Point de fusion = 251 - 252 °C .
R.M.N. $^1$H (DMSO $D_6$), δ (ppm): 2,6 (m, 2H), 2,9 (t, 2H), 4,04 (t, 2H) ; 5,9 (s, 2H) ; 7,15 (m, 9H) ; 7,9 (d, 1H), 8,18 (dxd, 1H), 8,4 (dxd, 1H).

**Exemple 8 (Composé N°8 du tableau 2)**

*N*-(2,3-dihydro-1*H*-pyrrolo[1,2-*a*]benzimidazol-6-yl)-1-benzyl-5-trifluorométhyl-1*H*-pyrrolo[2,3-*b*]pyridine-2-carboxamide

On ajoute sous argon, à une solution de 0,18 g (1,03 mmole) de 6-amino-2,3-dihydro-1*H*-pyrrolo[1,2-*a*]benzimidazole (Freedman et al., J.Het.Chem. 1966, 3, (3), 257-259*)* dans 20 mL de toluène sec, 0,65 mL de triméthylaluminium (2M dans le toluène). Après 15 min d'agitation à 0°C, on porte le mélange à 50°C et on maintient l'agitation durant 30 min. Le mélange est ensuite refroidi à 0°C et on ajoute 0,3 g (0,86 mmole) de 1-benzyl-5-trifluorométhyl-1H-pyrrolo[2,3-b]pyridine-2-carboxylate d'éthyle, obtenu à l'étape 4.1. On porte à reflux le mélange réactionnel pendant 6 h puis on le refroidit et on ajoute 150 mL d'eau, 2 gouttes d'une solution aqueuse de soude à 35% et 150 mL de dichlorométhane, sous agitation. La phase aqueuse est séparée et extraite deux fois avec 50 mL de dichlorométhane. Les phases organiques sont rassemblées, lavées avec 100 mL d'eau, séchées sur sulfate de sodium puis concentrées sous pression réduite. Le produit résultant est purifié par chromatographie sur colonne de silice en éluant avec un mélange de méthanol - dichlorométhane. On isole ainsi 0,37 g de produit que l'on recristallise dans l'acétonitrile pour obtenir, après séchage sous vide, 0,3 g du composé attendu sous la forme d'une poudre.
Point de fusion = 268-270 °C
R.M.N. $^1$H (DMSO $D_6$), δ (ppm): 2,6 (m, 2H), 2,9 (t, 2H), 4,04 (t, 2H) ; 6 (s, 2H) ; 7,12 (m, 5H) ; 7,42 (m, 3H) ; 7,92 (s, 1H), 8,7 (d, 2H).

**Exemple 9 (Composé N°148 du tableau 3)**

*N*-(pyrido[1,2-*a*]benzimidazol-7-yl)-5-fluoro-1-(3-fluorobenzyl)-1*H*-indole-2-carboxamide On ajoute sous argon, à une solution de 0,348 g (1,9 mmole) de 7-amino-pyrido[1,2-a]benzimidazole (Begunov et al., Russian J. Org. Chem. 2004, (40), 11, 1694-1696*)* dans 70 mL de toluène sec, 1,19 mL de triméthylaluminium (2M dans le toluène). Après 15 min d'agitation à 0°C, on porte le mélange à 50°C et on maintient l'agitation durant 30 min. Le mélange est ensuite refroidi à 0°C et on ajoute 0,5 g (1,9 mmole) de 5-fluoro-1-(3-fluorobenzyl)-1*H*-indole-2-carboxylate d'éthyle, obtenu à l'étape 2.1. On porte à reflux le mélange réactionnel pendant 12 h puis on le refroidit et l'ajoute à 20 mL d'une solution glacée d'acide chlorhydrique 1 N. Le pH de la phase aqueuse est ajusté à 9. On recueille par filtration un insoluble que l'on purifie par chromatographie préparative (éluant : dichlorométhane - méthanol). Le produit ainsi purifié est recristallisé dans le méthanol. On obtient 0,53 g du produit attendu sous forme d'un solide blanc après séchage sous pression réduite.
Point de fusion (base) : 261 - 262 °C
R.M.N. $^1$H (DMSO $D_6$), δ (ppm): 5,9 (s, 2H) ; 7,01 (m, 6H) ; 7,55 (m, 6H) ; 8,2 (d, 2H) ; 9,0 (s, 1H) ;

**Exemple 10 (Composé N°149 du tableau 3)**

*N*-(pyrido[1,2-*a*]benzimidazol-7-yl)-5-fluoro-1-[(pyridin-4-yl)méthyl]-1*H*-indole-2-carboxamide

On ajoute sous argon, à une solution de 0,442 g (2,41 mmoles) de 7-amino-pyrido[1,2-*a*]benzimidazole (Begunov et al., Russian J. Org. Chem. 2004, (40), 11, 1694-1696) dans 70 mL de toluène sec, 1,51 mL de triméthylaluminium (2M dans le toluène). Après 15 min d'agitation à 0°C, on porte le mélange à 50°C et on maintient l'agitation durant 30 min. Le mélange est ensuite refroidi à 0°C et on ajoute 0,6 g (2,01 mmoles) de 5-fluoro-1-[(pyridin-4-yl)méthyl]-1*H*-indole-2-carboxylate d'éthyle, obtenu à l'étape 1.1. On porte à reflux le mélange réactionnel pendant 12 h puis on le refroidit et l'ajoute à 20 mL d'une solution glacée d'acide chlorhydrique 1N. Le pH de la phase aqueuse est ajusté à 9. On filtre un insoluble que l'on purifie par chromatographie préparative (éluant : dichlorométhane - méthanol). Le produit ainsi purifié est recristallisé dans le méthanol. On obtient 0,35 g du produit attendu sous forme d'un solide blanc après séchage sous pression réduite.
Point de fusion (base) : 277-278°C
R.M.N. $^1$H (DMSO $D_6$), δ (ppm): 5,9 (s, 2H) ; 6,99 (m, 3H) ; 7,11 (dxt, 1H) ; 7,58 (m, 6H) ; 8,21 (m, 2H) ; 8,44 (d, 2H) ; 9,05 (d, 1H).

**Exemple 11 (Composé N°150 du tableau 3)**

*N*-(pyrimido[1,2-*a*]benzimidazol-7-yl)-5-fluoro-1-(3-fluorobenzyl)-1*H*-indole-2-carboxamide

11.1 Acide 5-fluoro-1-(3-fluorobenzyl)-1*H*-indole-2-carboxylique

On ajoute une solution aqueuse de soude, préparée à partir de 1,15 g (28,92 mmoles) de pastille de soude dans 50 mL d'eau, à une solution de 7,6 g (24,10 mmoles) de 5-fluoro-1-(3-fluorobenzyl)-1*H*-indole-2-carboxylate d'éthyle (obtenu à étape 2.1) dans 240 mL d'éthanol. Le mélange est chauffé durant deux heures puis concentré sous pression réduite. Le solide résultant est repris dans 200 mL d'eau. La solution est lavée avec deux fois 100 mL d'éther éthylique, acidifiéé par ajouts successifs de petites quantités d'acide chlorhydrique concentré puis extraite avec 200 mL d'acétate d'éthyle. La phase organique est finalement lavée deux fois avec 100 mL d'eau, une fois avec 50 mL d'une solution saturée en chlorure de sodium, séchée sur sulfate de magnésium et concentrée sous pression réduite. On obtient, après séchage à 50°C sous pression réduite, 6,4 g du produit attendu sous la forme d'un solide qui sera utilisé tel quel dans l'étape suivante.

11.2 *N*-(pyrimido[1,2-a]benzimidazol-7-yl)-5-fluoro-1-(3-fluorobenzyl)-*1H*-indole-2-carboxamide (composé n° 150)

A une solution, agitée à 20°C, de 0,22 g (0,76 mmole) de composé préparé à l'étape 11.1, 146 mg (0,76 mmole) de chlorhydrate de, *N*-(3-diméthylaminopropyl)-*N'*-éthylcarbodiimide (EDAC) et 103 mg (0,76 mmole) de 1-hydroxybenzotriazole (HOBT) dans 5 mL de DMF, sont ajoutés 140 mg (0,532 mmole) de 7-amino-pyrimido[1,2-a]benzimidazole (VII-h) pur à 70%. Le mélange réactionnel est agité 48 heures à 20°C puis est versé dans 50 mL d'eau. Le mélange est ensuite extrait par 3 fois 30 mL d'acétate d'éthyle. Les phases organiques rassemblées sont lavées deux fois avec 20 mL d'eau, séchées sur sulfate de magnésium puis concentrées sous pression réduite. Le produit obtenu est purifié par chromatographie sur colonne de silice en éluant avec un mélange de dichlorométhane et de méthanol. On isole ainsi 90 mg du produit attendu.

PF = 295 - 298 °C
R.M.N. $^1H$ (DMSO $D_6$), δ (ppm) : 10,7 (s, 1H) ; 9,49 (d, 1H) ; 8,82 (d, 1H) ; 8,3 (m, 2H); 7,79 (d, 1H) ; 7,6 (m, 2H) ; 7,47 (s, 1H) ; 7,31 (m, 1H) ; 7,17 (m, 2H) ; 7,05 (m, 1H) ; 6,82 (m, 2H) 5,93 (s, 2H).

**Exemple 12 (Composé N°154 du tableau 4)**

Chlorhydrate (1 :1) de *N*-(2-méthoxy-pyrrolo[1,2-*a*]benzimidazol-6-yl)-5-fluoro-1-(3-fluorobenzyl)-*1H*-indole-2-carboxamide

Le composé 154 est préparé, selon un procédé similaire à la méthode décrite à l'exemple 11.2, à partir de l'acide préparé à l'étape 11.1 et du 6-amino-2-méthoxy-pyrrolo[1,2-a]benzimidazole (VII-I). Le solide résultant est ensuite dissout à chaud dans un mélange de 2,1 mL d'acide chlorhydrique 0,1N dans l'isopropanol et 1 mL de méthanol. Après refroidissement du milieu, on recueille un précipité par filtration que l'on sèche sous pression réduite. On isole ainsi le produit attendu sous la forme d'un chlorhydrate.
PF (HCl 1:1 ) = 220 - 225 °C
R.M.N. $^1H$ (DMSO $D_6$), δ (ppm): 3,21 (d, 1H) ; 3,35 (s, 3H) ; 3,52 (dxd, 1H) ; 4,38 (m, 2H) ; 4,8 (m, 1H) ; 5,89 (s, 2H) ; 6, 89 (m, 2H) ; 6,91-7,33 (m, 3H) ; 7,4-7,8 (m, 4H) ; 8,27 (s, 1H) ;10,68 (s, 1H).

**Exemple 13 (N°151 du tableau 3)**

Chlorhydrate (1 :1) de *N*-(pyrazino[1,2-*a*]benzimidazol-7-yl)-5-fluoro-1-(3-fluorobenzyl)-*1H*-indole-2-carboxamide

Le composé 151 est préparé selon un procédé similaire à la méthode décrite à l'exemple 12, à partir de l'acide préparé à l'étape 11.1 et du 7-amino-pyrazino[1,2-*a*]benzimidazole (VII-g).
PF (HCl 1:1) = 225 - 230 °C
R.M.N.$^1H$ (DMSO $D_6$), δ (ppm): 5,91 (s, 2H) ; 6,81 - 7,38 (m, 5H) ; 7,58 (m, 3H) ; 7,91 (d, 1H) ; 8,22 (d, 1H) ; 8,49 (m, 2H) ; 9,2 (d, 1H) ; 9,33 (s, 1H) ; 10,8 (s, 1H).

**Exemple 14 (N°26 du tableau 2)**

*N*-(2,3-dihydro-3-méthylimidazo[1,2-*a*]benzimidazol-6-yl)-5-Fluoro-1-(3-fluorobenzyl)-*1H*-indole-2-carboxamide

Le composé 26 est préparé selon un procédé similaire à la méthode décrite à l'exemple 1.2, à partir de 5-fluoro-1-(3-fluorobenzyl)-*1H*-indole-2-carboxylate d'éthyle, obtenu à l'étape 2.1 et du 6-amino-2,3-dihydro-1-méthyl-imidazo[1,2-*a*]benzimidazole (VII-c).
PF = 281- 282 °C
R.M.N.$^1H$ (DMSO $D_6$), δ (ppm): 3,1 (s, 3H) ; 4,1 - 4,35 (m, 4H) ; 5,89 (s, 2H) ; 6,82 (m, 2H) ; 6,92 - 7,2 (m, 2H) ; 7,21 - 7,35 (m, 2H) ; 7,39 (s, 1H) ; 7,53 (m, 3H) ; 7,91 (s, 1H).

**Exemple 15 (N°50 du tableau 2)**

*N*-(2,3-dihydro-1H-pyrrolo[1,2-*a*]benzimidazol-6-yl)-1-[(quinolin-2-yl)méthyl]-5-trifluorométhyl-1*H*-pyrrolo[2,3-*b*]pyridine-2-carboxamide

15.1 1-[(quinolin-2-yl)methyl]-5-trifluorométhyl-1*H*-pyrrolo[2,3-*b*]pyridine-2-carboxylate d'éthyle

A une suspension de 0,15 g (0,581 mmole) de 5-trifluorométhyl-1*H*-pyrrolo[2,3-*b*]pyridine-2-carboxylate d'éthyle (Nazare, M. et al. Angewandte Chemie, International Ed., 2004, 43(34), 4526-4528) dans 2 mL de tétrahydrofurane sec, on ajoute, sous agitation, 0,14 g (0,871 mmole) de (quinolin-2yl)méthanol, 0,228 g (0,871 mmole) de triphénylphosphine et 0,151 g (0,871 mmole) d'azodicarboxylate de diéthyle (DEAD). Après 20 h d'agitation à température ambiante, le mélange réactionnel est concentré sous pression réduite et le produit résultant est purifié par chromatographie sur colonne de silice en éluant avec un mélange de dichlorométhane et d'heptane. On obtient ainsi 0,18 g du produit attendu sous la forme d'une huile jaune

15.2 *N*-(2,3-dihydro-1*H*-pyrrolo[1,2-*a*]benzimidazol-6-yl)-1-[(quinolin-2-yl)méthyl]-5-trifluorométhyl-1*H*-pyrrolo[2,3-*b*] pyridine-2-carboxamide (composé n° 50)

Le composé 50 est préparé selon un procédé similaire à la méthode décrite dans l'exemple 5, à partir de 0,18

g (0,45 mmole) de composé obtenu à l'étape 15.1 et de 0,094 g (0,541 mmole) de 6-amino-2,3-dihydro-1*H*-pyrrolo [1,2-*a*]benzimidazole (Freedman et al., J.Het.Chem. 1966, 3, (3), 257 - 259*).* On obtient ainsi 0,22 g du produit attendu.
PF = 300 - 301°C
R.M.N. $^1$H (DMSO $D_6$), δ (ppm): 2,61 (q, 2H) ; 2,94 (t, 2H) ; 4,08 (t, 2H) ; 6,26 (s, 2H) ; 7,22 (d, 1H) ; 7,37 (d, 1H) ; 7,55 - 7,46 (m, 2H) ; 7,6 (s, 1H) ; 7,67 (dxt, 1H) ; 7,8 (d, 1H) ; 7,95 - 7,81 (m, 2H) ; 8,27 (d, 1H) ; 8,73 (s, 2H) ; 10,65 (s, 1H).

**<u>Exemple 16</u> (N°25 du tableau 2)**

*N*-(1,2,3,4-tétrahydropyrazino[1,2-*a*]benzimidazol-7-yl)-5-fluoro-1-(3-fluorobenzyl)-1H indole-2-carboxamide

16.1 *N*-(2-benzyloxycarbonyl-1,2,3,4-tétrahydropyrazino[1,2-*a*]benzimidazol-7-yl)-5-fluoro-1-(3-fluorobenzyl)-1*H*-indole-2-carboxamide.

Le composé est préparé, selon un procédé similaire à celui décrit à l'exemple 11.2, à partir de l'acide 5-fluoro-1-(3-fluorobenzyl)-1*H*-indole-2-carboxylique décrit à l'exemple 11.1 et de l'amine VII-f.

16.2 *N*-(1,2,3,4-tetrahydropyrazin[1,2-*a*]benzimidazol-7-yl)-5-fluoro-1-(3-fluorobenzyl)-1*H*-indole-2-carboxamide (composé n° 25)

Une suspension de 1 g (1,69 mmole) du composé préparé à l'étape précédente et de 0,1 g de palladium sur charbon à 10% dans 100 mL d'éthanol est agitée 6 heures à 20°C sous 5.5 bars d'hydrogène. La suspension est ensuite filtrée sur un tampon de célite et concentrée sous pression réduite. Le produit résultant est purifié par chromatographie sur colonne de silice en éluant avec un mélange de dichlorométhane et de méthanol. Le produit purifié est repris dans 10 mL d'une solution d'acide chlorhydrique 0,1N dans l'isopropanol, la solution est concentrée sous pression réduite et le solide résultant est trituré dans l'éther éthylique pour permettre d'isoler 0,48 g du produit attendu.
PF (1HCl) = 276 - 284 °C
R.M.N.$^1$H (DMSO $D_6$), δ (ppm): 3,73 (t, 2H) ; 4,38 (t, 2H) ; 4,59 (s, 3H) ; 5,59 (s, 2H) ; 6,9 (m, 2H) ; 7,02 (txd, 1H) ; 7,15 (txd, 1H) ; 7,31 (m, 1H) ; 7,45 (s, 1H) ; 7,58 (m, 3H) ; 8,1 (s, 1H) ; 9,97 (s, 1H) ; 10,45 (s, 1H).

**<u>Exemple 17</u> (N°81 du tableau 2)**

*N*-(3-méthyl-1,2,3,4-tétrahydropyrazino[1,2-*a*]benzimidazol-7-yl)-5-fluoro-1-(3-fluorobenzyl)-1*H*-indole-2-carboxamide

Le composé 81 est préparé selon un procédé similaire à celui décrit dans l'exemple 6, à partir de 5-fluoro-1-(3-fluorobenzyl)-1*H*-indole-2-carboxylate d'éthyle, obtenu à l'étape 2.1 et de 7-*a*mino-3-méthyl-1,2,3,4-tétrahydropyrazino [1,2-*a*]benzimidazole (VII-e).
R.M.N. $^1$H (DMSO $D_6$), δ (ppm): 2,45 (s, 3H), 2,89 (t, 2H), 3,74 (s, 2H) ; 4,1 (t, 2H) ; 5.9 (s, 2H) ; 6,81 - 7,6 (m, 10H) ; 7,97 (d, 1H) ; 10,46 (s, 1H).

**<u>Exemple 18</u> (N°84 du tableau 2)**

*N*-(2,3-dihydro-1*H*-pyrrolo[1,2-*a*]benzimidazol-6-yl)-5-fluoro-1-(2-benzyloxyéthyl)-1*H*-indole-2-carboxamide

18.1 5-fluoro-1-(2-benzyloxyéthyl)-1*H*-indole-2-carboxylate d'éthyle

On ajoute 94 μL (0,657 mmole) de 2-benzyloxyéthanol et 50,4 mg (0,438 mmole) de (cyanométhylene)triméthylphosphorane (Tet. Lett. 1996, 37, 2459 - 2462), à une solution de 68 mg (0,329 mmole) de 5-fluoro-1*H*-indole-2-carboxylate d'éthyle dans 2 mL de toluène, agitée à 20°C. Le mélange réactionnel est chauffé 12 heures à 110°C puis concentré sous pression réduite, repris dans 20 mL d'éther éthylique, filtré sur un tampon de célite et concentré sous pression réduite. Le résidu obtenu est purifié par HPLC préparative en éluant avec un mélange d'eau, d'acétonitrile et d'acide trifluoroacétique. On isole ainsi 99 mg d'une huile jaune.
R.M.N $^1$H(DMSO $D_6$), δ (ppm): 1,3 (t, 3H), 3,72 (t, 2H), 4,28 (q, 2H); 4,38 (s, 2H) ; 4,81 (t, 2H) ; 7,28 - 7,07 (m, 7H) ; 7,45 (dxd, 1H) ; 7,68 (dxd, 1H).

18.2 *N*-(2,3-dihydro-1*H*-pyrrolo[1,2-*a*]benzimidazol-6-yl)-5-fluoro-1-(2-benzyloxyéthyl)-1*H*-indole-2-carboxamide (composé n° 84)

Le composé 84 est préparé, selon un procédé similaire à la méthode décrite à l'exemple 6, à partir de 5-fluoro-1-(2-benzyloxyéthyl)-1H indole-2-carboxylate d'éthyle, obtenu à l'étape précédente, et du 6-*a*mino-2,3-dihydro-1*H*-pyrrolo[1,2-*a*]benzimidazole (Freedman et al., J.Het.Chem. 1966, 3, (3), 257 - 259).
PF=186-187°C
R.M.N. $^1H$ (DMSO $D_6$), δ (ppm): 2,63 (m, 2H) ; 2,93 (m, 2H) ; 3,75 (m, 2H) ; 4,09 (m, 2H) ; 4,39 (s, 2H) ; 4,81 (m, 2H) ; 7,01 - 7,19 (m, 6H) ; 7,25 (s, 1H) ; 7,31 - 7,62 (m, 3H) ; 7,62 (dxd, 1H) ; 7,97 (m, 1H) ; 10,23 (s, 1H).

**Exemple 19 (N°82 du tableau 2)**

*N*-(2,3-dihydro-1*H*-pyrrolo[1,2-*a*]benzimidazol-6-yl)-5-fluoro-1-{2-[(5-méthylpyridin-2-yl)oxy]éthyl}-1*H*-indole-2-carboxamide

19.1 2-[(5-méthylpyridin-2-yl)oxy]éthanol

Une suspension de 0,291 mL (4,12 mmoles) de 2-bromoéthanol, de 0,3 g (2,75 mmoles) de 2-hydroxy-5-méthylpyridine et de 0,85 g (6,18 mmoles) de carbonate de potassium dans 3 mL de diméthylformamide est agitée vigoureusement, 12 heures à reflux. Le mélange est ensuite dilué avec 100 mL d'eau et extrait avec 100 mL de dichlorométhane. La phase organique est lavée avec 50 mL d'eau, séchée sur sulfate de sodium, concentrée sous pression réduite puis chromatographiée sur colonne de silice en éluant avec un mélange d'hexane et d'acétate d'éthyle. On isole ainsi le produit attendu.
R.M.N. $^1H$ (DMSO $D_6$), δ (ppm): 2,2 (s, 3H) ; 3,66 - 3,71 (m, 2H) ; 4,22 (t, 2H) ; 4,77 (t, 1H) ; 6,71 (d, 1H) ; 7,51 (dxd, 1H) ; 7,95 (dxd, 1H).

19.2 5-fluoro-1-{2-[(5-méthylpyridin-2-yl)oxy]éthyl}-1*H*-indole -2-carboxylate d'éthyle

L'intermédiaire 5-fluoro-1-[2-[(5-méthylpyridin-2-yl)oxy]éthyl]-1*H*-indole-2-carboxylate d'éthyle est préparé de façon similaire à la méthode décrite dans l'exemple 18.1, à partir de 5-fluoro-1*H*-indole-2-carboxylate d'éthyle et de 2-(5-méthylpyridin-2-yloxy)éthanol préparé à l'étape précédente.

19.3 *N*-(2,3-dihydro-1H pyrrolo[1,2-*a*]benzimidazol-6-yl)-5-fiuoro-1-{2-[(5-méthylpyridin-2-yl)oxy]éthyl}-1*H*-indole-2-carboxamide (composé n° 82)

Le composé 82 est préparé selon procédé similaire à la méthode décrite à l'exemple 6, à partir du composé obtenu à l'étape précédente, et du 6-amino-2,3-dihydro-1*H*-pyrrolo[1,2-*a*]benzimidazole (Freedman et al., J.Het.Chem. 1966, 3, (3), 257 - 259).
PF=209-210 °C
R.M.N.$^1H$ (DMSO $D_6$), δ (ppm): 2,11 (s, 3H) ; 2,61 (m, 2H) ; 2,91 (m, 2H) ; 4,09 (m, 2H) ; 4,49 (m, 2H) ; 4,91 (m, 2H) ; 6,41 (d, 1H) ; 7,11 (dxt, 1H) ; 7,21 (s, 1H) ; 7,32 - 7,56 (m, 4H) ; 7,62 (dxd, 1H) ; 7,89 (d, 1H) ; 7,97 (d, 1H) ; 10,29 (s, 1H).

**Exemple 20 (N°83 du tableau 2)**

*N*-(2,3-dihydro-1*H*-pyrrolo[1,2-*a*]benzimidazol-6-yl)-5-fluoro-1-[2-{[(5-trifluorométhyl)pyridin-2-yl]oxy}éthyl]-1*H*-indole-2-carboxamide

20.1 2-[(5-trifluorométhyl)pyridin-2-yl)oxy]éthanol

Le composé est préparé selon une méthode similaire à celle décrite à l'exemple 19.1 à partir de 2-bromoéthanol et de 2-hydroxy-5-(trifluorométhyl)pyridine.

20.2. 5-fluoro-1-[2-{[(5-trifluorométhyl)pyridin-2-yl]oxy}éthyl]-1*H*-indole-2-carboxylate d'éthyle

Cet intermédiaire est préparé, de façon similaire à la méthode décrite dans l'exemple 18.1, à partir de 5-fluoro-1*H*-indole-2-carboxylate d'éthyle et de 2-[[5-(trifluorométhyl)pyridin-2-yl]oxy]éthanol préparé à l'étape précédente.

20.3 *N*-(2,3-dihydro-1*H*-pyrrolo[1,2-*a*]benzimidazol-6-yl)-5-fluoro-1[2-{[(5-trifluorométhyl)pyridin-2-yl]oxy}éthyl]-1*H*-indole-2-carboxamide (composé n° 83)

Le composé 83 est préparé selon un procédé similaire à la méthode décrite à l'exemple 6, à partir de 5-fluoro-1-[2-[[(5-trifluorométhyl)pyridin-2-yl]oxy]éthyl]-1*H*-indole-2-carboxylate d'éthyle, obtenu à l'étape précédente, et du 6-amino-2,3-dihydro-1*H*-pyrrolo[1,2-*a*]benzimidazole (Freedman et al., J.Het.Chem. 1966, 3, (3), 257-259).
$[MH]^+$ = 524
R.M.N. $^1$H (DMSO $D_6$), δ (ppm): 2,61 (m, 2H) ; 2,89 (m, 2H) ; 4,05 (m, 2H); 4,59 (m, 2H); 4,98 (m, 2H) ; 6,69 (d, 1H) ; 7,11 (dxt, 1H) ; 7,23 (s, 1H) ; 7,3 - 7,52 (m, 3H) ; 7,63 (dxd, 1H) ; 7,84 (dxd, 1H) ; 7,97 (s, 1H) ; 8,45 (s, 1H) ; 10,28 (s, 1H).

**<u>Exemple 21</u> (N°127 du tableau 2)**

*N*-(3,4-dihydro-1*H*-oxazino[1,4][4,3-*a*]benzimidazol-7-yl)-1-benzyl-5-fluoro-1*H*-pyrrolo[2,3-b]pyridine-2-carboxamide

21.1 2-Amino-3-iodo-5-fluoropyridine

Dans un bicol de 500 mL, muni d'un agitateur magnétique, sont introduits 5 g (44,6 mmoles) de 2-amino-5-fluoropyridine, 13,9 g (44,6 mmoles) de sulfate d'argent et 400 mL d'éthanol. On ajoute ensuite, par petites portions, 11,31 g (44,6 mmoles) d'iode en poudre. L'agitation est poursuivie à température ambiante pendant 24 heures. On élimine par filtration l'insoluble que l'on lave à l'éthanol et le filtrat est concentré sous pression réduite. Le résidu ainsi obtenu est repris dans un mélange d'acétate d'éthyle (200 mL) et d'une solution de carbonate de sodium (200 mL). Après séparation, la phase organique est lavée successivement avec une solution aqueuse de thiosulfate de sodium à 25% puis avec une solution aqueuse saturée en chlorure de sodium puis séchée sur sulfate de sodium et concentrée sous pression réduite. Le solide résultant est purifié par chromatographie sur colonne de silice en éluant avec un mélange de n-heptane et d'acétate d'éthyle. On obtient 2,67 g (11,22 mmoles) du produit attendu.
R.M.N.$^1$H (DMSO $D_6$), δ (ppm) : 7,95 (s, 1H) ; 7,85.(s, 1H) ; 5,9 (s, $NH_2$).

21.2 Acide 5-fluoro-1*H*-pyrrolo[2,3-*b*]pyridine-2-carboxylique

Dans un tube scellé de 25 mL, muni d'un agitateur magnétique et maintenu sous bullage d'argon, sont introduits, 0,5 g (2,10 mmoles) de 2-amino-3-iodo-5-fluoropyridine obtenu à l'étape 5.1, 0,55 g (6,3 mmoles) d'acide pyruvique, 0,71 g (6,3 mmoles) de 1,4-diazabicyclo[2.2.2]octane (DABCO) et 15 mL de diméthylformamide anhydre. Après quelques minutes, on ajoute 0,05 g (0,22 mmole) d'acétate de palladium. Le mélange réactionnel est maintenu sous agitation et sous bullage d'argon pendant 20 minutes puis rapidement scellé et porté à 100 °C pendant 2h30. La solution refroidie est concentrée sous pression réduite. Le résidu est ensuite repris par de l'acétate d'éthyle (100 mL) et de l'eau (75 mL). La phase organique est lavée à l'eau puis extraite par deux fois 50 mL d'une solution aqueuse de soude 2N. Les phases aqueuses basiques sont rassemblées, refroidies à 0°C puis acidifiées par ajout d'acide chlorhydrique (pH 3). On extrait le milieu par de l'acétate d'éthyle (4x50 mL), les phases organiques rassemblées sont séchées sur du sulfate de sodium puis concentrées sous pression réduite. On obtient 0,158 g (0,88 mmole) du produit attendu sous forme d'une poudre jaune.
R.M.N. $^1$H (DMSO $D_6$), δ (ppm) : 13,2 (s, 1H) ; 12,4 (s, 1H) ; 8,4 (d, 1H) ; 7,95 (dd, 1H) ; 7,1 (d, 1H).

21.3 5-Fluoro-1*H*-pyrrolo[2,3-*b*]pyridine-2-carboxylate d'éthyle

Dans un ballon de 100 mL, muni d'un agitateur magnétique, sont introduits 0,2 g ( 1,11 mmole) d'acide obtenu à l'étape 21.2 et 10 mL d'éthanol. On ajoute 1 mL d'acide sulfurique concentré au mélange réactionnel que l'on porte ensuite à reflux pendant 18 heures. La solution refroidie est concentrée à sec sous pression reduite. Le résidu est repris par de l'acétate d'éthyle (50 mL) que l'on lave successivement avec une solution aqueuse de soude normale (2 x 10 mL), avec de l'eau (10 mL) puis avec une solution aqueuse saturée en chlorure de sodium. On sèche la phase organique sur sulfate de sodium puis on la concentre sous pression réduite. On isole 0,21 g du produit attendu.
R.M.N. $^1$H (DMSO $D_6$), δ (ppm): 12,6 (s, NH) ; 8,4 (d, 1H) ; 8,0 (dd, 1H) ; 7,1 (d, 1H) ; 4,35 (q, 2H) ; 1,35 (t, 3H).

21.4 1-benzyl-5-Fluoro-1*H*-pyrrolo[2,3-6]pyridine-2-carboxylate d'éthyle

Le 1-benzyl-5-Fluoro-1*H*-pyrrolo[2,3-*b*]pyridine-2-carboxylate d'éthyle a été préparé selon une méthode similaire à celle décrite dans l'exemple 4.1 à partir d'alcool benzylique et du 5-Fluoro-1*H*-pyrrolo[2,3-*b*]pyridine-2-carboxylate d'éthyle préparé à l'étape précédente.
PF = 74 - 75 °C.

21.5 *N*-(3,4-dihydro-1*H*-oxazino[1,4][4,3-*a*]benzimidazol-7-yl)-1-benzyl-5-fluoro-1*H* pyrrolo[2,3-*b*]pyridine-2-carboxamide (composé n° 127)

Le composé n° 127 est préparé selon une méthode similaire à celle décrite dans l'exemple 4.2 à partir du 1-benzyl-5-Fluoro-1*H*-pyrrolo[2,3-*b*]pyridine-2-carboxylate d'éthyle, décrit à l'étape précédente, et de 7-amino-morpholino [4,3-*a*]benzimidazole (Mullock, E.B. J.Chem. Soc. Section C, 1970, (6), 829 - 833).
PF = 231 - 232 °C
R.M.N. $^1$H (DMSO $D_6$), δ (ppm): 4,15 (m, 4H), 4,91 (s, 2H), 5,9 (s, 2H) ; 7,02 - 7,26 (m, 5H) ; 7,35 (s, 1H) ; 7,5 (m, 2H) ; 8,01 (s, 1H) ; 8,12 (dxd, 1H) ; 8,46 (m, 1H) ; 10,44 (s, 1H).

**Exemple 22 (Composé N° 128 du tableau n° 2)**

*N*-(2,3-dihydro-1*H*-pyrrolo[1,2-*a*]benzimidazol-6-yl)-1-benzyl-5-fluoro-1*H*-pyrrolo[2,3-b]pyridine-2-carboxamide

Le composé n° 128 est préparé selon une méthode similaire à celle décrite dans l'exemple 4.2 à partir du 1-benzyl-5-Fluoro-1*H*-pyrrolo[2,3-*b*]pyridine-2-carboxylate d'éthyle, décrit à l'exemple 21.4, et du 6-amino-2,3-dihydro-1*H*-pyrrolo[1,2-a]benzimidazole (Freedman et al., J.Het.Chem. 1966, 3, (3), 257 - 259).
PF=259-260°C
R.M.N. $^1$H (DMSO $D_6$), δ (ppm): 2,59 (m, 2H), 2,89 (t, 2H), 4,05 (t, 2H) ; 5,89 (s, 2H) ; 7,01 - 7,24 (m, 5H) ; 7,35 (m, 2H) ; 7,45 (dxd, 1H) ; 7,91 (s, 1H) ; 8,1 (dxd, 1H) ; 8,41 (m, 1H) ; 10,41 (s, 1H).

**Exemple 23 (Composé N° 129 du tableau n° 2)**

*N*-(2,3-dihydro-1*H*-pyrrolo[1,2-*a*]benzimidazol-6-yl)-1-benzyl-5-trifluorométhyl-1*H*-pyrrolo[3,2-*b*]pyridine-2-carboxamide

23.1 3-amino-2-iodo-6-(trifluorométhyl)pyridine

On ajoute, en plusieurs portions, 1,56 g (6,17 mmoles) d'iode à un mélange, agité sous argon à 20°C, de 1 g (6,17 mmoles) de 3-amino-6-(trifluorométhyl)pyridine et de 1,25 g (6,17 mmoles) de sulfate d'argent dans 40 mL d'éthanol. L'agitation est maintenue pendant 18 heures. On élimine par filtration l'insoluble que l'on lave à l'éthanol, le filtrat est concentré sous pression réduite, le résidu est repris dans 100 mL de dichlorométhane. La phase organique est lavée successivement avec 20 mL d'une solution aqueuse de soude à 5%, 40 mL d'eau et 20 mL de solution aqueuse saturée de chlorure de sodium, séchée sur sulfate de sodium, concentrée sous pression réduite puis purifiée par chromatographie sur colonne de silice (éluants : heptane - acétate d'éthyle). On isole ainsi 1,17 g du produit attendu que l'on engage tel quel dans la suite de la synthèse.

23.2 Acide 5-trifluorométhyl-pyrrolo[3,2-*b*]pyridine-2-carboxylique

On introduit sous argon, dans un tube scellé, 0,5 g (1,74 mmole) de 3-amino-2-iodo-6-(trifluorométhyl)pyridine, obtenu à l'étape 23.1, 0,45 g (5,21 mmoles) d'acide pyruvique, 0,51 mL (5,21 mmoles) de 1,4-diazabicyclo[2.2.2]octane et 10 mL de diméthylformamide sec. La solution est dégazée quelques minutes puis on ajoute 0,19 g (0,87 mmole) d'acétate de palladium, on ferme le tube et on porte au reflux à 130°C durant 4 heures. La solution refroidie est ensuite concentrée sous pression réduite et le résidu est repris par 100 mL d'acétate d'éthyle. La phase organique est extraite successivement avec deux fois 50 mL d'une solution aqueuse de soude 2N. Les phases aqueuses basiques sont réunies, refroidies à 0°C, acidifiées par ajouts d'acide chlorhydrique puis extraites par 4 fois 50 mL d'acétate d'éthyle. Ces phases organiques sont rassemblées, lavées avec 20 mL d'une solution aqueuse saturée en chlorure de sodium, séchées sur sulfate de sodium puis concentrées sous pression réduite. On obtient 0,22 g de produit que l'on utilise tel quel dans l'étape suivante.

23.3 5-(trifluorométhyl)pyrrolo[3,2-*b*]pyridine-2-carboxylate d'éthyle

On ajoute 1 mL (18,71 mmoles) d'acide sulfurique concentré à une solution de 0,2 g (0,87 mmole) d'acide 5-trifluorométhyl-1*H*-pyrrolo[3,2-*b*]pyridine-2-carboxylique, obtenu à l'étape 23.2, dans 10 mL d'éthanol. On agite à reflux durant 20 heures puis on refroidit la solution que l'on concentre sous pression réduite. Le résidu résultant est ensuite repris avec 50 mL de dichlorométhane puis lavé successivement avec 20 mL d'une solution aqueuse saturée d'hydrogénocarbonate de sodium, 40 mL d'eau et 20 mL de solution aqueuse saturée de chlorure de sodium, séchée sur sulfate de sodium puis concentrée sous pression réduite. On obtient 0,19 g de produit que l'on utilise tel quel dans l'étape suivante:

23.4 1-benzyl-5-trifluorométhyl-pyrrolo[3,2-*b*]pyridine-2-carboxylate d'éthyle

Le 1-benzyl-5-trifluorométhyl-1*H*-pyrrolo[3,2-*b*]pyridine-2-carboxylate d'éthyle a été préparé selon une méthode similaire à celle décrite dans l'exemple 4.1 à partir d'alcool benzylique et du composé préparé à l'étape précédente.

23.5 *N*-(2,3-dihydro-1*H*-pyrrolo[1,2-*a*]benzimidazol-6-yl)-1-benzyl-5-trifluorométhyl-1*H*-pyrrolo[3,2-*b*]pyridine-2-carboxamide (composé n° 129)

Le composé n° 129 est préparé selon une méthode similaire à celle décrite dans l'exemple 4.2 à partir du 1-benzyl-5-trifluorométhyl-1*H*-pyrrolo[3,2-*b*]pyridine-2-carboxylate d'éthyle, décrit à l'étape précédente, et de 6-amino-2,3-dihydro-1*H*-pyrrolo[1,2-*a*]benzimidazole (Freedman et al., J.Het.Chem. 1966, 3, (3), 257-259).
PF = 251 - 252 °C
R.M.N. $^1H$ (DMSO $D_6$), δ (ppm): 2,61 (m, 2H), 2,91 (t, 2H), 4,09 (t, 2H) ; 5,92 (s, 2H); 7,05 - 7,29 (m, 5H) ; 7,31 - 7,5 (m, 2H) ; 7,56 (s, 1H) ; 7,7 (d, 1H) ; 7,91 (s, 1H) ; 8,29 (d, 1H) ; 10,51 (s, 1H).

**<u>Exemple 24</u> (Composé N°130 du tableau n° 2)**

*N*-(2,3-dihydro-1*H*-pyrrolo[1,2-*a*]benzimidazol-6-yl)-1-[(pyridin-4-yl)méthyl]-5-fluoro-1*H* pyrrolo[2,3-*b*]pyridine-2-carboxamide

24.1 1-[(pyridin-4-yl)méthyl]-5-fluoro-1*H*-pyrrolo[2,3-*b*]pyridine-2-carboxylate d'éthyle

On ajoute en un quart d'heure, à une solution agitée à 20°C de 5 g (24,02 mmoles) de 5-Fluoro-1*H*-pyrrolo [2,3-*b*]pyridine-2-carboxylate d'éthyle (exemple 21.3), 3,97 g (36,02 mmoles) de (pyridin-4-yl)méthanol et de 7,5 g (36,02 mmoles) de tributylphosphine dans 150 mL de toluène, 9,18 g (36,02 mmoles) de 1,1'-(azodicarbonyl)dipipéridine. Le mélange réactionnel est agité 16 heures à 20°C, filtré sur un tampon de célite, concentré sous pression réduite puis repris dans 100 mL de dichlorométhane. La solution organique est lavée deux fois par 50 mL d'une solution aqueuse de carbonate de potassium à 5% puis une fois avec 50 ml d'eau, séchée sur sulfate de sodium et concentrée sous pression réduite. Le produit obtenu est chromatographié sur colonne de silice en éluant avec un mélange d'heptane et d'acétate d'éthyle, on isole ainsi 4,5g de l'ester attendu.
PF = 120 - 121 °C

24.2 *N*-(2,3-dihydro-1Hpyrrolo[1,2-*a*]benzimidazol-6-yl)-1-[(pyridin-4-yl)méthyl]-5-fluoro-1*H*-pyrrolo[2,3-*b*]pyridine-2-carboxamide (composé n° 130)

Le composé n° 130 est préparé selon une méthode similaire à celle décrite dans l'exemple 1.2 à partir du 1-[(pyridin-4-yl)méthyl]-5-fluoro-1*H*-pyrrolo[2,3-*b*]pyridine-2-carboxylate d'éthyle, décrit à l'étape précédente, et du 6-amino-2,3-dihydro-1*H*-pyrrolo[1,2-*a*]benzimidazole (Freedman et al., J.Het.Chem. 1966, 3, (3), 257 - 259).
PF = 283 - 285 °C
R.M.N. $^1H$ (DMSO $D_6$), δ (ppm): 2,62 (m, 2H), 2,92 (t, 2H), 4,09 (t, 2H) ; 5,93 (s, 2H) ; 7,01 (d, 2H) ; 7,38 (d, 1H) ; 7,49 (m, 2H) ; 7,95 (s, 1H) ; 8,2 (d, 1H) ; 8,45 (m, 3H) ; 10,42 (s, 1H).

**<u>Exemple 25</u> (Composé N°131 du tableau n° 2)**

*N*-(2,3-dihydro-1*H*-pyrrolo[1,2-*a*]benzimidazol-6-yl)-1-[(pyridin-4-yl)méthyl]-5-trifluorométhyl-1*H*-pyrrolo[3,2-*b*]pyridine-2-carboxamide

25.1 1-[(pyridin-4-yl)méthyl]-5-trifluorométhyl-1*H*-pyrrolo[3,2-*b*]pyridine-2-carboxylate d'éthyle

Selon un procédé similaire à celui décrit dans l'exemple 24.1, à partir de 5 g (18, 4 mmoles) de 5-trifluorométhyl-1*H*-pyrrolo[3,2-*b*]pyridine-2-carboxylate d'éthyle (exemple 23.3) et de 3,04 g (27,6 mmoles) de (pyridin-4-yl)méthanol, on isole 4,2 g du composé attendu.
PF = 130 - 131 °C

25.2 *N*-(2,3-dihydro-1*H*-pyrrolo[1,2-*a*]benzimidazol-6-yl)-1-[(pyridin-4-yl)méthyl]-5-trifluorométhyl-1*H*-pyrrolo[3,2-*b*]pyridine-2-carboxamide (composé n° 131)

Selon un procédé similaire à celui décrit dans l'exemple 24.2, à partir de 0,3 g (0,86 mmole) de composé

obtenu à l'étape précédente, et de 0,178 g (1,03 mmole) de 6-amino-2,3-dihydro-1*H*-pyrrolo[1,2-*a*]benzimidazole (Freedman et al., J.Het.Chem. 1966, 3, (3), 257 - 259), on isole 0,25 g du composé attendu sous la forme d'un solide blanc.
PF = 270-271 °C
R.M.N. $^1H$ (DMSO $D_6$), δ (ppm): 2,61 (m, 2H), 2,92 (t, 2H), 4,09 (t, 2H) ; 5,99 (s, 2H) ; 7,01 (d, 2H) ; 7,43 (m, 2H) ; 7,71 (s, 1H) ; 7,79 (d, 1H) ; 7,95 (s, 1H) ; 8,25 (d, 1H) ; 8,43 (d, 2H) ; 10,51 (s, 1H).

**<u>Exemple 26</u> (Composé N°132 du tableau n° 2)**

Chlorhydrate (1.1) de *N*-(4-methyl-1,2,3,4-tétrahydropyrimido[1,2-*a*]benzimidazol-7-yl)-5-fluoro-1-(3-fluorobenzyl)-1*H*-indole-2-carboxamide

Le composé 132 est préparé selon un procédé similaire à celui de l'exemple 11.2, à partir de 0,312 g (1 ,09 mmole) d'acide 5-fluoro-1-(3-fluorobenzyl)-1*H*-indole-2-carboxylique et de 0,22 g (1,09 mmole) de 7-amino-4-méthyl-1,2,3,4-tétrahydropyrimido[1,2-a]benzimidazole (VII-m). On isole 0,41 g du produit attendu sous la forme d'un solide jaune pâle. Le composé est repris dans 50 ml d'une solution d'acide chlorhydrique 0,1N dans l'isopropànol. La solution obtenue est concentrée sous pression réduite et on isole ainsi le composé 139 sous la forme d'un chlorhydrate.
PF (HCl 1:1) = 343 - 349 °C
R.M.N.$^1H$ (DMSO $D_6$), δ (ppm):

**<u>Exemple 27</u> (Composé N°24 du tableau n° 2)**

*N*-(3,4-dihydro-1*H*-oxazino[1,4][4,3-*a*]benzimidazol-7-yl)-1-benzyl-5-trifluorométhyl-1*H*-pyrrolo[2,3-*b*]pyridine-2-carboxamide

Le composé 24 a été préparé, selon une méthode similaire à celle décrite à l'exemple 4.2, par réaction entre le 1-benzyl-5-trifluorométhyl-1*H*-pyrrolo[2,3-*b*]pyridine-2-carboxylate d'éthyle, décrit dans l'exemple 4.1 et le 7-aminooxazino[1,4][4,3-a]benzimidazole (Mullock, E.B. J.Chem. Soc. Section C, 1970, (6), 829 - 833).
PF = 236 - 237 °C
R.M.N. $^1H$ (DMSO $D_6$), δ (ppm): 4,12 (m, 4H), 4,91 (s, 2H), 5,95 (s, 2H) ; 7,02 - 7,28 (m, 5H) ; 7,5 (m, 3H) ; 8,01 (d, 1H) ; 8,68 (d, 1H) ; 8,78 (d, 1H) ; 10,55 (s, 1H).

**<u>Exemple 28</u> (Composé N°15 du tableau n°2)**

*N*-(3,4-dihydro-1*H*-oxazino[1,4][4,3-*a*]benzimidazol-7-yl)-5-fluoro-1-(3-fluorobenzyl)-1*H*-indole-2-carboxamide

Le composé 15 a été préparé, selon une méthode similaire à celle décrite à l'exemple 6, par réaction entre le 5-fluoro-1-(3-fluorobenzyl)-1*H*-indole-2-carboxylate d'éthyle, obtenu à l'étape 2.1 et le 7-*a*minooxazino[1,4][4,3-*a*]benzimidazole (Mullock, E.B. J.Chem. Soc. Section C, 1970, (6), 829 - 833).
PF=216-217 °C
R.M.N. $^1H$ (DMSO $D_6$), δ (ppm): 4,15 (m, 4H), 4,91 (s, 2H), 5,85 (s, 2H) ; 6,82 - 7,61 (m, 10H) ; 8 (s, 1H) ; 10,39 (s, 1H).

**<u>Exemple 29</u> (Composé N°133 du tableau n°2)**

*N*-[2,3-dihydro-3-(terbutoxycarbonyl)imidazo[1,2-*a*]benzimidazol-6-yl]-1-benzyl-5-trifluorométhyl-1*H*-pyrrolo[3,2-*b*]pyridine-2-carboxamide.

Le composé 133 est preparé, selon un procédé décrit dans l'exemple 11, à partir du 6-*a*mino-2,3-dihydro-1-terbutoxycarbonyl-imidazo[1,2-*a*]bénzimidazole (VII-d) et du 1-benzyl-5-trifluorométhyl-pyrrolo[3,2-*b*]pyridine-2-carboxylate d'éthyle (23.4).
PF = 169 - 174 °C
R.M.N. $^1H$ (DMSO $D_6$), δ (ppm): 1,51 (s, 9H), 4,27 (m, 4H), 5,94 (s, 2H) ; 7,09 - 7,31 (m, 6H) ; 7,42 (dxd, 1H) ; 7,58 (s, 1H) ; 7,71 (d, 1H) ; 7,84 (s, 1H) ; 8,29 (d, 1H) ; 10,52 (s, 1H).

**<u>Exemple 30</u> (Composé N°137 du tableau n°2)**

*N*-(2,3-dihydro-imidazo[1,2-*a*]benzimidazol-6-yl)-1-benzyl-5-trifluorométhyl-1H pyrrolo[3,2-*b*]pyridine-2-carboxamide.

Une solution de 0,18 g (0,31 mmole) de composé 133 (exemple 29) dans 10 mL d'acide chlorhydrique 2M

dans l'éther diéthylique est agitée 16 heures à 20°C. Après ce temps, on isole le produit 137 attendu en filtrant un précipité jaune qui est séché sous pression réduite.
R.M.N. $^1H$ (DMSO $D_6$), δ (ppm): 4,3 (m, 4H), 5,98 (s, 2H) ; 7,11 (m, 1H) ; 7,25 (m, 3H) ; 7,41 (d, 1H) ; 7,59 (dxd, 1H) ; 7,68 (s, 1H) ; 7,78 (d, 1H) ; 7,89 (s, 1H) ; 8,35 (d, 1H) ; 9,39 (s, 1H) ; 10,82 (s, 1H) ; 13,35 (s,1H).

Les tableaux 2, 3 et 4 qui suivent illustrent les structures chimiques et les propriétés physiques de quelques exemples de composés selon l'invention.

Dans ces tableaux :

- la colonne « G1 » représente un atome ou groupe d'atomes se lisant de gauche à droite ;
- la colonne « PF (°C) ou $[MH]^+$ » renseigne les points de fusion des produits en degrés Celsius (°C) ou bien le pic obtenu en spectrométrie de masse, après ionisation chimique ;
- dans la colonne « Sel/base », « - » représente un composé sous forme de base libre, alors que « HCl » représente un composé sous forme de chlorhydrate et le rapport entre parenthèses est le rapport (acide:base)
- « $CH_3$ » correspond à un groupe méthyle, « $CF_3$ » correspond à un groupe trifluorométhyle, « Et » correspond à un groupe éthyle, « t-Bu » à un groupe tertiobutyle, « i-Pr » correspond à un groupe isopropyle, « benzyl » à un groupe phénylméthyle ;

**Tableau 2**

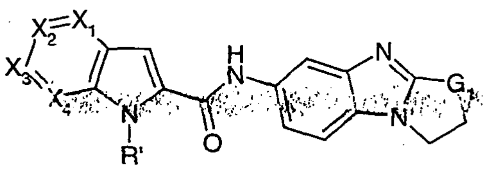

| N° | $X_1,X_2,X_{3,4}$ | R' | $G_1$ | Sel / Base | PF (°C) ou [MH]+ |
|---|---|---|---|---|---|
| 1. | CH, C-F, CH, CH | (pyridin-4-yl)méthyl | $(CH_2)_2$ | - | 292- 293 |
| 2. | CH, C-F, CH, CH | 3-fluorobenzyl | $(CH_2)_2$ | - | 286 - 287 |
| 3. | CH, CH, CH, N | benzyl | $(CH_2)_2$ | - | 233- 235 |
| 4. | CH, C-$CF_3$, CH, N | benzyl | $(CH_2)_2$ | - | 241 - 243 |
| 5. | CH, C-F, CH, CH | (pyridin-4-yl)méthyl | $CH_2$ | - | 266- 267 |
| 6. | CH, C-F, CH, CH | 3-fluorobenzyl | $CH_2$ | - | 256 - 257 |
| 7. | CH, CH, CH, N | benzyl | $CH_2$ | - | 251-252 |
| 8. | CH, C-$CF_3$, CH, N | benzyl | $CH_2$ | - | 268 - 270 |
| 9. | CH, C-F, CH, CH | 2-(pyridin-3-yl)éthyl | $CH_2$ | - | 251-252 |
| 10. | CH, C-F, CH, CH | H | $CH_2$ | - | 350 - 352 |
| 11. | CH, C-F, CH, CH | 4,6-diméthyl-pyridin-2-yl | $CH_2$ | - | 254 - 255 |
| 12. | CH, C-F, CH, CH | 3-fluorobenzyl | NH | - | $[MH]^+$= 444 |
| 13. | CH, C-F, CH, CH | Méthyl | $CH_2$ | - | 269 - 270 |
| 14. | CH, C-$CF_3$, CH, N | H | $CH_2$ | - | 380 - 385 |
| 15. | CH, C-F, CH, CH | 3-fluorobenzyl | $CH_2O$ | - | 216 - 217 |
| 16. | CH, C-F, CH, CH | (thiazol-2-yl)méthyl | $CH_2$ | - | 248 - 249 |
| 17. | CH, C-F, CH, CH | 3-fluorobenzyl , | CHOH | - | 260 - 263 |
| 18. | CH, C-F, CH, CH | (3-méthyl-pyridin-2-yl) méthyl | $CH_2$ | - | 247 - 248 |
| 19. | CH, C-F, CH, CH | 3-fluorobenzyl | S | - | 246 - 247 |
| 20. | CH, C-F, CH, CH | (pyridin-4-yl)méthyl | $CH_2O$ | - | 283 - 284 |

(suite)

| N° | $X_1$,$X_2$,$X_3$,4 | R' | $G_1$ | Sel / Base | PF (°C) ou [MH]+ |
|---|---|---|---|---|---|
| 21. | CH, C-F, CH, CH | (5-méthyl-pyridin-2-yl) méthyl | $CH_2$ | - | 257 - 260 |
| 22. | CH, C-F, CH, CH | (6-méthyl-pyridin-2-yl) méthyl | $CH_2$ | - | 277 - 279 |
| 23. | CH, C-F, CH, CH | (pyrimidin-4-yl)méthyl | $CH_2$ | - | 236 - 241 |
| 24. | CH, C-$CF_3$, CH, N | benzyl | $CH_2O$ | - | 236 - 237 |
| 25. | CH, C-F, CH, CH | 3-fluorobenzyl | $CH_2NH$ | HCl 1:1 | 276 - 284 |
| 26. | CH, C-F, CH, CH | 3-fluorobenzyl | $NCH_2$ | - | 281 - 282 |
| 27. | CH, C-tBu, CH, CH | (pyridin-4-yl)méthyl | $CH_2$ | - | 273 -274 |
| 28. | C-$OCH_3$ CH, CH, C-$CH_3$ | (pyridin-4-yl)méthyl | $CH_2$ | - | 304 - 305 |
| 29. | CH, CH, C-N$(CH_3)_2$, CH | (pyridin-4-yl)méthyl | $CH_2$ | - | 282 - 283 |
| 30. | CH, CH, C-$CF_3$, CH | (pyridin-4-yl)méthyl | $CH_2$ | - | 284 - 285 |
| 31. | C-Br, CH, CH, CH | (pyridin-4-yl)méthyl | $CH_2$ | - | 304 - 305 |
| 32. | CH, CH, C-tBu, CH | (pyridin-4-yl)méthyl | $CH_2$ | - | 241 - 242 |
| 33. | CH, CCl, CH, CH | (pyridin-4-yl)méthyl | $CH_2$ | - | $[MH]^+$ 442 |
| 34. | C-$CH_3$, CH, C-$CH_3$, CH | (pyridin-4-yl)méthyl | $CH_2$ | - | 291 - 292 |
| 35. | CH, CH, CH, CH | (pyridin-4-yl)méthyl | $CH_2$ | - | 290 - 291 |
| 36. | C-$CH_3$, CH, C-H, CH | (pyridin-4-yl)méthyl | $CH_2$ | - | 310 - 311 |
| 37. | CH, CH, C-$SCH_3$, CH | (pyridin-4-yl)méthyl | $CH_2$ | - | 263-264 |
| 38. | CH, CH, C-iPr, CH | (pyridin-4-yl)méthyl | $CH_2$ | - | 262 - 263 |
| 39. | CH, CH, C-Et, CH | (pyridin-4-yl)méthyl | $CH_2$ | - | 255 - 256 |
| 40. | CH, CH, CBr, CH | (pyridin-4-yl)méthyl | $CH_2$ | - | 298 - 299 |
| 41. | CH, C-$NO_2$, CH, | (pyridin-4-yl)méthyl | $CH_2$ | - | $[MH]^+$ 453 |
| 42. | C-$CH_3$, CH, CH, C-$CH_3$ | (pyridin-4-yl)méthyl | $CH_2$ | - | 308 - 309 |
| 43. | CH, C-$SO_2CH_3$, CH,CH | (pyridin-4-yl)méthyl | $CH_2$ | - | $[MH]^+$ 486 |
| 44. | C-F, CH, CH, CH | (pyridin-4-yl)méthyl | $CH_2$ | - | 275 - 276 |
| 45. | CH, CH, C-OiPr, CH | (pyridin-4-yl)méthyl | $CH_2$ | - | 257 - 258 |
| 46. | CH, CH, C-$CH_3$, CH | (pyridin-4-yl)méthyl | $CH_2$ | - | 302 - 303 |
| 47. | C-$CH_3$, CH, C-O $CH_3$, C-$CH_3$ | (pyridin-4-yl)méthyl | $CH_2$ | - | 311 - 312 |
| 48. | CH, CH, CH, C-iPr | (pyridin-4-yl)méthyl | $CH_2$ | - | 210 - 211 |
| 49. | CH, C-$CF_3$, CH, N | 3-fluorobenzyl | $CH_2$ | - | 253 - 254 |
| 50. | CH, C-$CF_3$, CH, N | (quinolin-2-yl)méthyl | $CH_2$ | - | 300 - 301 |
| 51. | CH, C-$CF_3$, CH, N | (quinoxalin-2-yl)méthyl | $CH_2$ | - | $[MH]^+$ 528 |
| 52. | CH, C-$CF_3$, CH, N | (5-trifluorométhyl-furan-2-yl)méthyl | $CH_2$ | - | $[MH]^+$ 534 |
| 53. | CH, C-$CF_3$, CH, N | 3-(diméthylamino)benzyl | $CH_2$ | - | $[MH]^+$ 519 |
| 54. | CH, C-$CF_3$, CH, N | (pyridin-3-yl)méthyl | $CH_2$ | - | $[MH]^+$ 477 |
| 55. | CH, C-$CF_3$, CH, N | (pyridin-2-yl)méthyl | $CH_2$ | - | $[MH]^+$ 477 |

(suite)

| N° | $X_1$,$X_2$,$X_3$,4 | R' | $G_1$ | Sel / Base | PF (°C) ou [MH]+ |
|---|---|---|---|---|---|
| 56. | CH, C-$CF_3$, CH, N | (6-méthylpyridin-2-yl) méthyl | $CH_2$ | - | $[MH]^+$ 491 |
| 57. | CH, C-$CF_3$, CH, N | (2-méthylpyridin-3-yl) méthyl | $CH_2$ | - | $[MH]^+$ 491 |
| 58. | CH, C-$CF_3$, CH, N | (2-méthylpyridin-4-yl) méthyl | $CH_2$ | - | 275 - 276 |
| 59. | CH, C-$CF_3$, CH, N | (6-trifluorométhyl-pyridin-3-yl)méthyl | $CH_2$ | - | $[MH]^+$ 545 |
| 60. | CH, C-$CF_3$, CH, N | (pyrazin-2-yl)méthyl | $CH_2$ | - | $[MH]^+$ 478 |
| 61. | CH, C-$CF_3$, CH, N | (5-méthylpyrazin-2-yl) méthyl | $CH_2$ | - | 316 - 317 |
| 62. | CH, C-$CF_3$, CH, N | (thiazol-2-yl)méthyl | $CH_2$ | - | $[MH]^+$ 483 |
| 63. | CH, C-$CF_3$, CH, N | (benzothiazol-2-yl) méthyl | $CH_2$ | - | 307 - 308 |
| 64. | CH, C-$CF_3$, CH, N | [2-(morpholin-1yl)-pyridin-3-yl]méthyl | $CH_2$ | - | $[MH]^+$ 562 |
| 65. | CH, C-$CF_3$, CH, N | 2-(diéthylaminocarbonyl) méthyl | $CH_2$ | - | 276 - 277 |
| 66. | CH, C-F, CH, CH | 3-(diméthylamino)benzyl | $CH_2$ | - | 292 - 293 |
| 67. | CH, C-F, CH, CH | 2-fluorobenzyl | $CH_2$ | - | 250 - 251 |
| 68. | CH, C-F, CH, CH | 4-fluorobenzyl | $CH_2$ | - | 218 - 219 |
| 69. | CH, C-F, CH, CH | 4-(imidazol-1-yl)benzyl | $CH_2$ | - | $[MH]^+$ 491 |
| 70. | CH, C-F, CH, CH | benzyl | $CH_2$ | - | 232 - 233 |
| 71. | CH, C-F, CH, CH | (6-trifluorométhyl-pyridin-3-yl)méthyl | $CH_2$ | - | 251 - 252 |
| 72. | CH, C-F, CH, CH | (2-méthyl-pyridin-3-yl) méthyl | $CH_2$ | - | 291 - 292 |
| 73. | CH, C-F, CH, CH | (pyridin-2-yl)éthyl | $CH_2$ | - | 180 - 181 |
| 74. | CH, C-F, CH, CH | (pyridin-4-yl)éthyl | $CH_2$ | - | $[MH]^+$ 440 |
| 75. | CH, C-F, CH, CH | (pyridin-3-yl)propyl | $CH_2$ | - | 208 - 209 |
| 76. | CH, C-F, CH, CH | (pyridin-4-yl)propyl | $CH_2$ | - | $[MH]^+$ 454 |
| 77. | CH, C-F, CH, CH | (pyridin-2-yl)propyl | $CH_2$ | - | 170 - 171 |
| 78. | CH, C-F, CH, CH | (6-méthyl-pyridin-2-yl) propyl | $CH_2$ | - | $[MH]^+$ 468 |
| 79. | CH, C-F, CH, CH | [2-(4-méthyl-phénylthio) pyridin-3-yl]méthyl | $CH_2$ | - | $[MH]^+$ 548 |
| 80. | CH, C-F, CH, CH | (quinolin-3-yl)méthyl | $CH_2$ | - | $[MH]^+$ 476 |
| 81. | CH, C-F, CH, CH | 3-fluorobenzyl | $CH_2$ $N(CH_3)$ | - | $[MH]^+$ 472 |
| 82. | CH, C-F, CH, CH | 2-[5-méthylpyridin-2-yloxy]éthyl | $CH_2$ | - | 209-210 |

(suite)

| N° | $X_1$,$X_2$,$X_3$,4 | R' | $G_1$ | Sel / Base | PF (°C) ou [MH]+ |
|---|---|---|---|---|---|
| 83. | CH, C-F, CH, CH | 2-[(5-trifluorométhyl)-pyridin-2-yloxy]éthyl | $CH_2$ | - | $[MH]^+$ 524 |
| 84. | CH, C-F, CH, CH | 2-(benzyloxy)éthyl | $CH_2$ | - | 186 - 187 |
| 85. | CH, CH, CH, N | 3-méthoxybenzyl | $CH_2$ | - | 407 - 408 |
| 86. | CH, CH, CH, N | 2-fluorobenzyl | $CH_2$ | - | $[MH]^+$ 426 |
| 87. | CH, CH, CH, N | 4-fluorobenzyl | $CH_2$ | - | 350 - 351 |
| 88. | CH, CH, CH, N | 3-chlorobenzyl | $CH_2$ | - | $[MH]^+$ 442 |
| 89. | CH, CH, CH, N | 3-méthylbenzyl | $CH_2$ | - | 211 - 212 |
| 90. | CH, CH, CH, N | (naphth-1-yl)méthyl | $CH_2$ | - | $[MH]^+$ 458 |
| 91. | CH, CH, CH, N | 2-(3-trifluorométhyl-phényl)éthyl | $CH_2$ | - | 281 - 282 |
| 92. | CH, CH, CH, N | 2-(2-trifluorométhyl-phényl)éthyl | $CH_2$ | - | 230 - 231 |
| 93. | CH, CH, CH, N | 2-(3-fluorophényl)éthyl | $CH_2$ | - | $[MH]^+$ 440 |
| 94. | CH, CH, CH, N | 2-(4-fluorophényl)éthyl | $CH_2$ | - | 233 - 234 |
| 95. | CH, CH, CH, N | 3-phénylpropyl | $CH_2$ | - | $[MH]^+$ 436 |
| 96. | CH, CH, CH, N | (pyridin-3-yl)méthyl | $CH_2$ | - | $[MH]^+$ 409 |
| 97. | CH, CH, CH, N | (4-méthyl-2-phényl-pyrimidin- 5-yl)méthyl | $CH_2$ | - | $[MH]^+$ 500 |
| 98. | CH, CH, CH, N | (pyridin-2-yl)méthyl | $CH_2$ | - | 265 - 266 |
| 99. | CH, CH, CH, N | (6-méthylpyridin-2-yl) méthyl | $CH_2$ | - | 262 - 263 |
| 100. | CH, CH, CH, N | (2-méthylpyridin-4-yl) méthyl | $CH_2$ | - | 263 - 264 |
| 101. | CH,CH,CH,N | (2-méthylpyridin-3-yl) méthyl | $CH_2$ | - | $[MH]^+$ 423 |
| 102. | CH, CH, CH, N | (6-trifluorométhyl-pyridin-3-yl)méthyl | $CH_2$ | - | $[MH]^+$ 477 |
| 103. | CH, CH, CH, | [6-(pyrrolidin-1-yl)pyridin-3- yl)méthyl | $CH_2$ | - | $[MH]^+$ 478 |
| 104. | CH, CH, CH, N | [6-(*N*-diéthylamino) pyridin- 3- yl) méthyl | $CH_2$ | - | 227 - 228 |
| 105. | CH, CH, CH, N | [2-(pyrrolidin-1-yl)pyridin-3-yl)méthyl | $CH_2$ | - | $[MH]^+$ 478 |
| 106. | CH, CH, CH, N | [6-(morpholin-1-yl) pyridin-3-yl)méthyl | $CH_2$ | - | 252 - 253 |
| 107. | CH, CH, CH, N | 2-(pyridin-2-yl)éthyl | $CH_2$ | - | $[MH]^+$ 423 |
| 108. | CH, CH, CH, N | 2-(pyridin-3-yl)éthyl | $CH_2$ | - | 408 - 409 |
| 109. | CH, CH, CH, N | 2-(pyridin-4-yl)éthyl | $CH_2$ | - | $[MH]^+$ 423 |
| 110. | CH, CH, CH, N | 3-(pyridin-4-yl)propyl | $CH_2$ | - | $[MH]^+$ 437; |
| 111. | CH, CH, CH, N | 3-(pyridin-3-yl)propyl | $CH_2$ | - | 217 - 218 |

(suite)

| N° | $X_1$,$X_2$,$X_3$,4 | R' | $G_1$ | Sel / Base | PF (°C) ou [MH]+ |
|---|---|---|---|---|---|
| 112. | CH, CH, CH, N | 3-(6-méthylpyridin-2-yl) propyl | $CH_2$ | - | 182 - 183 |
| 113. | CH, CH, CH, N | (quinolin-2-yl)méthyl | $CH_2$ | - | 254 - 255 |
| 114. | CH, CH, CH, N | (quinolin-3-yl)méthyl | $CH_2$ | - | 275 - 276 |
| 115. | CN, CH, CH, N | (quinolin-4-yl)méthyl | $CH_2$ | - | $[MH]^+$ 459 |
| 116. | CH, CH, CH, N | (3,5-diméthyl-4-méthoxypyridin-2-yl) méthyl | $CH_2$ | - | 275 - 276 |
| 117. | CH, CH, CH, N | (quinolin-6-yl)méthyl | $CH_2$ | - | 286 - 287 |
| 118. | CH, CH, CH, N | 3-(N-diméthylamino) benzyl | $CH_2$ | - | 155 - 156 |
| 119. | CH, CH, CH, N | (quinoxalin-2-yl)méthyl | $CH_2$ | - | 287 - 288 |
| 120. | CH, CH, CH, N | [2-(4-méthylphénylthio) pyridin-3-yl]méthyl | $CH_2$ | - | $[MH]^+$ 531 |
| 121. | CH, CH, CH, N | (pyrazin-2-yl)méthyl | $CH_2$ | - | $[MH]^+$ 410 |
| 122. | CH, CH, CH, N | (5-méthylpyrazin-2-yl) méthyl | $CH_2$ | - | $[MH]^+$ 424 |
| 123. | CH, CH, CH, N | (benzothiazol-2yl)méthyl | $CH_2$ | - | $[MH]^+$ 465 |
| 124. | CH, CH, CH, N | (thiazol-2yl)méthyl | $CH_2$ | - | $[MH]^+$ 415 |
| 125. | CH, CH, CH, N | 2-(diéthylaminocarbonyl) méthyl | $CH_2$ | - | 271 - 272 |
| 126. | CH, CH, CH, N | (5-trifluorométhyl-furan-2-yl)méthyl | $CH_2$ | - | [MH] 466 |
| 127. | CH, C-F, CH, N | benzyl | $CH_2O$ | - | 231 - 232 |
| 128. | CH, C-F, CH, N | benzyl | $CH_2$ | - | 259 - 260 |
| 129. | N, C-$CF_3$, CH, CH | benzyl | $CH_2$ | - | 251 - 252 |
| 130. | CH, C-F, CH, N | (pyridin-4-yl)méthyl | $CH_2$ | - | 283 - 285 |
| 131. | N, C-$CF_3$, CH, CH | (pyridin-4-yl)méthyl | $CH_2$ | - | 270 - 271 |
| 132. | CH, C-F, CH, CH | 3-fluorobenzyl | $N(CH_3)CH_2$ | HCl (1:1) | 343 - 349 |
| 133. | N, C-$CF_3$, CH, CH | benzyl | NC(O)OtBu | - | 169 -174 |
| 134. | CH, C-F, CH, N | benzyl | NC(O)OtBu | - | 160 - 164 |
| 135. | CH, C-F, CH, N | (pyridin-4-yl)méthyl | NC(O)OtBu | - | 303 - 308 |
| 136. | CH, C-F, CH, CH | 3-fluorobenzyl | NC(O)OtBu | - | $[MH]^+$ 544 |
| 137. | N, C-$CF_3$, CH, CH | benzyl | NH | HCl (2:1) | $[MH]^+$ 477 |
| 138. | CH, C-F, CH, N | benzyl | NH | HCl (1:1) | $[MH]^+$ 427 |
| 139. | CH, C-F, CH, N | (pyridin-4-yl)méthyl | NH | | $[MH]^+$ 428 |
| 140. | CH, C-$CF_3$, CH, N | benzyl | N(Me) | HCl (1:1) | 337 - 339 |

(suite)

| N° | $X_1$,$X_2$,$X_{3,4}$ | R' | $G_1$ | Sel / Base | PF (°C) ou [MH]+ |
|---|---|---|---|---|---|
| 141. | CH, C-F, CH, N | benzyl | N(Me) | HCl (1:1) | 330 - 333 |
| 142. | CH, C-F, CH, N | (pyridin-4-yl)méthyl | N(Me) | HCl (3:2) | 318 - 320 |
| 143. | CH, C-F, CH, CH | 3-fluorobenzyl | N(Me)$(CH_2)_2$ | HCl 1:1 | 325 - 331 |
| 144. | CH, C-$CF_3$, CH, N | benzyl | NH | HCl (1:1) | $[MH]^+$ 477 |
| 145. | CH, C-$CF_3$, CH, N | (pyridin-4-yl)méthyl | $CH_2$ | - | 278- 279 |
| 146. | CH, C-$CF_3$, CH, N | (pyridin-4-yl)méthyl | $CH_2O$ | - | 299 -300 |
| 147. | CH, C-$CF_3$, CH, N | benzyl | NC(O)OtBu | - | $[MH]^+$ 577 |

Les données RMN de quelques exemples du tableau sont indiquées ci-après, à titre d'exemples.

RMN pour le composé N°9 : R.M.N. $^1H$ (DMSO $D_6$), δ (ppm): 2,68 (m, 2H) ; 2,98 (t, 2H) ; 3,04 (t, 2H) ; 4,11 (t, 2H) ; 4,82 (t, 2H) ; 7,1 (dxt, 1H) ; 7,2 (m, 1H) ; 7,3 (s, 1H) ; 7,49 (m, 5H) ; 8 (s, 1H) ; 8,3 (s, 1H) ; 8,32 (d,1H) ; 10,11 (s, 1H).

RMN pour le: composé N°10 : R.M.N. $^1H$ (DMSO $D_6$), δ (ppm): 2,61 (m, 2H) ; 2,92 (t, 2H) ; 4,08 (t, 2H) ; 7,05 (dxt, 1H) ; 7,41 (m, 5H) ; 8,03 (s, 1H) ; 10,19 (s,1H) ; 11,8 (s, 1H).

RMN pour le composé N°11 : R.M.N. $^1H$ (DMSO $D_6$), δ (ppm): 2,32 (s, 3H) ; 2,4 (s, 3H) ; 2,61 (m, 2H) ; 2,8 (t, 2H) ; 4,06 (t, 2H) ; 7,11 (m, 3H) ; 7,41 (m, 5H) ; 7,89 (s, 1H) ; 10,4 (s, 1H);

RMN pour le composé N°12 : R.M.N. $^1H$ (DMSO $D_6$), δ (ppm): 4,27 (m, 4H) ; 5,88 (s, 2H) ; 6,89 (m, 2H) ; 7,02 (txd, 1H) ; 7,14 (txd, 1H) ; 7,31 ( m, 2H) ; 7,42 (s, 1H) ; 7,59 (m, 3H) ; 7,99 (s, 1H) ; 9,39 (s, 1H) ; 10, 61 (s, 1H);

RMN pour le composé N°13 : R.M.N.$^1H$ (DMSO $D_6$), δ (ppm): 2,61 (m, 2H) ; 2,91 (t, 2H) ; 4,03. (s, 3H) ; 4,1 (t, 2H) ; 7,11 (dxt, 1H) ; 7,25 (s, 1H) ; 7,48 (m, 4H) ; 8,1 (s, 1H) ; ;10,25 (s, 1H) ;

RMN pour le composé N°14 : R.M.N. $^1H$ (DMSO $D_6$), δ (ppm): 2,61 (m, 2H) ; 2,90 (t, 2H) ; 4,08 (t, 2H) ; 7,4 (m, 3H) ; 8.01 (s, 1H) ; 8,6 (d, 2H) ; 10, 3 (s, 1H) ;12,78 (s, 1H) ;

RMN pour le composé N°17 : R.M.N. $^1H$ (DMSO $D_6$), δ (ppm): 2,39 (m, 1H) ; 2,89 (m, 1H) ; 4,02 (m, 1H) ; 4,18 (m, 1H) ; 5,07 (m, 1H) ; 5,85 (s, 2H) ; 6,8 (m, 2H) ; 6,97 - 7,32 (m, 3H) ; 7,34 -7,6 (m, 5H) ; 8,02 (s, 1H).

RMN pour le composé N°18 : R.M.N. $^1H$ (DMSO $D_6$), δ (ppm): 2,4 (s, 3H) ; 2,59 (m, 2H) ; 2,9 (t, 2H) ; 4,05 (t, 2R) ; 5,95 (s, 2H) ; 6,92 - 7,11 (m, 2H) ; 7,27-7,27 (m, 6H) ; 7,89 (d, 1H) ; 8,02 (d, 1H) ; 10,09 (s, 1H).

RMN pour le composé N°49 : R.M.N. $^1H$ (DMSO $D_6$), δ (ppm): 2,66 (m, 2H) ; 2,95 (dxd, 2H) ; 4,11 (dxd, 2H) ; 6 (s, 2H) ; 6,93 (m, 2H) ; 7,05 (dxt, 1H) ; 7,3 - 7,5 (m, 3H) ; 7,59 (s, 1H) ; 7,95 (d, 1H) ; 8,71 (d, 1H) ; 8,81 (d, 1H) ; 10,55 (s, 1H).

RMN pour le composé N°136 : R.M.N. $^1H$ (DMSO $D_6$), δ (ppm): 1,51 (s, 9H) ; 4,27 (m, 4H) ; 5,92 (s, 2H) ; 7,09 - 7,3 (m, 6H) ; 7,41 (dxd, 1H) ; 7,61 (s, 1H) ; 7,85 (s, 1H) ; 8,66 (s, 1H) ; 8,79 (s, 1H) ; 10,45 (s, 1H).

RMN pour le composé N°138 : R.M.N. $^1H$ (DMSO $D_6$), δ (ppm): 4,3 (m, 4H) ; 5,92 (s, 2H) ; 7,1 (m, 2H) ; 7,21 (m, 3H) ; 7,39 (d, 1H) ; 7,41 (s, 1H) ; 7,58 (d, 1H) ; 7,99 (s, 1H) ; 8,17 (dxd, 1H) ; 8,49 (s, 1H) ; 9,3 (s, 1H) ; 10,62 (s, 1H).

RMN pour le composé N°139 : R.M.N. $^1H$ (DMSO $D_6$), δ (ppm): 4,03 (m, 4H) ; 5,92 (s, 2H) ; 6,92 (s, 1H) ; 7,05 (m, 3H) ; 7,23 ,(d, 1H) ; 7,46 (s, 1H) ; 7,56 (s, 1H) ; 8,19 (dxd, 1H) ; 8,41 (d, 3H) ; 10,32 (s, 1H).

RMN pour le composé N°143 : R.M.N. $^1H$ ($CDCl_3$), δ (ppm): 2,25 (m, 4H) ; 3,41 (s, 3H) ; 3,49 (m, 2H) ; 4,24 (m, 2H) ; 6,15 (s, 2H) ; 7,01 - 7,37 (m, 6H) ; 7,41 - 7,65 (m, 3H) ; 7,79 (m, 2H) 8,25 (s, 1H).

RMN pour le composé N°144 : R.M.N. $^1H$ (DMSO $D_6$), δ (ppm): 4,29 (m, 4H) ; 5,98 (s, 2H) ; 7,11 (m, 2H) ; 7,25 (m, 3H) ; 7,4 (d, 1H) ; 7,55 (d, 1H) ; 7,57 (s, 1H) ; 7,99 (s, 1H) ; 8,71 (s, 1H) ; 8,8 (s, 1H) ; 9,26 (s, 1H) ; 10,74 (s, 1H).

RMN pour le composé N°145 : R.M.N. $^1H$ (DMSO $D_6$), δ (ppm): 2,61 (m, 2H) ; 2,92 (t, 2H) ; 4, 09 (t, 2H) ; 5,98 (s, 2H) ; 7,01 (d, 2H) ; 7,41 (m, 2H) ; 7,62 (s, 1H) ; 7,9 (s, 1H) ; 8,39 (d, 2H) ; 8,71 (d, 2H) ; 10,55 (s, 1H).

RMN pour le composé N°146 : R.M.N. $^1H$ (DMSO $D_6$), δ (ppm): 4, 19 (m, 4H) ; 4,95 (s, 2H) ; 6 (s, 2H) ; 7,05 (m, 2H) ; 7,51 (m, 2H) ; 7,66 (s, 1H) ; 7,99 (s, 1H) ; 8,44 (d, 2H) ; 8,78 (d, 2H).

**Tableau 3**

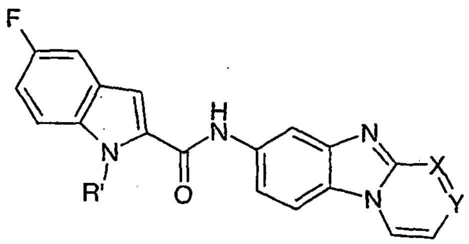

| N° | X | Y | R' | Sel / Base | PF (°C) |
|---|---|---|---|---|---|
| 148 | CH | CH | 3-fluorobenzyl | - | 261 - 262 |
| 149. | CH | CH | (pyridin-4-yl)méthyl | - | 277 - 278 |
| 150. | N | CH | 3-fluorobenzyl | - | 295 - 298 |
| 151. | CH | N | 3-fluorobenzyl | HCl 1 :1 | 225 - 230 |

**Tableau 4**

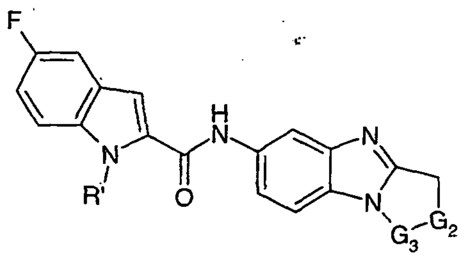

| N° | R' | $G_2$ | $G_3$ | Sel/Base | PF (°C) ou [MH]+ |
|---|---|---|---|---|---|
| 152. | (pyridin-4-yl)méthyl | $CH_2$ | $CH(CH_3)$ | HCl 1:1 | 285-290 |
| 153. | (pyridin-4-yl)méthyl | CHOH | $CH_2$ | HCl 1:1 | 295 - 300 |
| 154. | 3-fluorobenzyl | $CHOCH_3$ | $CH_2$ | HCl 1:1 | 220 - 225 |

Les données RMN de quelques exemples du tableau sont indiquées ci-après, à titre d'exemples :

<u>RMN pour le composé N°152</u> : R.M.N. $^1H$ (DMSO $D_6$), δ (ppm): 1,59 (d, 3H) ; 2,38 (m, 1H) ; 2,94 (m, 1H); 3,33 (m, 2H) ; 4,87 (m, 1H) ; 6,5 (s, 2H) ; 7,2 (dxt, 1H) ; 7,36 (d, 2H) ; 7,5-7,7 (m, 3H) ; 7,84 (m, 2H) ; 8,35 (s, 1H) ; 8,65 (d, 2H).
<u>RMN pour le composé N°153</u> : R.M.N.$^1H$ (DMSO $D_6$), δ (ppm): 3,11 (d, 1H) ; 3,62 (dxd, 1H) ; 4,2 (dxd, 1H) ; 4,49 (dxd, 1H) ; 5,1 (m, 1H) ; 6,09 (s, 2H) ; 7,19 (dxt, 1H) ; 7,43 (d, 2H) ; 7,5-7,7 (m, 3H) ; 7,81 (d, 2H) ; 8,32 (s, 1H) ; 8,7 (d, 2H) ; 10,7 (s, 1H).

Les composés selon l'invention ont fait l'objet d'essais pharmacologiques *in vitro* et *in vivo* qui ont mis en évidence leur intérêt comme substances à activités thérapeutiques. Ces composés présentent une activité antagoniste ou agoniste pour les récepteurs TRPV1 (ou VR1).

<u>Test d'inhibition du courant induit par la capsaïcine sur les DRG de rat</u>

- Culture primaire de cellule de ganglions de racine dorsale (DRG) de rat :

Les neurones du DRG expriment naturellement le récepteur TRPV1.
Les cultures primaires de DRG de rats nouveaux nés sont préparées à partir de ratons de 1 jour. Brièvement, après dissection, les ganglions sont trypsinés et les cellules dissociées mécaniquement par trituration ménagée. Les cellules sont re-suspendues dans un milieu de culture basal Eagle contenant 10 % de sérum de veau foetal, 25 mM KCl, 2 mM

glutamine, 100 μg/mL gentamicine et 50 ng/mL de NGF, puis déposées sur des lamelles de verre recouvertes de laminine (0,25 x $10^6$ cellules par lamelle) qui sont ensuite placées dans des boîtes 12 puits Corning. Les cellules sont incubées à 37°C en atmosphère humidifiée contenant 5% de $CO_2$ et 95% d'air. De la cytosine β-D-arabinoside (1 μM) est ajoutée 48 h après la mise en culture, pour prévenir le développement des cellules non neuronales. Les lamelles sont transférées dans les chambres expérimentales pour les études de patch-clamp après 7-10 jours de culture.

- Electrophysiologie :

Les chambres de mesure (volume, 800 μl) contenant la préparation cellulaire sont placées sur la platine d'un microscope inversé (Olympus IMT2) équipé d'optiques Hoffman (Modulation Contrast, New York) et observées au grossissement de 400X. Les chambres sont continuellement perfusées par gravité (2,5 mL/min) à l'aide d'un distributeur de solutions acceptant 8 entrées et dont la sortie unique, constituée par un tube de polyéthylène (ouverture 500μm) est placée à moins de 3 mm de la cellule étudiée. La configuration "cellule entière" de la technique de patch-clamp a été utilisée. Les pipettes en verre borosilicaté (résistance 5-10 MOhms) sont approchées de la cellule grâce à un micromanipulateur piézoélectrique 3D (Burleigh, PC1000). Les courants globaux (potentiel de membrane fixé à -60 mV) sont enregistrés avec un amplificateur Axopatch 1D (Axon Instruments, Foster city, Californie), connecté à un PC piloté par les logiciels de Pclamp8 (Axon Instrument). Les traces de courant sont enregistrées sur papier et simultanément digitalisées (fréquence d'échantillonnage 15 à 25 Hz) et acquises sur le disque dur du PC.
L'application d'une solution de capsaïcine 300 nM, provoque sur les cellules de DRG (voltage fixé à -70 mV) un courant cationique entrant. Afin de minimiser la désensibilisation des récepteurs, l'intervalle d'une minute minimum entre deux applications de capsaïcine est respecté. Après une période contrôle (stabilisation de la réponse capsaïcine seule), les composés de l'invention à tester sont appliqués seuls à une concentration donnée (concentration de 10 nM ou de 1 nM) pendant une durée de 4 à 5 minutes, au cours desquelles plusieurs tests capsaïcine + composé sont réalisés (obtention de l'inhibition maximale). Les résultats sont exprimés en % d'inhibition de la réponse capsaïcine contrôle.
Dans le cas des composés antagonistes VR1, les pourcentage d'inhibition de la réponse capsaïcine (1 microM ) sont compris entre 20% et 100% pour les composés les plus actifs de l'invention testés à des concentrations de 0,1 à 10 nM. Ce sont donc des antagonistes efficaces des récepteurs de type TRPV1. Le tableau 5 donne quelques exemples des % d'inhibition obtenus avec les composés de l'invention.

**Tableau 5**

| N°composé | % inhibition en patch DRG |
|---|---|
| 1 | 37% (10 nM) |
| 5 | 53% (10 nM) |

L'effet propre agoniste des composés peut être évalué par la mesure du courant induit à différentes concentrations de composé sur le DRG de rat, en présence ou non de capsazépine.

Test d'irritation cornéenne souris

Le caractère irritant de la capsaïcine est aisément apprécié au niveau de la cornée puisque cet organe est un des plus innervés par les fibres C. Dans ce contexte, d'après des expériences préliminaires, l'application d'une très faible quantité de capsaïcine (2 μl à une concentration de 160 μM) à la surface de la cornée d'un animal entraîne un certain nombre de comportements stéréotypés liés à l'irritation et qu'il est facile de répertorier. Parmi ceux-ci, on note : clignement de l'oeil, frottement de l'oeil instillé par la patte avant ipsilatérale, frottement de la face avec les deux pattes avant, grattement de la face ipsilatérale par la patte arrière. La durée de ces comportements ne dépasse pas les 2 minutes d'observation, et l'animal reprend alors son activité normale. Son aspect est par ailleurs également normal. La souris n'est pas recluse dans un coin avec les poils hérissés et ne développe aucun signe observable de souffrance. On peut en conclure que la durée d'action de la capsaïcine à ces doses est inférieure à 2 minutes.

Résumé de la méthodologie :

Le principe de la série d'expériences est de déterminer si les composés de l'invention peuvent influencer la réponse comportementale induite par une quantité donnée de capsaïcine. La capsaïcine est initialement diluée à 25 mM dans le DMSO et diluée, pour son utilisation finale, dans le sérum physiologique avec du Tween 80 à 10%. Il apparaît, à partir d'études contrôles que dans ces conditions, le solvant n'a aucun effet.
En pratique, le produit à tester, préparé à 25 mM dans le DMSO et dilué pour son utilisation finale dans le sérum

physiologique avec 10% de Tween 80 à la plus forte concentration de 500 μM, est administré en application locale à la surface de la cornée sous un volume de 2 μl, 10 minutes avant l'application de la capsaicine. L'animal reçoit l'instillation oculaire de 2 μl d'une solution de capsaïcine à 160 μM préparée comme indiqué ci-dessus. Au cours d'une observation de 2 minutes suivant l'instillation, le nombre de frottements de l'oeil instillé par la patte antérieure ipsilatérale est compté pour chaque animal.
Pour un groupe donné, le pourcentage de protection est calculé comme suit :

P= 100 – ((nombre moyen de grattages du groupe traité par le composé / nombre moyen de grattages du groupe traité par le solvant) x 100)

Ce pourcentage de protection est moyenne pour chaque groupe d'animaux (n = nombre d'animaux testés avec le composé de l'invention).

Les pourcentages de protection évalués, dans ce modèle, pour les composés de l'invention les plus actifs, utilisés à la concentration de 500μM, sont compris entre 20% et 100% (voir exemple dans le tableau 6) :

**Tableau 6**

| n°composé | % P 500μM |
|---|---|
| 8 | 71% |
| 15 | 56% |
| 26 | 46% |

Les résultats de ces essais montrent que les composés les plus actifs de l'invention bloquent, in vivo, les effets induits par la stimulation des récepteurs TRPV1.

Les composés de l'invention peuvent donc être utilisés pour la préparation de médicaments, notamment pour la préparation d'un médicament destiné à prévenir ou à traiter les pathologies dans lesquelles les récepteurs de type TRPV1 sont impliqués.

Les composés de l'invention peuvent être utiles pour prévenir ou traiter les pathologies dans lesquelles les récepteurs de type TRPV1 sont impliqués.

Ainsi, l'invention a pour objet des médicaments qui comprennent au moins un composé de formule (I), ou un sel pharmaceutiquement acceptable, ou encore un hydrate ou un solvat dudit composé.

Ces médicaments trouvent leur emploi en thérapeutique, notamment dans la prévention et/ou le traitement de la douleur et de l'inflammation, de la douleur chronique, neuropathique (traumatique, diabétique, métabolique, infectieuse, toxique, induite par un traitement anticancéreux ou hiatrogène), (ostéo-) arthritique, rhumatismale, des fibromyalgies, de la douleur du dos, de la douleur liée au cancer, de la névralgie faciale, des céphalées, de la migraine, de la douleur dentaire, de la brûlure, du coup de soleil, de la morsure ou de la piqûre, de la névralgie post-herpétique, de la douleur musculaire, de la compression nerveuse (centrale et/ou périphérique), des traumatismes de la moelle et/ou du cerveau, de l'ischémie (de la moelle et/ou du cerveau), de la neurodégénérescence, des accidents vasculaires hémorragiques (de la moelle et/ou du cerveau), de la douleur post-stroke.
Les composés de l'invention peuvent également être utilisés pour prévenir et/ou traiter les désordres métaboliques tels que le diabète.
Les composés de l'invention peuvent également être utilisés pour prévenir et/ou traiter les désordres urologiques tels que l'hyperactivité de la vessie, l'hyperéflexie vésicale, l'instabilité vésicale, l'incontinence, la miction d'urgence, l'incontinence urinaire, la cystite, la colique néphrétique, l'hypersensibilité pelvienne et la douleur pelvienne.
Les composés de l'invention peuvent être utiles pour prévenir et/ou traiter les désordres gynécologiques comme la vulvodynie, les douleurs liées aux salpingites, aux dysménorrhées.
On peut également utiliser ces produits pour prévenir et/ou traiter les désordres gastro-intestinaux tels que le désordre du réflexe gastro-esophagique, l'ulcère de l'estomac, l'ulcère du duodénum, la dyspepsie fonctionnelle, la colite, l'IBS, la maladie de Crohn, la pancréatite, l'oesophagite, la colique hépatique.
De même, les produits de la présente invention peuvent être utiles dans la prévention et/ou le traitement des désordres respiratoires tels que l'asthme, la toux, la maladie pulmonaire obstructive chronique (COPD), la bronchoconstriction et les désordres inflammatoires.
Ces produits peuvent également être utilisés pour prévenir et/ou traiter le psoriasis, le pruritis, les irritations dermiques, des yeux ou des muqueuses, l'herpès, le zona.

Les composés de l'invention peuvent également être utilisés pour traiter la dépression.
Les composés de l'invention peuvent aussi être utilisés pour traiter les maladies du système nerveux central telles que la sclérose en plaques

Selon un autre de ses aspects, la présente invention concerne des compositions pharmaceutiques comprenant, en tant que principe actif, au moins un composé selon l'invention. Ces compositions pharmaceutiques contiennent une dose efficace d'au moins un composé selon l'invention, ou un sel pharmaceutiquement acceptable, un hydrate ou solvat dudit composé, ainsi qu'au moins un excipient pharmaceutiquement acceptable.
Lesdits excipients sont choisis selon la forme pharmaceutique et le mode d'administration souhaité, parmi les excipients habituels qui sont connus de l'homme du métier.

Les compositions pharmaceutiques de la présente invention peuvent être administrées par voie orale, sublinguale; sous-cutanée, intramusculaire, intra-veineuse, topique, locale, intratrachéale, intranasale, transdermique ou rectale. Ces compositions peuvent être administrées sous forme unitaire, en mélange avec des excipients pharmaceutiques classiques. Elles sont destinées à être administrées aux animaux et aux êtres humains pour la prophylaxie ou le traitement des troubles ou des maladies citées ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscaramellose sodique | 6,0 mg |
| Amidon de maïs | 15,0 mg |
| Hydroxypropyl-méthylcellulose | 2,25 mg |
| Stéarate de magnésium | 3,0 mg |

Lesdites formes unitaires sont dosées pour permettre une administration journalière de 0,001 à 30 mg de principe actif par kg de poids corporel, selon la forme galénique.

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

Les composés de l'invention peuvent également être utilisés pour la préparation de médicaments, notamment pour la préparation d'un médicament destiné à prévenir ou à traiter les pathologies dans lesquelles les récepteurs de type TRPV1 sont impliqués, telles que mentionnées précédemment.

Les composés de l'invention peuvent également être utilisé dans une méthode de traitement des pathologies indiquées ci-dessus, qui comprend l'administration, à un patient, d'une dose efficace d'au moins un composé selon l'invention, ou un de ses sels pharmaceutiquement acceptables ou hydrates ou solvats.

## Revendications

1. Composé répondant à la formule générale (I)

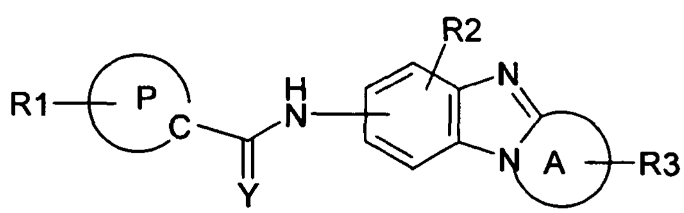

(I)

dans laquelle :

A représente, avec la liaison C-N du motif benzimidazole auquel il est fusionné, un hétérocycle monocyclique ou un hétéroaryle monocyclique de 4 à 7 chaînons, contenant de un à trois hétéroatomes choisis parmi O, S ou N , y compris l'atome d'azote du motif benzimidazole ;

P représente un hétérocycle bicyclique ou hétéroaryle bicyclique à 8-, 9-, 10- ou 11 chaînons, comprenant de 1 à 6 hétéroatomes sélectionnés parmi N, O et S ; P étant lié au groupe -C(Y)- par un atome de carbone ;

à la condition que, lorsque A représente un hétérocycle saturé à 7 chaînons, P est différent du groupe 2,3-dihydro-1,4-benzodioxane, du groupe 1-benzopyran-2-one, du groupe isoindole ;

$R_1$ représente de un à quatre atomes ou groupes, identiques ou différents, choisis parmi un atome d'hydrogène, un atome d'halogène, un groupe oxo, thio, $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, aryloxy-$C_1$-$C_6$-alkyle, hétéroaryloxy-$C_1$-$C_6$-alkyle, aryl-$C_1$-$C_3$-alkylènoxy-$C_1$-$C_6$-alkyle, hétéroaryl-$C_1$-$C_3$-alkylènoxy-$C_1$-$C_6$-alkyle, arylthio-$C_1$-$C_6$-alkyle, hétéroarylthio-$C_1$-$C_6$-alkyle, aryl-$C_1$-$C_3$-alkylène-thio-$C_1$-$C_6$-alkyle, hétéroaryl-$C_1$-$C_3$-alkylène-thio-$C_1$-$C_6$-alkyle, $C_1$-$C_6$-alcoxy, $C_3$-$C_7$-cycloalkyloxy, $C_3$-$C_7$-cycloalkyle-$C_1$-$C_3$-alkylènoxy, $C_1$-$C_6$-fluoroalcoxy, cyano, C(O)$NR_4R_5$, nitro, $NR_4R_5$, $C_1$-$C_6$-thioalkyle, $C_3$-$C_7$-cycloalkylthio, $C_3$-$C_7$-cycloalkyle-$C_1$-$C_3$-alkylène-thio, -S(O)-$C_1$-$C_6$-alkyle, -S(O)-$C_3$-$C_7$-cycloalkyle, -S(O)-$C_1$-$C_3$-alkylène-$C_3$-$C_7$-cycloalkyle, $C_1$-$C_6$-alkyle-$S(O)_2$-, $C_1$-$C_6$-fluoroalkyle-$S(O)_2$-, $C_3$-$C_7$-cycloalkyle-$S(O)_2$-, $C_3$-$C_7$-cycloalkyle-$C_1$-$C_3$-alkylène-$S(O)_2$-, $SO_2NR_4R_5$, $SF_5$, $NR_6C(O)R_7$, $NR_6SO_2R_8$ , $R_4R_5NC(O)$-$C_1$-$C_3$-alkylène, aryle, hétéroaryle, aryl-$C_1$-$C_5$-alkylène, hétéroaryl-$C_1$-$C_5$-alkylène, aryloxy, arylthio, hétéroaryloxy ou hétéroarylthio ;

les dits groupes hétéroaryle ou aryle de $R_1$ étant éventuellement substitués par un ou plusieurs substituants $R_9$, identiques ou différents l'un de l'autre ;

à la condition que lorsque $R_1$ est attaché à un atome d'azote de P, alors $R_1$ est différent d'un atome d'halogène, d'un groupe oxo, thio, cyano, nitro, $SF_5$, $NR_4R_5$, $C_1$-$C_6$-thioalkyle, thioaryle, thiohétéroaryle, $C_1$-$C_6$-alcoxy, aryloxy, hétéroaryloxy, -$NR_6COR_7$ et $NR_6SO_2R_8$ ;

Y représente un atome d'oxygène ou de soufre ;

$R_2$ représente un atome d'hydrogène, un atome d'halogène, un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle ou $C_1$-$C_6$-alcoxy ;

$R_3$ représente de un à trois atomes ou groupes, identiques ou différents, choisis parmi un atome d'hydrogène, un atome d'halogène, un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, hydroxyle, $C_1$-$C_6$-alcoxy, $C_3$-$C_7$-cycloalkyloxy, $C_3$-$C_7$-cycloalkyle-$C_1$-$C_3$-alkylénoxy, lorsque $R_3$ est porté par un atome de carbone ;

ou

$R_3$ représente de un à deux atomes ou groupes, identiques ou différents, choisis parmi un atome d'hydrogène, un atome d'halogène, un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, aryle-C(O)-, $C_1$-$C_6$-alkyle-C(O)-, $C_3$-$C_7$-cycloalkyle-C(O)-, $C_3$-$C_7$-cycloalkyle-$C_1$-$C_3$-alkylène-C(O)-, $C_1$-$C_6$-fluoroalkyle-C(O)-, aryle-$S(O)_2$-, $C_1$-$C_6$-alkyle-$S(O)_2$-, $C_1$-$C_6$-fluoroalkyle-$S(O)_2$-, $C_3$-$C_7$-cycloalkyle-$S(O)_2$-, $C_3$-$C_7$-cycloalkyle-$C_1$-$C_3$-alkylène-$S(O)_2$-, $C_1$-$C_6$-alkyle-O-C(O)-, aryle-$C_1$-$C_3$-alkyle-O-C(O)-, $C_3$-$C_7$-cycloalkyle-O-C(O)-, $C_3$-$C_7$-cycloalkyle-$C_1$-$C_3$-alkylène-O-C(O)-, $C_1$-$C_6$-fluoroalkyle-O-C(O)-, aryle-O-C(O)- , hétéroaryl-O-C(O)- , lorsque $R_3$ est porté par un atome d'azote ;

$R_4$ et $R_5$, représentent, indépendamment l'un de l'autre, un atome d'hydrogène ou un groupe $C_1$-$C_6$-alkyle, $C_3$-$C_7$-cycloalkyle, $C_3$-$C_7$-cycloalkyle-$C_1$-$C_3$-alkylène, aryle-$C_1$-$C_5$-alkylène ou aryle ;

ou $R_4$ et $R_5$ forment ensemble, avec l'atome d'azote qui les porte, un groupe azétidine, pyrrolidine, pipéridine, azépine, morpholine, thiomorpholine, pipérazine, homopipérazine, le groupe $NR_4R_5$ étant éventuellement substitué par un groupe $C_1$-$C_6$-alkyle, $C_3$-$C_7$-cycloalkyle, $C_3$-$C_7$-cycloalkyle-$C_1$-$C_3$-alkylène, aryle-$C_1$-$C_6$-alkyléne, aryle, hétéroaryle, aryle-$S(O)_2$-, $C_1$-$C_6$-alkyle-$S(O)_2$-, $C_1$-$C_6$-fluoroalkyle-$S(O)_2$-, $C_3$-$C_7$-cycloalkyle-$S(O)_2$-, $C_3$-$C_7$-cycloalkyle-$C_1$-$C_3$-alkylène-$S(O)_2$-, aryle-C(O)-, $C_1$-$C_6$-alkyle-C(O)-, $C_3$-$C_7$-cycloalkyle-C(O)-, $C_3$-$C_7$-cycloalkyle-$C_1$-$C_3$-alkylène-C(O)-, $C_1$-$C_6$-fluoroalkyle-C(O)-, hydroxyle, $C_1$-$C_6$-alkyloxy, $C_1$-$C_6$-fluoroalkyle, aryloxy-$C_1$-$C_6$-alkylène, aryloxy, hétéroaryloxy-$C_1$-$C_6$-alkylène, hétéroaryloxy ;

$R_6$ et $R_7$ représentent, indépendamment l'un de l'autre, un atome d'hydrogène, un groupe $C_1$-$C_6$-alkyle, $C_3$-$C_7$-cycloalkyle, $C_3$-$C_7$-cycloalkyle-$C_1$-$C_3$-alkylène, aryle-$C_1$-$C_6$-alkylène ou aryle ;

ou $R_6$ et $R_7$ forment ensemble un lactame de 4 à 7 chaînons comprenant l'atome d'azote et le groupe C(O) qui les portent ;

$R_8$ représente un groupe $C_1$-$C_6$-alkyle, $C_3$-$C_7$-cycloalkyle, $C_3$-$C_7$-cycloalkyle-$C_1$-$C_3$-alkylène, aryle-$C_1$-$C_6$-alkylène ou aryle ;

ou $R_6$ et $R_8$ forment ensemble un sultame de 4 à 7 chaînons comprenant l'atome d'azote et le groupe $S(O)_2$ qui les portent ;

$R_9$ représente un atome d'halogène, un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, $C_1$-$C_6$-alcoxy, $C_1$-$C_6$-fluoroalcoxy, nitro, cyano, $NR_4R_5$, $R_4R_5N$-$C_1$-$C_3$-alkylène, aryle, hétéroaryle, aryloxy, arylthio, hétéroaryloxy ou hétéroarylthio, lesdits groupes hétéroaryle ou aryle étant éventuellement substitués par un ou plusieurs substituants choisis parmi un atome d'halogène, un groupe $C_1$-$C_6$-alkyle, $C_3$-C7-cycloalkyle, $C_3$-$C_7$-cycloalkyle-$C_1$-$C_3$-alkylène, $C_1$-$C_6$-fluoroalkyle, $C_1$-$C_6$-alcoxy, $C_1$-$C_6$-fluoroalcoxy, nitro, cyano, $NR_4R_5$, $R_4R_5N$-$C_1$-$C_3$-alkylène ;

le ou les atomes de soufre de l'hétérocycle A peuvent être sous forme oxydée (S(O) ou $S(O)_2$) ;
le ou les atomes d'azote peuvent être sous forme oxydée (N-oxyde) ;
à l'état de base ou de sel d'addition à un acide, ainsi qu'à l'état d'hydrate ou de solvate ;
le 2-(2-chlorophényl)-1,3-dioxo-N-(7,8,9,10-tétrahydro-6H-azepino[1,2-a]benzimidazol-3-yl)isoindoline-5-carboxamide étant exclu.

**2.** Composé de l'invention selon la revendication 1, **caractérisé en ce qu'**il répond à la formule générale (II) :

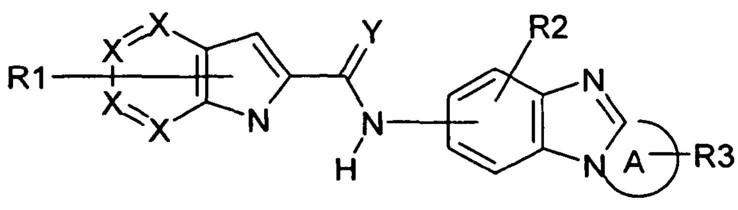

(II)

dans laquelle
X représente un atome de carbone ou un atome d'azote ; lesdits X étant identiques ou différents entre eux et le nombre de X = N n'étant pas supérieur à 2 ;
$R_1$, $R_2$, $R_3$, Y et A étant tels que définis dans la formule générale (I) selon la revendication 1, $R_1$ pouvant être lié au motif à 6 éléments ou à 5 éléments du bicycle ;
à l'état de base ou de sel d'addition à un acide, ainsi qu'à l'état d'hydrate ou de solvate.

**3.** Composé de l'invention selon la revendication 1 ou 2, **caractérisé en ce qu'**il répond à la formule générale (III) :

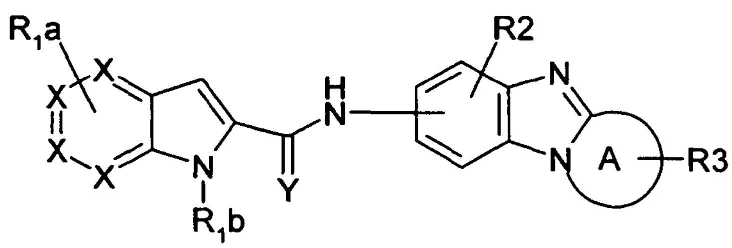

(III)

dans laquelle :

$R_{1a}$ représente un ou plusieurs atomes ou groupes, identiques ou différents, choisi parmi un atome d'hydrogène, un atome d'halogène ou un groupe $C_1$-$C_6$-alkyle, $C_1$-$C_6$-fluoroalkyle, $C_1$-$C_6$-alcoxy, $C_1$-$C_6$-thioalkyle, $C_1$-$C_6$-alkyle-$S(O)_2$-, $NR_4R_5$, nitro ;
$R_{1b}$ représente un atome d'hydrogène, un groupe $C_1$-$C_6$-alkyle, hétéroaryloxy-$C_1$-$C_6$-alkyle, aryl-$C_1$-$C_3$-alkylènoxy-$C_1$-$C_6$-alkyle, $R_4R_5NC(O)$-$C_1$-$C_3$-alkylène, aryle, hétéroaryle, aryl-$C_1$-$C_6$-alkylène ou hétéroaryl-$C_1$-$C_5$-alkylène ;
lesdits groupes hétéroaryle ou aryle de $R_{1b}$ étant éventuellement substitués par un ou plusieurs substituants $R_9$, identiques ou différents l'un de l'autre ;
$R_9$ représente un atome d'halogène ou un groupe $C_1$-$C_6$-alkyle, $C_1$-$C_6$-alcoxy, $C_1$-$C_6$-fluoroalkyle, aryle, hétéroaryle, $NR_4R_5$, arylthio, lesdits groupes hétéroaryle ou aryle étant éventuellement substitués par un ou plusieurs substituants choisis parmi un atome d'halogène, un groupe $C_1$-$C_6$-alkyle, $C_3$-$C_7$cycloalkyle, $C_3$-$C_7$-cycloalkyle-$C_1$-$C_3$-alkylène, $C_1$-$C_6$-fluoroalkyle, $C_1$-$C_6$-alcoxy, $C_1$-$C_6$-fluoroalcoxy, nitro, cyano, $R_4R_5N$-$C_1$-$C_3$-alkylène ;
$R_2$, $R_3$, $R_4$, $R_5$, A, X et Y étant tels que définis dans la formule générale (II) selon la revendication 2 ;

à l'état de base ou de sel d'addition à un acide, ainsi qu'à l'état d'hydrate ou de solvate.

4. Composé de l'invention selon l'une quelconque des revendications 1 à 3, **caractérisé en ce qu'**il répond à la formule générale (IV) :

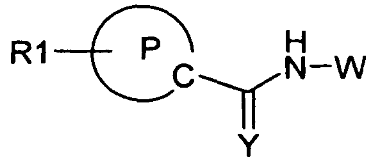

(IV)

dans laquelle W est un hétérocycle tricyclique ou un hétéroaryle tricyclique choisi parmi :

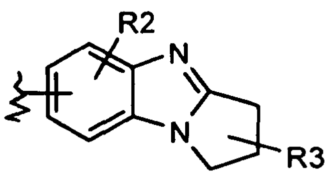

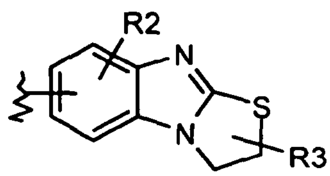

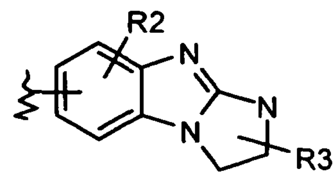

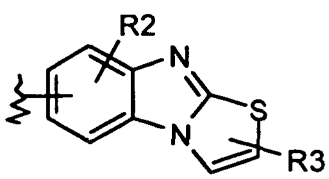

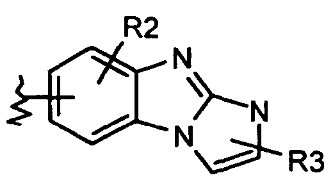

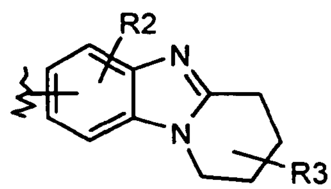

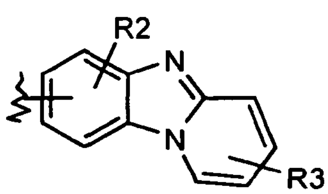

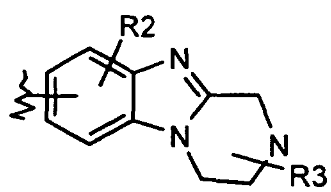

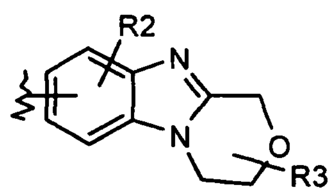

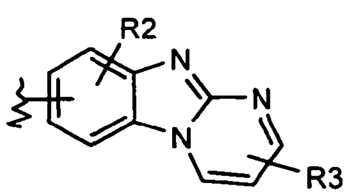

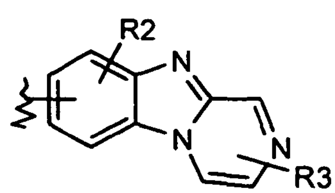

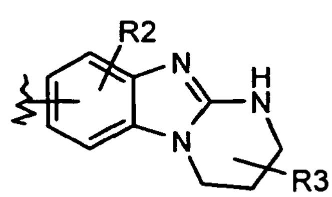

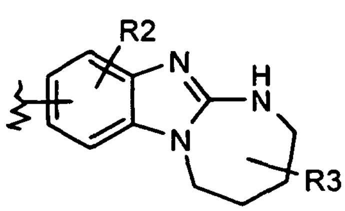

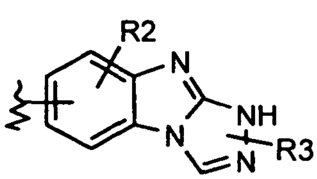

$R_1$, $R_2$, $R_3$, P et Y étant tels que définis dans la formule générale (I) selon la revendication 1;

à l'état de base ou de sel d'addition à un acide, ainsi qu'à l'état d'hydrate ou de solvate.

**5.** Composé de l'invention selon l'une quelconque des revendications 1 à 4, **caractérisé en ce qu'**il répond à la formule générale (V)

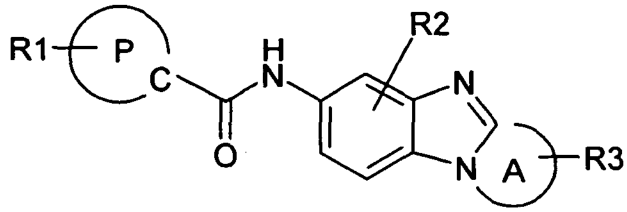

(V)

dans laquelle :

$R_1$, $R_2$, $R_3$, A et P sont tels que définis dans la formule générale (I) selon la revendication 1;

à l'état de base ou de sel d'addition à un acide, ainsi qu'à l'état d'hydrate ou de solvate.

**6.** Composé de l'invention selon l'une quelconque des revendications 1 à 5, **caractérisé en ce qu'**il répond à la formule générale (V)

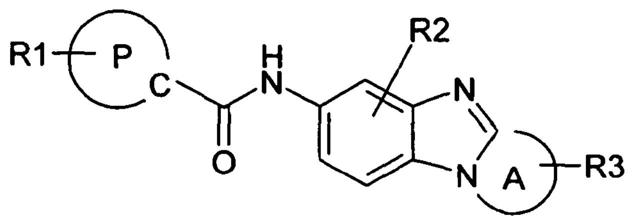

(V)

dans laquelle :

$R_2$ représente un atome d'hydrogène, un groupe $C_1$-$C_6$-alkyle ou $C_1$-$C_6$-alcoxy ;
$R_3$ représente de un à trois atomes ou groupes, identiques ou différents, choisis parmi un atome d'hydrogène, un groupe $C_1$-$C_6$-alkyle, $C_1$-$C_6$-alcoxy ou hydroxyle, lorsque $R_3$ est porté par un atome de carbone ;
ou
$R_3$ représente de un à deux atomes ou groupes, identiques ou différents, choisis parmi un atome d'hydrogène, un groupe $C_1$-$C_6$-alkyle, $C_1$-$C_6$-alkyle-O-C(O)-, aryl-$C_1$-$C_3$-alkyle-O-C(O)-, lorsque $R_3$ est porté par un atome d'azote ;
$R_1$, A et P étant tels que définis dans la formule générale (I) selon la revendication 1;

à l'état de base ou de sel d'addition à un acide, ainsi qu'à l'état d'hydrate ou de solvate.

**7.** Composé de l'invention selon l'une quelconque des revendications 1 à 6, **caractérisé en ce qu'**il répond à la formule générale (V)

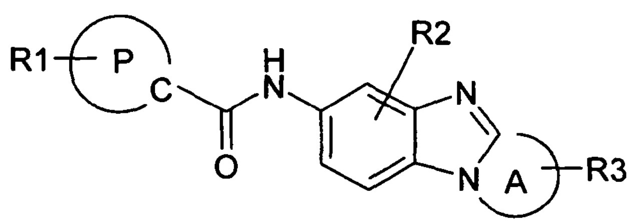

(V)

dans laquelle :

A représente, avec la liaison C-N du motif benzimidazole auquel il est fusionné, un hétérocycle monocyclique ou un hétéroaryle monocyclique de 5 à 7 chaînons, contenant de un à trois hétéroatomes choisis parmi O, S ou N, y compris l'atome d'azote du motif benzimidazole ;
$R_1$, $R_2$, $R_3$ et P étant tels que définis dans la formule générale (I) selon la revendication 1 ; à l'état de base ou de sel d'addition à un acide, ainsi qu'à l'état d'hydrate ou de solvate.

**8.** Composé de l'invention selon l'une quelconque des revendications 1 à 7, **caractérisé en ce qu'**il répond à la formule générale (Va) :

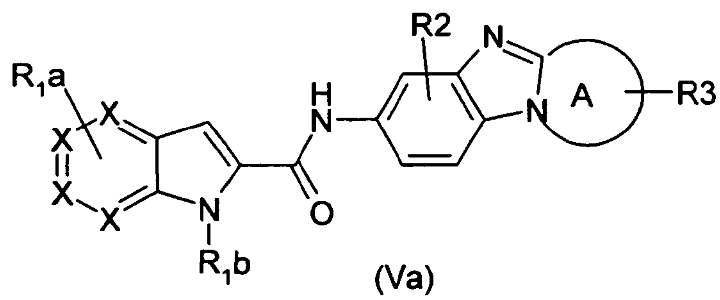

(Va)

dans laquelle

A représente, avec la liaison C-N du motif benzimidazole auquel il est fusionné, un hétérocycle monocyclique ou un hétéroaryle monocyclique de 5 à 7 chaînons, contenant de un ou deux hétéroatomes choisis parmi O, S ou N, y compris l'atome d'azote du motif benzimidazole ;
X représente un atome de carbone ou un atome d'azote ; lesdits X étant identiques ou différents entre eux et le nombre de X = N n'étant pas supérieur à 1 ;
$R_{1a}$ représente un ou plusieurs atomes ou groupes, identiques ou différents, choisi parmi un atome d'hydrogène, un atome d'halogène ou un groupe $C_1$-$C_6$-alkyle, $C_1$-$C_6$-fluoroalkyle, $C_1$-$C_6$-alcoxy, $C_1$-$C_6$-thioalkyle, $C_1$-$C_6$-alkyle-$S(O)_2$-, $NR_4R_5$, nitro ;
$R_{1b}$ représente un atome d'hydrogène, un groupe $C_1$-$C_6$-alkyle, hétéroaryloxy-$C_1$-$C_6$-alkyle, aryl-$C_1$-$C_3$-alkylènoxy-$C_1$-$C_6$-alkyle, $R_4R_5NC(O)$-$C_1$-$C_3$-alkylène, aryle, hétéroaryle, aryl-$C_1$-$C_6$-alkylène ou hétéroaryl-$C_1$-$C_6$-alkylène ;
lesdits groupes hétéroaryle ou aryle de $R_{1b}$ étant éventuellement substitués par un ou plusieurs substituants $R_9$, identiques ou différents l'un de l'autre ;
$R_2$ représente un atome d'hydrogène ;
$R_3$ représente de un atome ou groupe choisi parmi un atome d'hydrogène, un groupe $C_1$-$C_6$-alkyle, $C_1$-$C_6$-alcoxy ou hydroxyle, lorsque $R_3$ est porté par un atome de carbone ;
ou
$R_3$ représente de un atome ou groupe choisi parmi un atome d'hydrogène, un groupe $C_1$-$C_6$-alkyle, $C_1$-$C_6$-alkyle-O-C(O)-, lorsque $R_3$ est porté par un atome d'azote ;
$R_4$ et $R_5$, représentent, indépendamment l'un de l'autre, un groupe $C_1$-$C_6$-alkyle ;
ou $R_4$ et $R_5$ forment ensemble, avec l'atome d'azote qui les porte, un groupe pyrrolidine ou morpholine ;
$R_9$ représente un atome d'halogène ou un groupe $C_1$-$C_6$-alkyle, $C_1$-$C_6$-alcoxy, $C_1$-$C_6$-fluoroalkyle, aryle, hétéroaryle, $NR_4R_5$, arylthio, lesdits groupes aryle étant éventuellement substitués par un ou plusieurs groupes $C_1$-$C_6$-alkyle ;

à l'état de base ou de sel d'addition à un acide, ainsi qu'à l'état d'hydrate ou de solvate.

**9.** Composé de l'invention selon l'une quelconque des revendications 1 à 8, **caractérisé en ce qu'**il répond à la formule générale (I) dans laquelle à la fois $R_1$ et/ou $R_2$ et/ou $R_3$, et/ou A, et/ou P et/ou Y sont tels que définis dans les revendications 1 à 8 ;
à l'état de base ou de sel d'addition à un acide, ainsi qu'à l'état d'hydrate ou de solvate.

**10.** Procédé de préparation d'un composé de formule (I) selon l'une quelconque des revendications 1 à 9, **caractérisé**

**en ce que** l'on fait réagir un composé de formule générale (VI) :

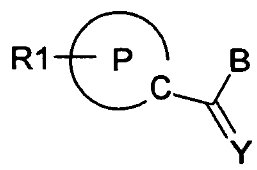

**(VI)**

dans laquelle P, $R_1$ et Y sont tels que définis dans la formule générale (I) selon la revendication 1 et B représente un groupe $C_1$-$C_6$-alcoxy, $C_3$-$C_7$-cycloalkyloxy, $C_3$-$C_7$-cycloalkyle-$C_1$-$C_3$-alkylénoxy, aryle-$C_1$-$C_3$-alkylénoxy, avec un amidure du composé de formule générale (VII) :

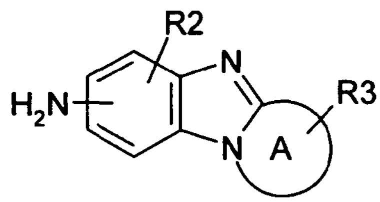

**(VII)**

dans laquelle A, $R_2$ et $R_3$ sont tels que définis dans la formule générale (I) selon la revendication 1, au reflux d'un solvant, l'amidure du composé de formule générale (VII) étant préparé par action préalable du triméthylaluminium sur les amines de formule générale (VII).

**11.** Procédé de préparation d'un composé de formule (I) selon l'une quelconque des revendications 1 à 9, **caractérisé en ce que** l'on transforme un composé de formule générale (VI) :

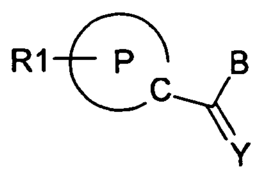

**(VI)**

dans laquelle P, $R_1$ et Y sont tels que définis dans la formule générale (I) selon la revendication 1 et B représente un groupe hydroxyle, en chlorure d'acide par action du chlorure de thionyle au reflux d'un solvant, puis **en ce que** l'on fait réagir, en présence d'une base, le composé de formule générale (VI) obtenu, dans laquelle P, $R_1$ et Y sont tels que définis dans la formule générale (I) selon la revendication 1 et B représente un atome de chlore, avec le composé de formule générale (VII) :

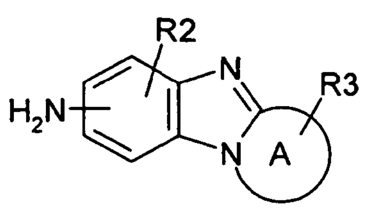

**(VII)**

dans laquelle A, $R_2$ et $R_3$ sont tels que définis dans la formule générale (I) selon la revendication 1 ;
ou bien **en ce que** l'on effectue une réaction de couplage entre un composé de formule générale (VI) dans laquelle P, $R_1$ et Y sont tels que définis dans la formule générale (I) selon la revendication 1 et B représente un groupe

hydroxyle, et le composé de formule générale (VII) dans laquelle A, $R_2$ et $R_3$ sont tels que définis dans la formule générale (I) selon la revendication 1, en présence d'un agent de couplage et d'une base, dans un solvant.

**12.** Composé de formule générale (VIIa), (VIIb), (VIIc), (VIId), (VIIe), (VIIf), (VIIg), (VIIh), (VIIi), (VIIj), (VIIk), (VIIl), (VIIm), (VIIn) :

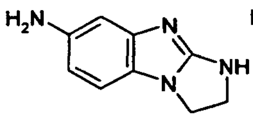
(VII-a)

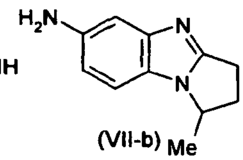
(VII-b)

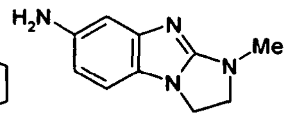
(VII-c)

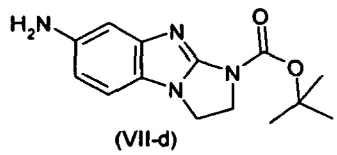
(VII-d)

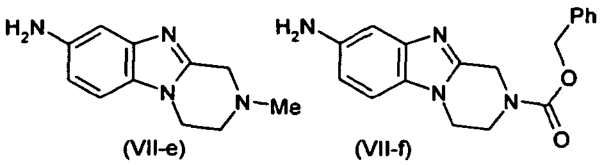
(VII-e) (VII-f)

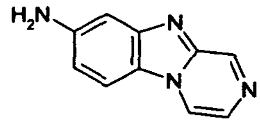
(VII-g)

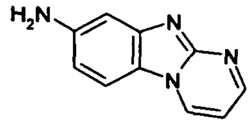
(VII-h)

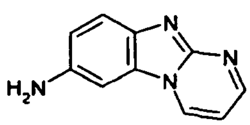
(VII-i)

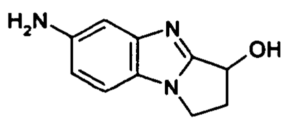
(VII-j)

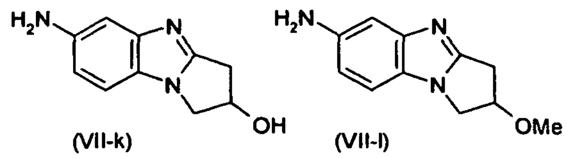
(VII-k) (VII-l)

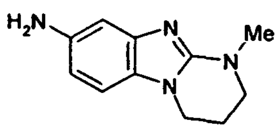
(VII-m)

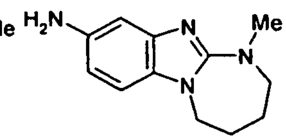
(VII-n)

**13.** Médicament, **caractérisé en ce qu'**il comprend au moins un composé de formule (I) selon l'une quelconque des revendications 1 à 9, ou un sel d'addition de ce composé à un acide pharmaceutiquement acceptable, ou encore un hydrate ou un solvate du composé de formule (I).

**14.** Composition pharmaceutique, **caractérisée en ce qu'**elle comprend au moins un composé de formule (I) selon l'une quelconque des revendications 1 à 9, ou un sel pharmaceutiquement acceptable, un hydrate ou un solvate de ce composé, ainsi qu'au moins un excipient pharmaceutiquement acceptable.

**15.** Utilisation d'un composé de formule (I) selon l'une quelconque des revendications 1 à 9 pour la préparation d'un médicament destiné à la prévention et au traitement des pathologies dans lesquelles les récepteurs de type TRPV1 sont impliqués.

**16.** Utilisation d'un composé de formule (I) selon l'une quelconque des revendications 1 à 9 pour la préparation d'un médicament destiné à prévenir ou à traiter la douleur, l'inflammation, les désordres métaboliques, les désordres urologiques, les désordres gynécologiques, les désordres gastro-intestinaux, les désordres respiratoires, le psoriasis, le pruritis, les irritations dermiques, des yeux ou des muqueuses, l'herpès, le zona, la sclérose en plaques et la dépression.

## Claims

**1.** Compound corresponding to formula (I)

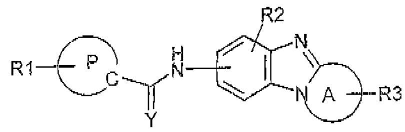

(I)

in which:

A is, with the C-N bond of the benzimidazole unit with which it is fused, a 4- to 7-membered monocyclic heterocycle or monocyclic heteroaryl, containing from one to three heteroatoms selected from O, S and N, including the nitrogen atom of the benzimidazole, unit;
P is an 8-, 9-, 10- or 11-membered bicyclic heterocycle or bicyclic heteroaryl, comprising from 1 to 6 heteroatoms selected from N, O and S; P being linked to the -C(Y)- group by a carbon atom;
with the proviso that, when A is a 7-membered saturated heterocycle, P is other than the 2,3-dihydro-1,4-benzodioxane group, the 1-benzopyran-2-one group and the isoindole group;
$R_1$ is from one to four atoms or groups, which may be identical or different, selected from a hydrogen atom, a halogen atom, and an oxo, thio, $C_1$-$C_6$-alkyl, $C_3$-$C_7$-cycloalkyl, $C_3$-$C_7$-cycloalkyl-$C_1$-$C_3$-alkylene, $C_1$-$C_6$-fluoroalkyl, aryloxy-$C_1$-$C_6$-alkyl, heteroaryloxy-$C_1$-$C_6$-alkyl, aryl-$C_1$-$C_3$-alkylenoxy-$C_1$-$C_6$-alkyl, heteroaryl-$C_1$-$C_3$-alkylenoxy-$C_1$-$C_6$-alkyl, arylthio-$C_1$-$C_6$-alkyl, heteroarylthio-$C_1$-$C_6$-alkyl, aryl-$C_1$-$C_3$-alkylenethio-$C_1$-$C_6$-alkyl, hetaroaryl-$C_1$-$C_3$-alkylanathio-$C_1$-$C_6$-alkyl, $C_1$-$C_6$-alkoxy, $C_3$-$C_7$-cycloalkyloxy, $C_3$-$C_7$-cycloalkyl-$C_1$-$C_3$-alkylenoxy, $C_1$-$C_6$-fluoroalkoxy, cyano, C(O)$NR_4R_5$, nitro, $NR_4R_5$, $C_1$-$C_6$-thioalkyl, $C_3$-$C_7$-cycloalkylthio, $C_3$-$C_7$-cycloalkyl-$C_1$-$C_3$-alkylenethio, -S(O)-$C_1$-$C_6$-alkyl, -S(O)-$C_3$-$C_7$-cycloalkyl, -S(O)-$C_1$-$C_3$-alkylene-$C_3$-$C_7$-cycloalkyl, $C_1$-$C_6$-alkyl-$S(O)_2$-, $C_1$-$C_6$-fluoroalkyl-$S(O)_2$-, $C_3$-$C_7$-cycloalkyl$S(O)_2$-, $C_3$-$C_7$-cycloalkyl-$C_1$-$C_3$-alkylene-$S(O)_2$-, $SO_2NR_4R_5$, $SF_5$, $NR_6C(O)R_7$, $NR_6SO_2R_8$, $R_4R_5NC(O)$-$C_1$-$C_3$-alkylene, aryl, heteroaryl, aryl-$C_1$-$C_5$-alkylene, heteroaryl-$C_1$-$C_5$-alkylene, aryloxy, arylthio, heteroaryloxy or heteroarylthio group;
said heteroaryl or aryl groups of $R_1$ being optionally substituted with one or more substituents $R_9$, which may be identical to or different from one another;
with the proviso that, when $R_1$ is attached to a nitrogen atom of P, then $R_1$ is different from a halogen atom, and from an oxo, thio, cyano, nitro, $SF_5$, $NR_4R_5$, $C_1$-$C_6$-thioalkyl, thioaryl, thioheteroaryl, $C_1$-$C_6$-alkoxy, aryloxy, heteroaryloxy, -$NR_6COR_7$ and $NR_6SO_2R_8$ group;
Y is an oxygen or sulphur atom;
$R_2$ is a hydrogen atom, a halogen atom, or a $C_1$-$C_6$-alkyl, $C_3$-$C_7$-cycloalkyl, $C_3$-$C_7$-cycloalkyl-$C_1$-$C_3$-alkylene, $C_1$-$C_6$-fluoroalkyl or $C_1$-$C_6$-alkoxy group;
$R_3$ is from one to three atoms or groups, which may be identical or different, selected from a hydrogen atom, a halogen atom, and a $C_1$-$C_6$-alkyl, $C_3$-$C_7$-cycloalkyl, $C_3$-$C_7$-cycloalkyl-$C_1$-$C_3$-alkylene, $C_1$-$C_6$-fluoroalkyl, hydroxyl, $C_1$-$C_6$-alkoxy, $C_3$-$C_7$-cycloalkyloxy or $C_3$-$C_7$-cycloalkyl-$C_1$-$C_3$-alkylenoxy group, when $R_3$ is carried by a carbon atom;
or
$R_3$ is from one to two atoms or groups, which may be identical or different, selected from a hydrogen atom, a halogen atom, and a $C_1$-$C_6$-alkyl, $C_3$-$C_7$-cycloalkyl, $C_3$-$C_7$-cycloalkyl-$C_1$-$C_3$-alkylene, $C_1$-$C_6$-fluoroalkyl, aryl-C(O)-, $C_1$-$C_6$-alkyl-C(O)-, $C_3$-$C_7$-cycloalkyl-C(O)-, $C_3$-$C_7$-cycloalkyl-$C_1$-$C_3$-alkylene-C(O)-,$C_1$-$C_6$-fluoroalkyl-C(O)-, aryl-$S(O)_2$-, $C_1$-$C_6$-alkyl-$S(O)_2$-, $C_1$-$C_6$-fluoroalkyl-$S(O)_2$-, $C_3$-$C_7$-cycloalkyl-$S(O)_2$-,$C_3$-$C_7$-cycloalkyl-$C_1$-$C_3$-alkylene-$S(O)_2$-, $C_1$-$C_6$-alkyl-O-C(O)-, aryl-$C_1$-$C_3$-alkyl-O-C(O)-, $C_3$-$C_7$-cycloalkyl-O-C(O)-, $C_3$-$C_7$-cycloalkyl-$C_1$-$C_3$-alkylene-O-C(O)-, $C_1$-$C_6$-fluoroalkyl-O-C(O)-, aryl-O-C(O)- or heteroaryl-O-C(O)- group, when $R_3$ is carried by a nitrogen atom;
$R_4$ and $R_5$ are, independently of one another, a hydrogen atom or a $C_1$-$C_6$-alkyl, $C_3$-$C_7$-cycloalkyl, $C_3$-$C_7$-cycloalkyl-$C_1$-$C_3$-alkylene, aryl-$C_1$-$C_5$-alkylene or aryl group; or $R_4$ and $R_5$ form, together with the nitrogen atom which carries them, an azetidine, pyrrolidine, piperidine, azepine, morpholine, thiomorpholine, piperazine or homopiperazine group, the $NR_4R_5$ group being optionally substituted with a $C_1$-$C_6$-alkyl, $C_3$-$C_7$-cycloalkyl, $C_3$-$C_7$-cycloalkyl-$C_1$-$C_3$-alkylene, aryl-$C_1$-$C_6$-alkylene, aryl, heteroaryl, aryl-$S(O)_2$-, $C_1$-$C_6$-alkyl-$S(O)_2$-, $C_1$-$C_6$-fluoroalkyl-$S(O)_2$-,$C_3$-$C_7$-cycloalkyl-$S(O)_2$-, $C_3$-$C_7$-cycloalkyl-$C_1$-$C_3$-alkylene-$S(O)_2$-,aryl-C(O)-, $C_1$-$C_6$-alkyl-C(O)-, $C_3$-$C_7$-cycloalkyl-C(O)-, $C_3$-$C_7$-cycloalkyl-$C_1$-$C_3$-alkylene-C(O)-, $C_1$-$C_6$-fluoroalkyl-C(O)-, hydroxyl, $C_1$-$C_6$-alkyloxy, $C_1$-$C_6$-fluoroalkyl, aryloxy-$C_1$-$C_6$-alkylene, aryloxy, heteroaryloxy-$C_1$-$C_6$-alkylene or heteroaryloxy group;

$R_6$ and $R_7$ are, independently of one another, a hydrogen atom, or a $C_1$-$C_6$-alkyl, $C_3$-$C_7$-cycloalkyl, $C_3$-$C_7$-cycloalkyl-$C_1$-$C_3$-alkylene, aryl-$C_1$-$C_6$-alkylene or aryl group; or $R_6$ and $R_7$ together form a 4- to 7-membered lactam comprising the nitrogen atom and the C(O) group which carry them;
$R_8$ is a $C_1$-$C_6$-alkyl, $C_3$-$C_7$-cycloalkyl, $C_3$-$C_7$-cycloalkyl-$C_1$-$C_3$-alkylene, aryl-$C_1$-$C_6$-alkylene or aryl group; or $R_6$ and $R_8$ together form a 4- to 7-membered sultam comprising the nitrogen atom and the $S(O)_2$ group which carry them;
$R_9$ is a halogen atom, or a $C_1$-$C_6$-alkyl, $C_3$-$C_7$-cycloalkyl, $C_3$-$C_7$-cycloalkyl-$C_1$-$C_3$-alkylene, $C_1$-$C_6$-fluoroalkyl, $C_1$-$C_6$-alkoxy, $C_1$-$C_6$-fluoroalkoxy, nitro, cyano, $NR_4R_5$, $R_4R_5N$-$C_1$-$C_3$-alkylene, aryl, heteroaryl, aryloxy, arylthio, heteroaryloxy or heteroarylthio group, said heteroaryl or aryl groups being optionally substituted with one or more substituents selected from a halogen atom, and a $C_1$-$C_6$-alkyl, $C_3$-$C_7$-cycloalkyl, $C_3$-$C_7$-cycloalkyl-$C_1$-$C_3$-alkylene, $C_1$-$C_6$-fluoroalkyl, $C_1$-$C_6$-alkoxy, $C_1$-$C_6$-fluoro-alkoxy, nitro, cyano, $NR_4R_5$ or $R_4R_5N$-$C_1$-$C_3$-alkylene group;

the sulphur atom(s) of the heterocycle A may be in oxidized form (S(O) or $S(O)_2$);
the nitrogen atom(s) may be in oxidized form (N-oxide); in the form of a base or of an addition salt with an acid, and also in the form of a hydrate or of a solvate;
2-(2-chlorophenyl)-1,3-dioxo-N-(7,8,9,10-tetrahydro-6H-azepino[1,2-a]benzimidazol-3-yl)isoindoline-5-carboxamide being excluded.

2. Compound of the invention according to Claim 1, **characterized in that** it corresponds to formula (II):

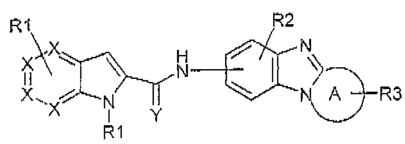
(II)

in which:

X is a carbon atom or a nitrogen atom; said X being identical to or different from one another and the number of X = N not being greater than 2;
$R_1$, $R_2$, $R_3$, Y and A being as defined in formula (I) according to Claim 1, it being possible for $R_1$ to be linked to the 6-element or 5-element unit of the bicycle;

in the form of a base or of an addition salt with an acid, and also in the form of a hydrate or of a solvate.

3. Compound of the invention according to Claim 1 or 2, **characterized in that** it corresponds to formula (III):

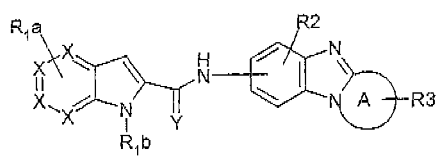
(III)

in which:

$R_{1a}$ is one or more atoms or groups, which may be identical or different, selected from a hydrogen atom, a halogen atom or a $C_1$-$C_6$-alkyl, $C_1$-$C_6$-fluoroalkyl, $C_1$-$C_6$-alkoxy, $C_1$-$C_6$-thioalkyl, $C_1$-$C_6$-alkyl-$S(O)_2$-, $NR_4R_5$ or nitro group;
$R_{1b}$ is a hydrogen atom, or a $C_1$-$C_6$-alkyl, heteroaryloxy-$C_1$-$C_6$-alkyl, aryl-$C_1$-$C_3$-alkylenoxy-$C_1$-$C_6$-alkyl, $R_4R_5NC(O)$-$C_1$-$C_3$-alkylene, aryl, heteroaryl, aryl-$C_1$-$C_6$-alkylene or heteroaryl-$C_1$-$C_6$-alkylene group;
said heteroaryl or aryl groups of $R_{1b}$, being optionally substituted with one or more substituents $R_9$, which may be identical to or different from one another;
$R_9$ is a halogen atom or a $C_1$-$C_6$-alkyl, $C_1$-$C_6$-alkoxy, $C_1$-$C_6$-fluoroalkyl, aryl, heteroaryl, $NR_4R_5$ or arylthio group, said heteroaryl or aryl groups being optionally substituted with one or more substituents selected from a halogen atom, and a $C_1$-$C_6$-alkyl, $C_3$-$C_7$-cycloalkyl, $C_3$-$C_7$-cycloalkyl-$C_1$-$C_3$-alkylene, $C_1$-$C_6$-fluoroalkyl, $C_1$-$C_6$-alkoxy, $C_1$-$C_6$-fluoroalkoxy, nitro, cyano or $R_4R_5N$-$C_1$-$C_3$-alkylene group;

$R_2$, $R_3$, $R_4$, $R_5$, A, X and Y being as defined in formula (II) according to Claim 2;

in the form of a base or of an addition salt with an acid, and also in the form of a hydrate or of a solvate.

**4.** Compound of the invention according to any one of Claims 1 to 3, **characterized in that** it corresponds to formula (IV):

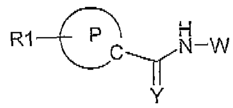

(IV)

in which W is a tricyclic heterocycle or a tricyclic heteroaryl selected from:

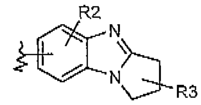
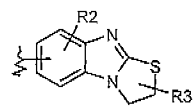
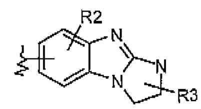
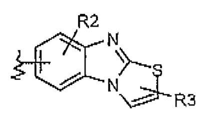
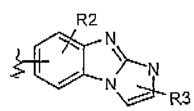
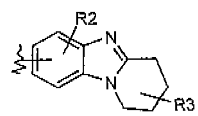
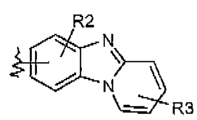
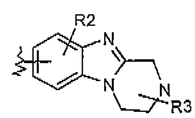
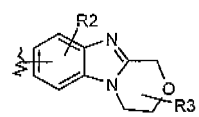
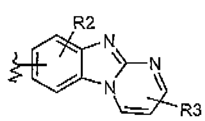
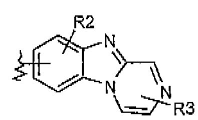
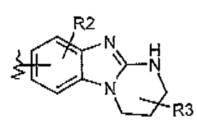
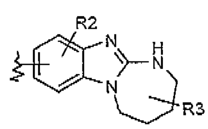
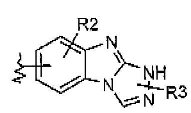

$R_1$, $R_2$, $R_3$, P and Y being as defined in formula (I) according to Claim 1;

in the form of a base or of an addition salt with an acid, and also in the form of a hydrate or of a solvate.

**5.** Compound of the invention according to any one of Claims 1 to 4, **characterized in that** it corresponds to formula (V)

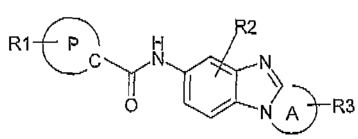

(V)

in which:

$R_1$, $R_2$, $R_3$, A and P are as defined in formula (I) according to Claim 1;

in the form of a base or of an addition salt with an acid, and also in the form of a hydrate or of a solvate.

**6.** Compound of the invention according to any one of Claims 1 to 5, **characterized in that** it corresponds to formula (V)

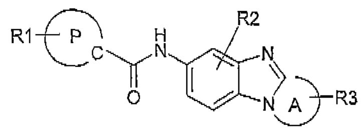

(V)

in which:

$R_2$ is a hydrogen atom, or a $C_1$-$C_6$-alkyl or $C_1$-$C_6$-alkoxy group;
$R_3$ is from one to three atoms or groups, which may be identical or different, selected from a hydrogen atom, and a $C_1$-$C_6$-alkyl, $C_1$-$C_6$-alkoxy or hydroxyl group, when $R_3$ is carried by a carbon atom;
or
$R_3$ is from one to two atoms or groups, which may be identical or different, selected from a hydrogen atom, and a $C_1$-$C_6$-alkyl., $C_1$-$C_6$-alkyl-O-C(O)- or aryl-$C_1$-$C_3$-alkyl-O-C(O)- group, when $R_3$ is carried by a nitrogen atom;
$R_1$, A and P being as defined in formula (I) according to Claim 1;

in the form of a base or of an addition salt with an acid, and also in the form of a hydrate or of a solvate.

**7.** Compound of the invention according to any one of Claims 1 to 6, **characterized in that** it corresponds to formula (V)

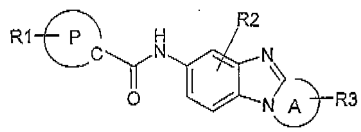

(V)

in which:

A is, with the C-N bond of the benzimidazole unit with which it is fused, a 5- to 7-membered monocyclic heterocycle or monocyclic heteroaryl containing from one to three heteroatoms selected from O, S and N, including the nitrogen atom of the benzimidazole unit;
$R_1$, $R_2$, $R_3$ and P being as defined in formula (I) according to Claim 1;

in the form of a base or of an addition salt with an acid, and also in the form of a hydrate or of a solvate.

**8.** Compound of the invention according to any one of Claims 1 to 7, **characterized in that** it corresponds to formula (Va):

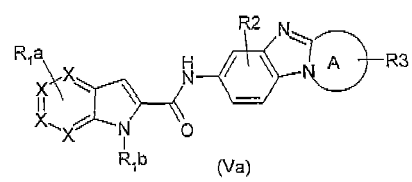

(Va)

in which
A is, with the C-N bond of the benzimidazole unit with which it is fused, a 5- to 7-membered monocyclic heterocycle or monocyclic heteroaryl containing one or two heteroatoms selected from O, S and N, including the nitrogen atom of the benzimidazole unit;
X is a carbon atom or a nitrogen atom; said X being identical to or different from one another and the number of X = N not being greater than 1;
$R_{1a}$ is one or more atoms or groups, which may be identical or different, selected from a hydrogen atom, a halogen atom or a $C_1$-$C_6$-alkyl group, a $C_1$-$C_6$-fluoroalkyl group, a $C_1$-$C_6$-alkoxy group, a $C_1$-$C_6$-thioalkyl group, a $C_1$-$C_6$-alkyl-$S(O)_2$- group, an $NR_4R_5$ group or a nitro group;
$R_{1b}$ is a hydrogen atom, or a $C_1$-$C_6$-alkyl, heteroaryloxy-$C_1$-$C_6$-alkyl, aryl-$C_1$-$C_3$-alkylenoxy-$C_1$-$C_6$-alkyl, $R_4R_5NC$

(O)-$C_1$-$C_3$-alkylene, aryl, heteroaryl, aryl-$C_1$-$C_6$-alkylene or heteroaryl-$C_1$-$C_6$-alkylene group; said heteroaryl or aryl groups of $R_{1b}$ being optionally substituted with one or more substituents $R_9$, which may be identical to or different from one another;
$R_2$ is a hydrogen atom;
$R_3$ is an atom or group selected from a hydrogen atom, a $C_1$-$C_6$-alkyl group, a $C_1$-$C_6$-alkoxy group, or a hydroxyl, group, when $R_3$ is carried by a carbon atom;
or
$R_3$ is an atom or group selected from a hydrogen atom, a $C_1$-$C_6$-alkyl group and a $C_1$-$C_6$-alkyl-O-C(O)- group, when $R_3$ is carried by a nitrogen atom;
$R_4$ and $R_5$ are, independently of one another, a $C_1$-$C_6$-alkyl group;
or $R_4$ and $R_5$ form, together with the nitrogen atom which carries them, a pyrrolidine or morpholine group;
$R_9$ is a halogen atom or a $C_1$-$C_6$-alkyl, $C_1$-$C_6$-alkoxy, $C_1$-$C_6$-fluoroalkyl, aryl, heteroaryl, $NR_4R_5$, or arylthio group, said aryl groups being optionally substituted with one or more $C_1$-$C_6$-alkyl groups;
in the form of a base or of an addition salt with an acid, and also in the form of a hydrate or of a solvate.

**9.** Compound of the invention according to any one of Claims 1 to 8, **characterized in that** it corresponds to formula (I) in which at the same time $R_1$ and/or $R_2$ and/or $R_3$, and/or A, and/or P and/or Y are as defined in Claims 1 to 8; in the form of a base or of an addition salt with an acid, and also in the form of a hydrate or of a solvate.

**10.** Process for preparing a compound of formula (I) according to any one of Claims 1 to 9, **characterized in that** a compound of formula (VI):

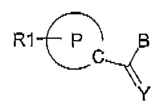

(VI)

in which P, $R_1$ and Y are as defined in formula (I) according to Claim 1 and B is a $C_1$-$C_6$-alkoxy, $C_3$-$C_7$-cycloalkyloxy, $C_3$-$C_7$-cycloalkyl-$C_1$-$C_3$-alkylenoxy or aryl-$C_1$-$C_3$-alkylenoxy group, with an amide of the compound of formula (VII):

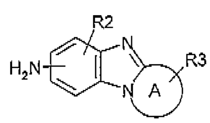

(VII)

in which A, $R_2$ and $R_3$ are as defined in formula (I) according to Claim 1, at the reflux of a solvent, the amide of the compound of formula (VII) being prepared by the prior action of trimethylaluminium on the amines of formula (VII).

**11.** Process for preparing a compound of formula (I) according to any one of Claims 1 to 9, **characterized in that** a compound of formula (VI):

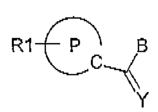

(VI)

in which P, $R_1$ and Y are as defined in formula (I) according to Claim 1 and B is a hydroxyl group, is converted to an acid chloride by the action of thionyl chloride at the reflux of a solvent, and then **in that** the compound of formula (VI) obtained, in which P, $R_1$ and Y are as defined in formula (I) according to Claim 1 and B is a chlorine atom, is reacted, in the presence of a base, with the compound of formula (VII):

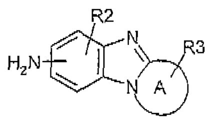

(VII)

in which A, $R_2$ and $R_3$ are as defined in formula (I) according to Claim 1;
or else **in that** a coupling reaction is carried out between a compound of formula (VI), in which P, $R_1$ and Y are as defined in formula (I) according to Claim 1 and B is a hydroxyl group, and the compound of formula (VII) in which A, $R_2$ and $R_3$ are as defined in formula (I) according to Claim 1, in the presence of a coupling agent and of a base, in a solvent.

**12.** Compound of formula (VIIa), (VIIb), (VIIc), (VIId), (VIIe), (VIIf), (VIIg), (VIIh), (VIIi), (VIIj), VIIk), (VIIl), (VIIm), (VIIn):

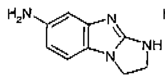
(VII-a)
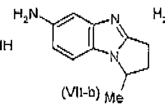
(VII-b)
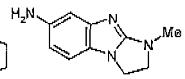
(VII-c)
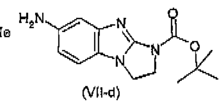
(VII-d)

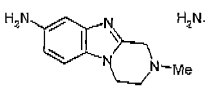
(VII-e)
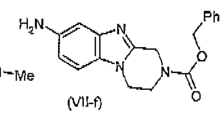
(VII-f)
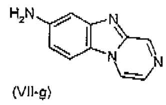
(VII-g)
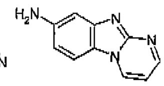
(VII-h)

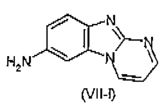
(VII-I)
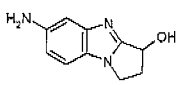
(VII-j)
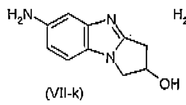
(VII-k)
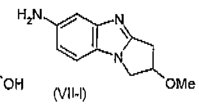
(VII-l)

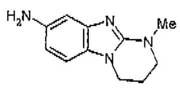
(VII-m)
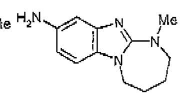
(VII-n)

**13.** Medicament, **characterized in that** it comprises at least one compound of formula (I) according to any one of Claims 1 to 9, or an addition salt of this compound with a pharmaceutically acceptable acid, or else a hydrate or a solvate of the compound of formula (I).

**14.** Pharmaceutical composition, **characterized in that** it comprises at least one compound of formula (I) according to any one of Claims 1 to 9, or a pharmaceutically acceptable salt, a hydrate or a solvate of this compound, and also at least one pharmaceutically acceptable excipient.

**15.** Use of a compound of formula (I) according to any one of Claims 1 to 9, for the preparation of a medicament for use in the prevention or treatment of pathologies in which TPPV1-type receptors are involved.

**16.** Use of a compound of formula (I) according to any one of Claims 1 to 9, for the preparation of a medicament for use in preventing or treating pain, inflammation, metabolic disorders, urological disorders, gynaecological disorders, gastrointestinal disorders, respiratory disorders, psoriasis, pruritis, dermal, ocular or mucosal irritations, herpes, shingles, multiple sclerosis and depression.

## Patentansprüche

**1.** Verbindung der allgemeinen Formel (I)

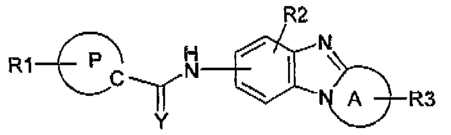

(I)

worin:

A mit der C-N-Bindung der Benzimidazol-Einheit, mit der es kondensiert ist, für einen 4- bis 7-gliedrigen monocyclischen Heterocyclus oder ein 4- bis 7-gliedriges monocyclisches Heteroaryl mit einem bis drei unter O, S oder N ausgewählten Heteroatomen einschließlich des Stickstoffatoms der Benzimidazol-Einheit steht;

P für einen 8-, 9-, 10- oder 11-gliedrigen bicyclischen Heterocyclus oder ein 8-, 9-, 10- oder 11-gliedriges bicyclisches Heteroaryl mit 1 bis 6 unter N, O und S ausgewählten Heteroatomen steht; wobei P über ein Kohlenstoffatom an die -C(Y)-Gruppe gebunden ist;

mit der Maßgabe, daß dann, wenn A für einen 7-gliedrigen gesättigten Heterocyclus steht, P von der 2,3-Dihydro-1,4-benzodioxangruppe, der 1-Benzopyran-2-ongruppe und der Isoindolgruppe verschieden ist;

$R_1$ für ein bis vier gleiche oder verschiedene Atome oder eine bis vier gleiche oder verschiedene Gruppen, die unter einem Wasserstoffatom, einem Halogenatom und einer Oxo-, Thio-, $C_1$-$C_6$-Alkyl-, $C_3$-$C_7$-Cycloalkyl-, $C_3$-$C_7$-Cycloalkyl-$C_1$-$C_3$-alkylen-, $C_1$-$C_6$-Fluoralkyl-, Aryloxy-$C_1$-$C_6$-alkyl-, Heteroaryloxy-$C_1$-$C_6$-alkyl-, Aryl-$C_1$-$C_3$-alkylenoxy-$C_1$-$C_6$-alkyl-, Heteroaryl-$C_1$-$C_3$-alkylenoxy-$C_1$-$C_6$-alkyl-, Arylthio-$C_1$-$C_6$-alkyl-, Heteroarylthio-$C_1$-$C_6$-alkyl, Aryl-$C_1$-$C_3$-alkylenthio-$C_1$-$C_6$-alkyl-, Heteroaryl-$C_1$-$C_3$-alkylenthio-$C_1$-$C_6$-alkyl-, $C_1$-$C_6$-Alkoxy-, $C_3$-$C_7$-Cycloalkyloxy-, $C_3$-$C_7$-Cycloalkyl-$C_1$-$C_3$-alkylenoxy-, $C_1$-$C_6$-Fluoralkoxy-, Cyano-, C(O)$NR_4R_5$-, Nitro-, $NR_4R_5$-, $C_1$-$C_6$-Thioalkyl-, $C_3$-$C_7$-Cycloalkylthio-, $C_3$-$C_7$-Cycloalkyl-$C_1$-$C_3$-alkylenthio-, -S(O)-$C_1$-$C_6$-Alkyl-, -S(O)-$C_3$-$C_7$-Cycloalkyl-, -S(O)-$C_1$-$C_3$-Alkylen-$C_3$-$C_7$-cycloalkyl-, $C_1$-$C_6$-Alkyl-$S(O)_2$-, $C_1$-$C_6$-Fluoralkyl-$S(O)_2$-, $C_3$-$C_7$-Cycloalkyl-$S(O)_2$-, $C_3$-$C_7$-Cycloalkyl-$C_1$-$C_3$-alkylen-$S(O)_2$-, $SO_2NR_9R_5$-, $SF_5$-, $NR_6C(O)R_7$-,$NR_6SO_2R_8$-, $R_4R_5NC(O)$-$C_1$-$C_3$-Alkylan-, Aryl-, Heteroaryl-, Aryl-$C_1$-$C_5$-alkylen-, Heteroaryl-$C_1$-$C_5$-alkylen-, Aryloxy-, Arylthio-, Heteroaryloxy- oder Heteroarylthiogruppe ausgewählt sind, steht;

wobei die Heteroaryl- oder Arylgruppen von $R_1$ gegebenenfalls durch einen oder mehrere gleiche oder voneinander verschiedene Substituenten $R_9$ substituiert sind;

mit der Maßgabe, daß $R_1$ dann, wenn es an ein Stickstoffatom von P gebunden ist, von einem Halogenatom und einer Oxo-, Thio-, Cyano-, Nitro-, $SF_5$-, $NR_4R_5$-, $C_1$-$C_6$-Thioalkyl-, Thioaryl-, Thioheteroaryl-, $C_1$-$C_6$-Alkoxy-, Aryloxy-, Heteroaryloxy-, $-NR_6COR_7$- und $NR_6SO_2R_8$-Gruppe verschieden ist;

Y für ein Sauerstoff- oder Schwefelatom steht;

$R_2$ für ein Wasserstoffatom, ein Halogenatom oder eine $C_1$-$C_6$-Alkyl-, $C_3$-$C_7$-Cycloalkyl-, $C_3$-$C_7$-Cycloalkyl-$C_1$-$C_3$-alkylen-, $C_1$-$C_6$-Fluoralkyl- oder $C_1$-$C_6$-Alkoxygruppe steht;

$R_3$ für ein bis drei gleiche oder verschiedene Atome oder eine bis drei gleiche oder verschiedene Gruppen, die unter einem Wasserstoffatom, einem Halogenatom und einer $C_1$-$C_6$-Alkyl-, $C_3$-$C_7$-Cycloalkyl-, $C_3$-$C_7$-Cycloalkyl-$C_1$-$C_3$-alkylen-, $C_1$-$C_6$-Fluoralkyl-, Hydroxyl-, $C_1$-$C_6$-Alkoxy-, $C_3$-$C_7$-Cycloalkyloxy- oder $C_3$-$C_7$-Cycloalkyl-$C_1$-$C_3$-alkylenoxygruppe ausgewählt sind, steht, wenn $R_3$ an ein Kohlenstoffatom gebunden ist;

oder

$R_3$ für ein oder zwei gleiche oder verschiedene Atome oder eine oder zwei gleiche oder verschiedene Gruppen, die unter einem Wasserstoffatom, einem Halogenatom und einer $C_1$-$C_6$-Alkyl-, $C_3$-$C_7$-Cycloalkyl-, $C_3$-$C_7$-Cycloalkyl-$C_1$-$C_3$-alkylen-, $C_1$-$C_6$-Fluoralkyl-, Aryl-C(O)-, $C_1$-$C_6$-Alkyl-C(O)-, $C_3$-$C_7$-Cycloalkyl-C(O)-, $C_3$-$C_7$-Cycloalkyl-$C_1$-$C_3$-alkylen-C(O)-, $C_1$-$C_6$-Fluoralkyl-C(O)-, Aryl-$S(O)_2$-, $C_1$-$C_6$-Alkyl-$S(O)_2$-, $C_1$-$C_6$-Fluoralkyl-$S(O)_2$-, $C_3$-$C_7$-Cycloalkyl-$S(O)_2$-, $C_3$-$C_7$-Cycloalkyl-$C_1$-$C_3$-alkylen-$S(O)_2$-, $C_1$-$C_6$-Alkyl-O-C(O)-, Aryl-$C_1$-$C_3$-alkyl-O-C(O)-, $C_3$-$C_7$-Cycloalkyl-O-C(O)-, $C_3$-$C_7$-Cycloalkyl-$C_1$-$C_3$-alkylen-O-C(O)-, $C_1$-$C_6$-Fluoralkyl-O-C(O)-, Aryl-O-C(O)- oder Heteroaryl-O-C(O)-Gruppe ausgewählt sind, steht, wenn $R_3$ an ein Stickstoffatom gebunden ist;

$R_4$ und $R_5$ unabhängig voneinander für ein Wasserstoffatom oder eine $C_1$-$C_6$-Alkyl-, $C_3$-$C_7$-Cycloalkyl-, $C_3$-$C_7$-Cycloalkyl-$C_1$-$C_3$-alkylen-, Aryl-$C_1$-$C_5$-alkylen- oder Arylgruppe stehen;

oder $R_4$ und $R_5$ zusammen mit dem Stickstoffatom, an das sie gebunden sind, eine Azetidin-, Pyrrolidin-, Piperidin-, Azepin-, Morpholin-, Thiomorpholin-, Piperazin- oder Homopiperazingruppe bilden, wobei die $NR_4R_5$-Gruppe gegebenenfalls durch eine $C_1$-$C_6$-Alkyl-, $C_3$-$C_7$-Cycloalkyl-, $C_3$-$C_7$-Cycloalkyl-$C_1$-$C_3$-alkylen-, Aryl-$C_1$-$C_6$-alkylen-, Aryl-, Heteroaryl-, Aryl-$S(O)_2$-, $C_1$-$C_6$-Alkyl-$S(O)_2$-, $C_1$-$C_6$-Fluoralkyl-$S(O)_2$-, $C_3$-$C_7$-Cycloalkyl-$S(O)_2$-, $C_3$-$C_7$-Cycloalkyl-$C_1$-$C_3$-alkylen-$S(O)_2$-, Aryl-C(O)-, $C_1$-$C_6$-Alkyl-C(O)-, $C_3$-$C_7$-Cycloalkyl-C(O)-, $C_3$-$C_7$-Cycloalkyl-$C_1$-$C_3$-alkylen-C(O)-, $C_1$-$C_6$-Fluoralkyl-C(O)-, Hydroxyl-, $C_1$-$C_6$-Alkyloxy-, $C_1$-$C_6$-Fluoralkyl-, Aryloxy-$C_1$-$C_6$-alkylen-, Aryloxy-, Heteroaryloxy-$C_1$-$C_6$-alkylen- oder Heteroaryloxygruppe substituiert ist;

$R_6$ und $R_7$ unabhängig voneinander für ein Wasserstoffatom oder eine $C_1$-$C_6$-Alkyl-, $C_3$-$C_7$-Cycloalkyl-, $C_3$-$C_7$-Cycloalkyl-$C_1$-$C_3$-alkylen-, Aryl-$C_1$-$C_6$-alkylen- oder Arylgruppe stehen;

oder $R_6$ und $R_7$ zusammen ein 4- bis 7-gliedriges Lactam, das das Stickstoffatom und die C(O)-Gruppe, an die sie gebunden sind, enthält, bilden;

$R_8$ für eine $C_1$-$C_6$-Alkyl-, $C_3$-$C_7$-Cycloalkyl-, $C_3$-$C_7$-Cycloalkyl-$C_1$-$C_3$-alkylen-, Aryl-$C_1$-$C_6$-alkylen- oder Arylgruppe steht;

oder $R_6$ und $R_8$ zusammen ein 4- bis 7-gliedriges Sultam, das das Stickstoffatom und die $S(O)_2$-Gruppe, an die sie gebunden sind, enthält, bilden;

$R_9$ für ein Halogenatom oder eine $C_1$-$C_6$-Alkyl-, $C_3$-$C_7$-Cycloalkyl-, $C_3$-$C_7$-Cycloalkyl-$C_1$-$C_3$-alkylen-, $C_1$-$C_6$-Fluoralkyl-, $C_1$-$C_6$-Alkoxy-, $C_1$-$C_6$-Fluoralkoxy-, Nitro-, Cyano-, $NR_4R_5$-, $R_4R_5N$-$C_1$-$C_3$-Alkylen-, Aryl-, Heteroaryl-, Aryloxy-, Arylthio-, Heteroaryloxy- oder Heteroarylthiogruppe steht,

wobei die Heteroaryl- oder Arylgruppen gegebenenfalls durch einen oder mehrere Substituenten, die unter einem Halogenatom und einer $C_1$-$C_6$-Alkyl-, $C_3$-$C_7$-Cycloalkyl-, $C_3$-$C_7$-Cycloalkyl-$C_1$-$C_3$-alkylen-, $C_1$-$C_6$-Fluoralkyl-, $C_1$-$C_6$-Alkoxy-, $C_1$-$C_6$-Fluoralkoxy-, Nitro-, Cyano-, $NR_4R_5$- oder $R_4R_5N$-$C_1$-$C_3$-Alkylengruppe ausgewählt sind, substituiert sind;

wobei das oder die Schwefelatome des Heterocyclus A in oxidierter Form (S(O) oder $S(O)_2$) vorliegen können;

wobei das oder die Stickstoffatome in oxidierter Form (N-Oxid) vorliegen können;

in Basen- oder Säureadditionssalzform sowie in Hydrat- oder Solvatform;

wobei 2-(2-Chlorphenyl)-1,3-clioxo-N-(7,8,9,10-tetrahydro-6H-azepino[1,2-a]benzimidazol-3-yl)-isoindolin-5-carboxamid ausgeschlossen ist.

2. Erfindungsgemäße Verbindung nach Anspruch 1, **dadurch gekennzeichnet, daß** sie der allgemeinen Formel (II) entspricht:

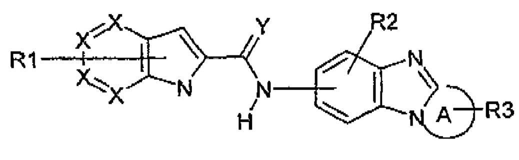
(II)

worin

X für ein Kohlenstoffatom oder ein Stickstoffatom steht; wobei die Variablen X gleich oder voneinander verschieden sind und die Zahl von X = N nicht größer als 2 ist;

$R_1$, $R_2$, $R_3$, Y und A die unter der allgemeinen Formel (I) nach Anspruch 1 angegebene Bedeutung besitzen, wobei $R_1$ an die Einheit mit 6 Elementen oder mit 5 Elementen des Bicyclus gebunden sein kann;

in Basen- oder Säureadditionssalzform sowie in Hydrat- oder Solvatform.

3. Erfindungsgemäße Verbindung nach Anspruch 1 oder 2, **dadurch gekennzeichnet, daß** sie der allgemeinen Formel (III) entspricht:

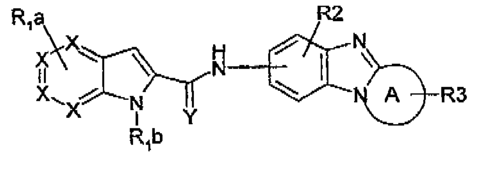
(III)

worin:

$R_{1a}$ für ein oder mehrere gleiche oder verschiedene Atome oder eine oder mehrere gleiche oder verschiedene Gruppen, die unter einem Wasserstoffatom, einem Halogenatom oder einer $C_1$-$C_6$-Alkyl-, $C_1$-$C_6$-Fluoroalkyl-, $C_1$-$C_6$-Alkoxy-, $C_1$-$C_6$-Thioalkyl-, $C_1$-$C_6$-Alkyl-$S(O)_2$-, $NR_4R_5$- oder Nitrogruppe ausgewählt sind, steht;

$R_{1b}$ für ein Wasserstoffatom oder eine $C_1$-$C_6$-Alkyl-, Heteroaryloxy-$C_1$-$C_6$-alkyl-, Aryl-$C_1$-$C_3$-alkylenoxy-$C_1$-$C_6$-alkyl-, $R_4R_5NC(O)$-$C_1$-$C_3$-Alkylen-, Aryl-, Heteroaryl-, Aryl-$C_1$-$C_6$-alkylen- oder Heteroaryl-$C_1$-$C_6$-alkylengruppe steht;

wobei die Heteroaryl- oder Arylgruppen von $R_{1b}$ gegebenenfalls durch einen oder mehrere gleiche oder voneinander verschiedene Substituenten $R_9$ substituiert sind;

$R_9$ für ein Halogenatom oder eine $C_1$-$C_6$-Alkyl-, $C_1$-$C_6$-Alkoxy-, $C_1$-$C_6$-Fluoralkyl-, Aryl-, Heteroaryl-, $NR_4R_5$-

oder Arylthiogruppe steht, wobei die Heteroaryl- oder Arylgruppen gegebenenfalls durch einen oder mehrere unter einem Halogenatom und einer $C_1$-$C_6$-Alkyl-, $C_3$-$C_7$-Cycloalkyl-, $C_3$-$C_7$-Cycloalkyl-$C_1$-$C_3$-alkylen-, $C_1$-$C_6$-Fluoralkyl-, $C_1$-$C_6$-Alkoxy-, $C_1$-$C_8$-Fluoralkoxy-, Nitro-, Cyano- oder $R_4R_5N$-$C_1$-$C_3$-alkylengruppe ausgewählte Substituenten substituiert sind;
$R_2$, $R_3$, $R_4$, $R_5$, A, X und Y die unter der allgemeinen Formel (II) nach Anspruch 2 angegebene Bedeutung besitzen;

in Basen- oder Säureadditionssalzform sowie in Hydrat- oder Solvatform.

4. Erfindungsgemäße Verbindung nach einem der Ansprüche 1 bis 3, **dadurch gekennzeichnet, daß** sie der allgemeinen Formel (IV) entspricht:

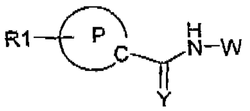 (IV)

worin W für einen tricyclischen Heterocyclus oder ein tricyclisches Heteroaryl steht, der bzw. das unter:

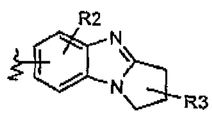
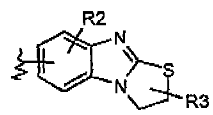
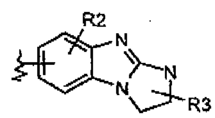
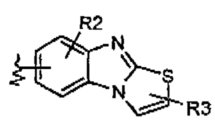
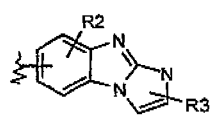
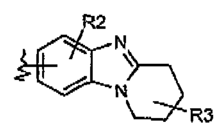
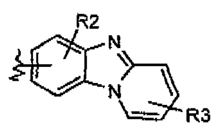
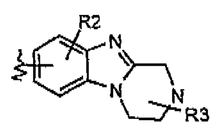
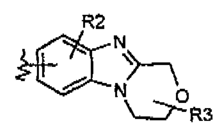
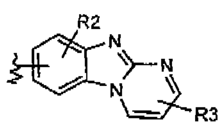
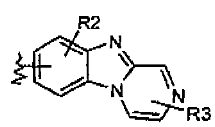
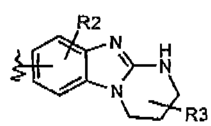
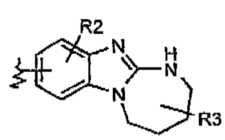
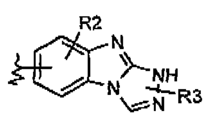

ausgewählt ist, wobei $R_1$, $R_2$, $R_3$, P und Y die unter der allgemeinen Formel (I) nach Anspruch 1 angegebene Bedeutung besitzen;
in Basen- oder Säureadditionssalzform sowie in Hydrat- oder Solvatform.

5. Erfindungsgemäße Verbindung nach einem der Ansprüche 1 bis 4, **dadurch gekennzeichnet, daß** sie der allgemeinen Formel (V) entspricht:

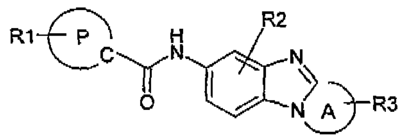

(V)

worin:

$R_1$, $R_2$, $R_3$, A und P die unter der allgemeinen Formel (I) nach Anspruch 1 angegebene Bedeutung besitzen;

in Basen- oder Säureadditionssalzform sowie in Hydrat- oder Solvatform.

**6.** Erfindungsgemäße Verbindung nach einem der Ansprüche 1 bis 5, **dadurch gekennzeichnet, daß** sie der allgemeinen Formel (V) entspricht:

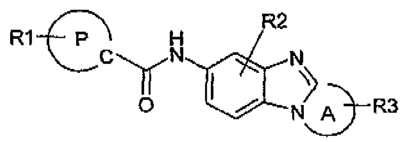

(V)

worin:

$R_2$ für ein Wasserstoffatom oder eine $C_1$-$C_6$-Alkyl- oder $C_1$-$C_6$-Alkoxygruppe steht;
$R_3$ für ein bis drei gleiche oder verschiedene Atome oder eine bis drei gleiche oder verschiedene Gruppen steht, die unter einem Wasserstoffatom und einer $C_1$-$C_6$-Alkyl-, $C_1$-$C_6$-Alkoxy- oder Hydroxylgruppe ausgewählt sind, wenn $R_3$ an ein Kohlenstoffatom gebunden ist;
oder
$R_3$ für ein oder zwei gleiche oder verschiedene Atome oder eine oder zwei gleiche oder verschiedene Gruppen steht, die unter einem Wasserstoffatom und einer $C_1$-$C_6$-Alkyl-, $C_1$-$C_6$-Alkyl-O-C(O)-oder Aryl-$C_1$-$C_3$-alkyl-O-C(O)-Gruppe ausgewählt sind, wenn $R_3$ an ein Stickstoffatom gebunden ist;

wobei $R_1$, A und P die unter der allgemeinen Formel (I) nach Anspruch 1 angegebene Bedeutung besitzen; in Basen- oder Säureadditionssalzform sowie in Hydrat- oder Solvatform.

**7.** Erfindungsgemäße Verbindung nach einem der Ansprüche 1 bis 6, **dadurch gekennzeichnet, daß** sie der allgemeinen Formel (V) entspricht:

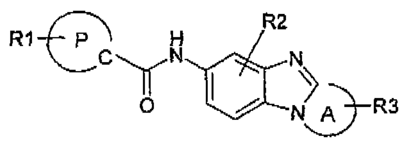

(V)

worin:

A mit der C-N-Bindung der Benzimidazol-Einheit, mit der es kondensiert ist, für einen 5- bis 7-gliedrigen monocyclischen Heterocyclus oder ein 5- bis 7-gliedriges monocyclisches Heteroaryl mit einem bis drei unter O, S oder N ausgewählten Heteroatomen einschließlich des Stickstoffatoms der Benzimidazol-Einheit steht;

wobei $R_1$, $R_2$, $R_3$ und P die unter der allgemeinen Formel (I) nach Anspruch 1 angegebene Bedeutung besitzen; in Basen- oder Säureadditionssalzform sowie in Hydrat- oder Solvatform.

**8.** Erfindungsgemäße Verbindung nach einem der Ansprüche 1 bis 7, **dadurch gekennzeichnet, daß** sie der allgemeinen Formel (Va) entspricht:

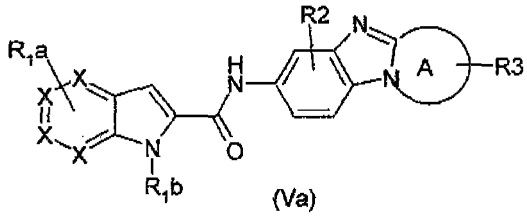

(Va)

worin

A mit der C-N-Bindung der Benzimidazol-Einheit, mit der es kondensiert ist, für einen 5- bis 7-gliedrigen monocyclischen Heterocyclus oder ein 5- bis 7-gliedriges monocyclisches Heteroaryl mit einem oder zwei unter O, S oder N ausgewählten Heteroatomen einschließlich des Stickstoffatoms der Benzimidazol-Einheit steht;

X für ein Kohlenstoffatom oder ein Stickstoffatom steht; wobei die Variablen X gleich oder voneinander verschieden sind und die Zahl von X = N nicht größer als 1 ist;

$R_{1a}$ für ein oder mehrere gleiche oder verschiedene Atome oder eine oder mehrere gleiche oder verschiedene Gruppen, die unter einem Wasserstoffatom, einem Halogenatom oder einer $C_1$-$C_6$-Alkyl-, $C_1$-$C_6$-Fluoroalkyl-, $C_1$-$C_6$-Alkoxy-, $C_1$-$C_6$-Thioalkyl-, $C_1$-$C_6$-Alkyl-S(O)$_2$-, $NR_4R_5$- oder Nitrogruppe ausgewählt sind, steht;

$R_{1b}$ für ein Wasserstoffatom oder eine $C_1$-$C_6$-Alkyl-, Heteroaryloxy-$C_1$-$C_6$-alkyl-, Aryl-$C_1$-$C_3$-alkylenoxy-$C_1$-$C_6$-alkyl-, $R_4R_5NC(O)$-$C_1$-$C_3$-Alkylen-, Aryl-, Heteroaryl-, Aryl-$C_1$-$C_6$-alkylen- oder Heteroaryl-$C_1$-$C_6$-alkylengruppe steht; wobei die Heteroaryl- oder Arylgruppen von $R_{1b}$ gegebenenfalls durch einen oder mehrere gleiche oder voneinander verschiedene Substituenten $R_9$ substituiert sind;

$R_2$ für ein Wasserstoffatom steht;

$R_3$ für ein Atom oder eine Gruppe steht, das bzw. die unter einem Wasserstoffatom und einer $C_1$-$C_6$-Alkyl-, $C_1$-$C_6$-Alkoxy- oder Hydroxylgruppe ausgewählt ist, wenn $R_3$ an ein Kohlenstoffatom gebunden ist;

oder

$R_3$ für ein Atom oder eine Gruppe steht, das bzw. die unter einem Wasserstoffatom und einer $C_1$-$C_6$-Alkyl- oder $C_1$-$C_6$-Alkyl-O-C(O)-Gruppe ausgewählt ist, wenn $R_3$ an ein Stickstoffatom gebunden ist;

$R_9$ und $R_5$ unabhängig voneinander für eine $C_1$-$C_6$-Alkylgruppe stehen;

oder $R_4$ und $R_5$ gemeinsam mit dem Stickstoffatom, an das sie gebunden sind, eine Pyrrolidin- oder Morpholingruppe bilden;

$R_9$ für ein Halogenatom oder eine $C_1$-$C_6$-Alkyl-, $C_1$-$C_6$-Alkoxy-, $C_1$-$C_6$-Fluoralkyl-, Aryl-, Heteroaryl-, $NR_4R_5$- oder Arylthiogruppe steht, wobei die Arylgruppen gegebenenfalls durch eine oder mehrere $C_1$-$C_6$-Alkylgruppen substituiert sind;

in Basen- oder Säureadditionssalzform sowie in Hydrat- oder Solvatform.

9. Erfindungsgemäße Verbindung nach einem der Ansprüche 1 bis 8, **dadurch gekennzeichnet, daß** sie der allgemeinen Formel (I) entspricht, worin gleichzeitig $R_1$ und/oder $R_2$ und/oder $R_3$ und/oder A und/oder P und/oder Y die in den Ansprüchen 1 bis 8 angegebene Bedeutung besitzen;
in Basen- oder Säureadditionssalzform sowie in Hydrat- oder Solvatform.

10. Verfahren zur Herstellung einer Verbindung der Formel (I) nach einem der Ansprüche 1 bis 9, **dadurch gekennzeichnet, daß** man eine Verbindung der allgemeinen Formel (VI):

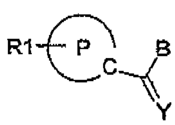

(VI)

worin P, $R_1$ und Y die unter der allgemeinen Formel (I) nach Anspruch 1 angegebene Bedeutung besitzen und B für eine $C_1$-$C_6$-Alkoxy-, $C_3$-$C_7$Cycloalkyloxy-, $C_3$-$C_7$-Cycloalkyl-$C_1$-$C_3$-alkylenoxy- oder Aryl-$C_1$-$C_3$-alkylenoxygruppe steht, unter Rückfluß eines Lösungsmittels mit einem Amid der Verbindung der allgemeinen Formel (VII);

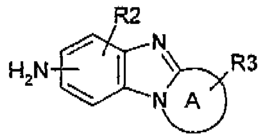

**(VII)**

worin A, $R_2$ und $R_3$ die unter der allgemeinen Formel (I) nach Anspruch 1 angegebene Bedeutung besitzen, umsetzt, wobei das Amid der Verbindung der allgemeinen Formel (VII) durch vorherige Einwirkung von Trimethylaluminium auf die Amine der allgemeinen Formel (VII) hergestellt wird.

**11.** Verfahren zur Herstellung einer Verbindung der Formel (I) nach einem der Ansprüche 1 bis 9, **dadurch gekennzeichnet, daß** man eine Verbindung der allgemeinen Formel (VI):

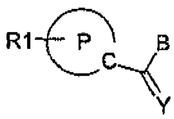

**(VI)**

worin P, $R_1$ und Y die unter der allgemeinen Formel (I) nach Anspruch 1 angegebene Bedeutung besitzen und B für eine Hydroxylgruppe steht, durch Einwirkung von Thionylchlorid unter Rückfluß eines Lösungsmittels in das Säurechlorid umwandelt und dann die erhaltene Verbindung der allgemeinen Formel (VI), worin P, $R_1$ und Y die unter der allgemeinen Formel (I) nach Anspruch 1 angegebene Bedeutung besitzen und B für ein Chloratom steht, in Gegenwart einer Base mit der Verbindung der allgemeinen Formel (VII):

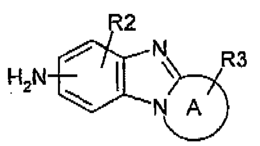

**(VII)**

worin A, $R_2$ und $R_3$ die unter der allgemeinen Formel (I) nach Anspruch 1 angegebene Bedeutung besitzen, umsetzt; oder auch eine Kupplungsreaktion zwischen einer Verbindung der allgemeinen Formel (VI), worin P, $R_1$ und Y die unter der allgemeinen Formel (I) nach Anspruch 1 angegebene Bedeutung besitzen und B für eine Hydroxylgruppe steht, und der Verbindung der allgemeinen Formel (VII), worin A, $R_2$ und $R_3$ die unter der allgemeinen Formel (I) nach Anspruch 1 angegebene Bedeutung besitzen, in Gegenwart eines Kupplungsmittels und einer Base in einem Lösungmittel durchführt.

**12.** Verbindung der allgemeinen Formel (VIIa), (VIIb), (VIIc), (VIId), (VIIe), (VIIf), (VIIg), (VIIh), (VIIi), (VIIj), (VIIk), (VIIl), (VIIm), (VIIn):

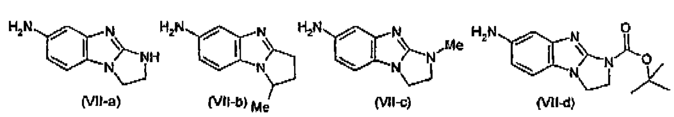

(VII-a) (VII-b) (VII-c) (VII-d)

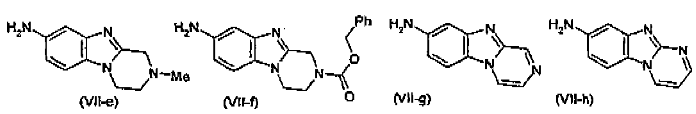

(VII-e) (VII-f) (VII-g) (VII-h)

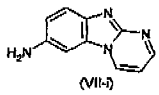
(VII-i)

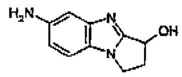
(VII-j)

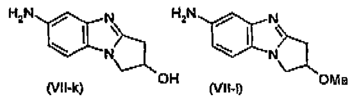
(VII-k) (VII-l)

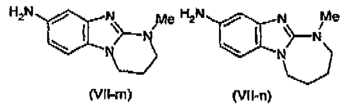
(VII-m) (VII-n)

**13.** Arzneimittel, **dadurch gekennzeichnet, daß** es mindestens eine Verbindung der Formel (I) nach einem der Ansprüche 1 bis 9 oder ein Additionssalz dieser Verbindung mit einer pharmazeutisch unbedenklichen Säure oder auch ein Hydrat oder Solvat der Verbindung der Formel (I) enthält.

**14.** Pharmazeutische Zusammensetzung, **dadurch gekennzeichnet, daß** sie mindestens eine Verbindung der Formel (I) nach einem der Ansprüche 1 bis 9 oder ein pharmazeutisch unbedenkliches Salz, ein Hydrat oder ein Solvat dieser Verbindung sowie mindestens einen pharmazeutisch unbedenklichen Hilfsstoff enthält.

**15.** Verwendung einer Verbindung der Formel (I) nach einem der Ansprüche 1 bis 9 zur Herstellung eines Arzneimittels zur Prävention oder Behandlung von Pathologien, an denen die Rezeptoren vom TRPV1-Typ beteiligt sind.

**16.** Verwendung einer Zusammensetzung der Formel (I) nach einem der Ansprüche 1 bis 9 zur Herstellung eines Arzneimittels zur Prävention oder Behandlung von Schmerzen, Entzündungen, Stoffwechselstörungen, urologischen Störungen, gynäkologischen Störungen, gastrointestinalen Störungen, respiratorischen Störungen, Psoriasis, Pruritis, Haut-, Augen- oder Schleimhautreizungen, Herpes, Zona, multipler Sklerose und Depression.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2005028445 A
- WO 200505798 A
- EP 1535922 A
- US 2006135447 A, S.L. Chupak
- WO 2004101563 A


### Littérature non-brevet citée dans la description


- **J. March.** Advances in Organic Chemistry. Wiley Interscience, 2001
- **R.S. Begunov et al.** *Russian J. Org. Chem.,* 2004, vol. 40 (11), 1740-1742
- **V.M. Reddy et al.** *J. Indian Chem. Soc.,* 1984, 89-91
- **K.V.B. Rao et al.** *Eur.J. Med. Chem.,* 1981, vol. 16 (1), 35-38
- **R.J. North et al.** *J.Het.Chem.,* 1969, vol. 6, 655
- **A.R. Freedman et al.** *J.Het.Chem.,* 1966, vol. 3 (3), 257
- **Mullock, E.B.** *J.Chem. Soc. Section C,* 1970, 829-833
- **A. Furstner et al.** *J Am Chem Soc,* 2002, vol. 124 (46), 13856
- **G. Quéguiner et al.** *J Org Chem,* 1998, vol. 63 (9), 2892
- **S.L. Buchwald et al.** *J Am Chem Soc,* 2002, vol. 124, 11684
- *Pharmazie,* 1990, vol. 45, 346
- **G.A.Olah.** *J.Org.Chem.,* 1993, 3194-3195
- **R.J. North et al.** *J.HetChem.,* 1969, vol. 6, 655
- *J.Med.Chem.,* 1995, vol. 38 (20), 4098-4105
- **Saunders et al.** *J.Chem.Soc.,* 1955, 3275-3287
- **Nazare, M. et al.** *Angewandte Chemie, International Ed.,* 2004, vol. 43 (34), 4526-4528
- **Freedman et al.** *J.Het.Chem.,* 1966, vol. 3 (3), 257-259
- **Freedman et al.** *J.Het.Chem.,* 1966, vol. 3 (3), 257-259
- **Begunov et al.** *Russian J. Org. Chem.,* 2004, vol. 11 (40), 1694-1696
- **Nazare, M. et al.** *Angewandte Chemie, International Ed,* 2004, vol. 43 (34), 4526-4528
- *Tet. Lett.,* 1996, vol. 37, 2459-2462